# EUROPEAN PATENT SPECIFICATION

(11) **EP 1 121 369 B1**
(45) Date of publication and mention of the grant of the patent: **08.12.2004**
(21) Application number: 99970662.5
(22) Date of filing: 14.10.1999
(51) Int. Cl.: C07H 19/056, A61K 31/70

(54) **RIBAVIRIN-INTERFERON ALFA COMBINATION THERAPY FOR ERADICATING DETECTABLE HCV-RNA IN PATIENTS HAVING CHRONIC HEPATITIS C INFECTION**
RIBAVIRIN-INTERFERON-ALPHA-KOMBINATIONSTHERAPIE ZUR VERNICHTUNG NACHWEISBARER HCV-RNA IN CHRONISCH HEPATITIS C INFIZIERTEN PATIENTEN
THERAPIE COMBINEE RIBAVIRINE-INTERFERON ALPHA PERMETTANT DE SUPPRIMER L'ARN DE VHC POUVANT ETRE DETECTE CHEZ DES PATIENTS ATTEINTS DE L'HEPATITE C CHRONIQUE

(30) Priority: 16.10.1998 US 174059; 07.07.1999 US 348534
(43) Date of publication of application: 08.08.2001
(73) Proprietor: SCHERING CORPORATION, Kenilworth, New Jersey 07033-0530 (US)
(72) Inventor: GANGULY, Ashit, K., Upper Montclair, NJ 07043 (US); MCCORMICK, Jinping, Edison, NJ 08820 (US); LOVEY, Raymond, G., West Caldwell, NJ 07006 (US); BENNETT, Frank, Piscataway, NJ 08854 (US); SAKSENA, Anil, K., Upper Montclair, NJ 07043 (US); GIRIJAVALLABHAN, Viyyoor, M., Parsippany, NJ 07054 (US)
(74) Representative: Ritter, Stephen David
(86) International application number: PCT/US1999/021448
(87) International publication number: WO 2000/023454

(56) References cited:
- EP-A- 0 090 405
- EP-A- 0 707 855
- WO-A-94/22887
- WO-A-98/21223
- DE-A- 2 511 828
- US-A- 4 925 930
- BRISSOT, P.: "Traitement de l'hépatite chronique C par les associations thérapeutiques" GASTROENTEROLOG. CLIN. BIOL., vol. 20, 1997, pages S89-S95, XP000872624

## Description

### BACKGROUND OF THE INVENTION

This invention relates to ribavirin derivatives and uses thereof for eradicating detectable HCV-RNA in patients having chronic hepatitis C infection.

The present invention further relates to uses of such ribavirin derivatives for treating patients having chronic hepatitis C infection by administering a therapeutically effective amount thereof and a therapeutically effective amount of interferon-alpha. Chronic infection with hepatitis C virus is an insidious and slow-progressing disease having a significant impact on the quality of life. It can eventually result in cirrhosis of the liver, decompensated liver disease and/or hepatocelluar carcinoma.

Combination treatment with interferon alfa-2b and ribavirin of patients with chronic hepatitis C is disclosed by Reichard et al. (The Lancet 1998; 351;83-87; and T. Polynard et al. (The Lancet, 1998, Vol. 352, Oct. 31, p 1426-1432). See also J.G. McHutchinson et al. (N. Engl. J. Med., 1998, 339:1485-1492); and G.L. Davis et al. (N. Engl. J. Med., 1998, 339:1493-1499). However, this combination therapy is not always effective due to side effects associated ribavirin such as ribavirin-related hemolysis, and anemia.

WO 98/21223 discloses nucleoside analogues comprising at least two hydroxy functions on the sugar or acyclic moieties, one of which sugar or acyclic hydroxy functions is esterified with an aliphatic amino acid and the other of which is esterified with a saturated or monounsaturated, optionally substituted fatty acid having 6 to 22 carbon atoms.

There is a definite need for more potent, safer ribavirin derivatives having fewer side effects for use as monotherapy or in combination with antiviral agents, e.g., interferon-alpha, to treat patients having suscept-ible viral infections, e.g., chronic hepatitis C infections, in a long-term, effective manner.

### SUMMARY OF THE INVENTION

The present invention provides a compound represented by formula III 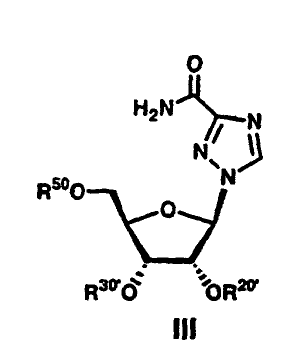 wherein at least one of R^{50'} R^{30'} or R^{20'} is represented by the formula 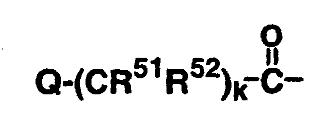 wherein the other two of R^{50'} R^{30'} or R^{20'} is represented by H or the formula 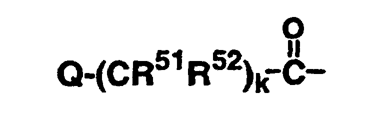 wherein Q is: 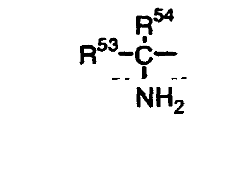 wherein R⁵¹ and R⁵² are independently H, alkyl, alkenyl , alkynyl, (C₃-C₇)cycloalkyl, arylalkyl, or alkyl, alkenyl , alkynyl, (C₃-C₇)cycloalkyl, arylalkyl, substituted by halo, OH, SH , CF₃, ,SR⁵⁷,OR⁵⁷ or NH₂ or
wherein R⁵¹ and R⁵² taken together with the carbon atom in (C R⁵¹R⁵²) form a cyclopropane , cyclobutane, cyclopentane, or cyclohexane.;
wherein R⁵³ and R⁵⁴ are independently H, alkanoyl,alkyl, aryl, arylalkyl, alkenyl , alkynyl, or alkanoyl, alkyl, aryl, arylalkyl, alkenyl, alkynyl substituted by halo, OH, SH , CF₃, ,SR⁵⁷,OR⁵⁷; or R⁵³ and R⁵⁴ are independently 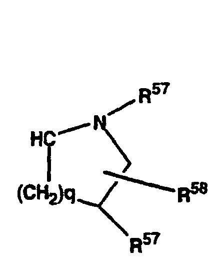 or 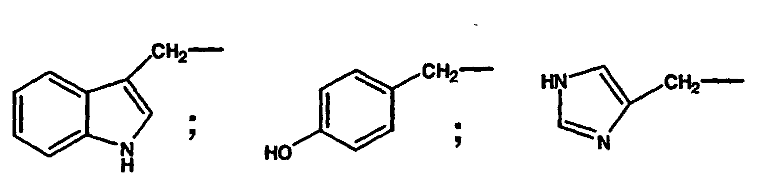 wherein R⁵⁷ is H, alkyl, alkanoyl, alkenoyl, aryl, arylalkyl, alkenyl, alkynyl, or, alkyl, aryl, arylalkyl, alkenyl, alkynyl substituted by halo, OH, SH,CF₃,alkanoylthienyl,or alkanoyloxy;
wherein R⁵⁸ is H, alkyl, aryl, arylalkyl, alkenyl,or alkynyl;
and q is 0,1 or 2, and k is 1 or 2;
or a pharmaceutically acceptable salt thereof.
In a preferred embodiment, R^{30'} and R^{20'} in the compounds of Formula III are each H.

The present invention provides a compound represented by the formula IV 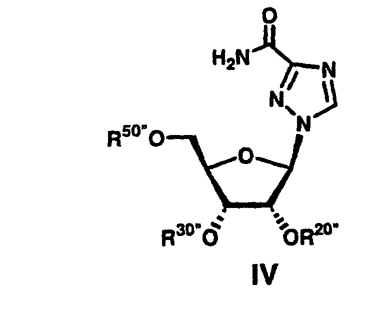 wherein at least one of R^{50"}, R^{30"}, R^{20"} is represented by the formula 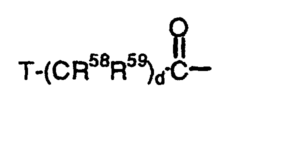 and wherein the other two of R^{50"}, R^{30"}, R^{20"} is H or is represented by the formula 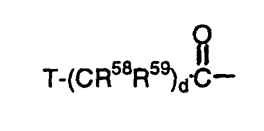 wherein T is a moiety represented by the formulas:
H₂NCH₂-, H₂NCH₂ CH₂-, CH₃CH(H₂N)-, CH₃CH₂CH(H₂N)-, CH₃(CH₂)₂CH(H₂N)-, (CH₃)₂CHCH(H₂N)-, (CH₃)₂CHCH₂CH(H₂N)-, CH₃CH₂CH(CH₃ )CH(H₂N)-, PhCH₂CH(H₂N)-, HOOCCH₂CH₂CH(H₂N)-, HOOCCH₂CH(H₂N)-, HSCH₂CH(H₂N)-, CH₃SCH₂CH₂CH(H₂N)-, HOCH₂CH(H₂N)-, ,H₂N(CH2)₄ CH(H₂N)-, CH₃CH(OH)CH(H₂N), 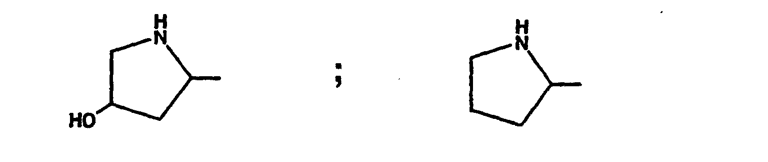 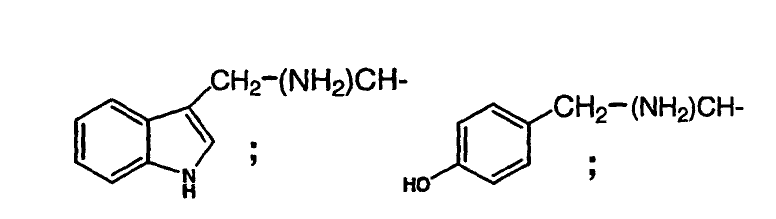 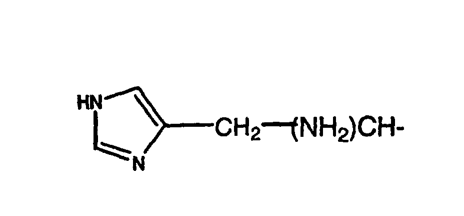 wherein R⁵⁸ and R⁵⁹ are independently H, alkyl, alkenyl, alkynyl, (C₃-C₇)cycloalkyl, arylalkyl, or alkyl, alkenyl, alkynyl, (C₃-C₇)cycloalkyl, arylalkyl, substituted by halo, OH, SH, CF₃, SR⁶⁰,OR⁶⁰ or NR⁶⁰R⁶¹ or wherein R⁵⁸ and R⁵⁹ taken together with the carbon atom in (C R⁵⁸ R⁵⁹) form a cyclopropane, cyclobutane, cyclopentane, or cyclohexane.;
wherein R⁶⁰ is H, alkyl, alkanoyl, alkenoyl, aryl, arylalkyl, alkenyl, alkynyl, or, alkyl, aryl, arylalkyl, alkenyl, alkynyl substituted by halo, OH, SH,CF₃,alkanoylthienyl,or alkanoyloxy; wherein R⁶¹ is H, alkyl, aryl, arylalkyl, alkenyl,or alkynyl; and d is 0, 1 or 2; or a pharmaceutically acceptable salt thereof.

In a preferred embodiment, R^{30"} and R^{20"} in the compounds of Formula IV are each H.

The present invention provides a compound represented by the formula V 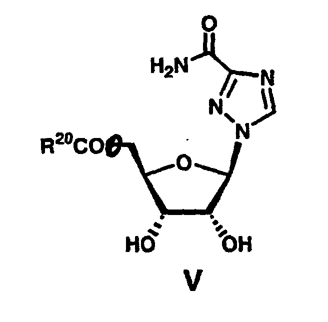 wherein R²⁰ CO- is a natural or unnatural amino acid moiety represented by the formulas 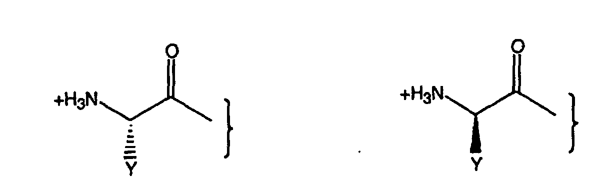 Y = H, CH₃; CH₃CH₂-; CH₃CH₂CH₂-; Me₂CH-; Me₂CH₂CH₂-; CH₃CH₂CH(Me)-PhCH₂-; HOOCCH₂CH₂-; HSCH₂-; HOOCCH₂-; MeSCH₂CH₂-; HOCH₂-; 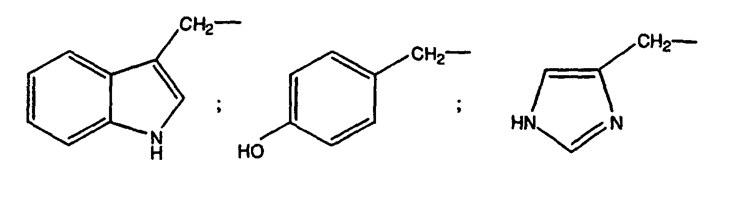 or Y is H₂N(CH₂)₄- or CH₃CH(OH)-; or a pharmaceutically acceptable salt thereof; or Y taken together with the α carbon and N form 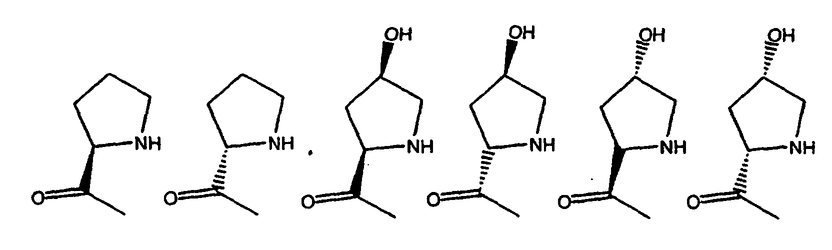 or a phamaceutically acceptable salt thereof;
or Y taken together with the α carbon and N form 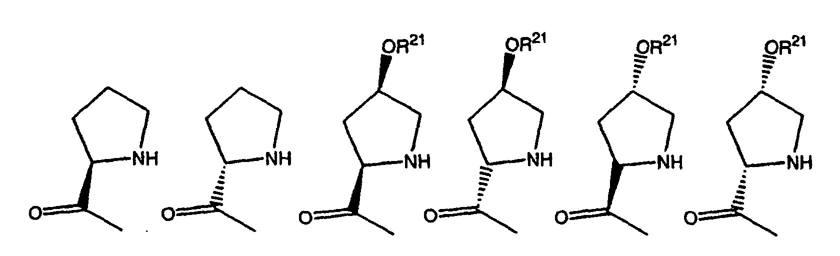 or a phamaceutically acceptable salt thereof.

The present invention provides a compound represented by the formula VI: 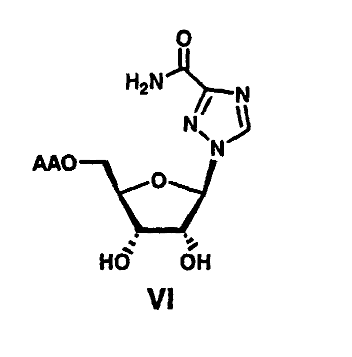 or a pharmaceutically acceptable salt thereof;
wherein AA is a natural or unnatural amino acid moiety of the formula:

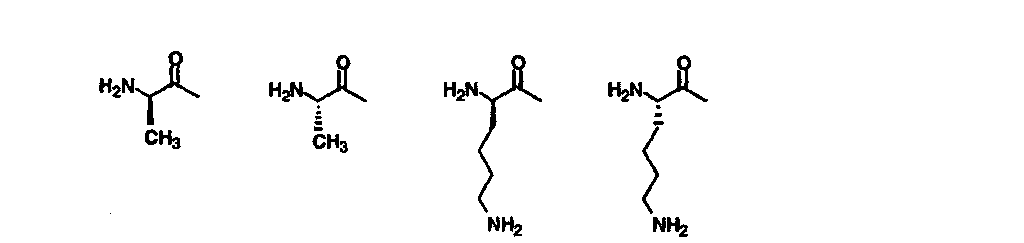

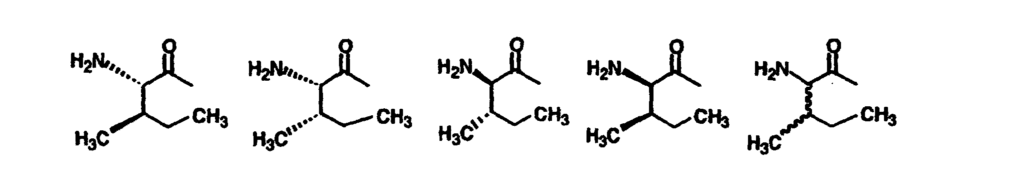

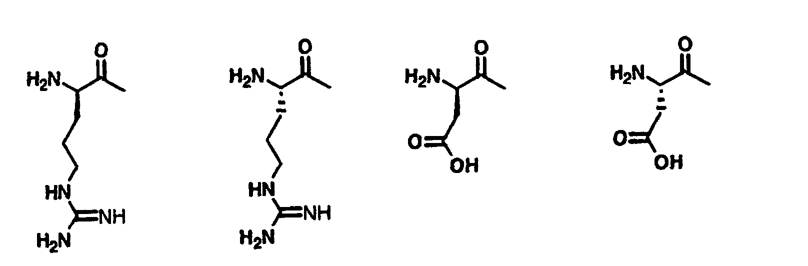

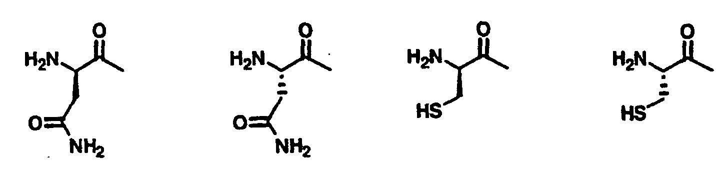

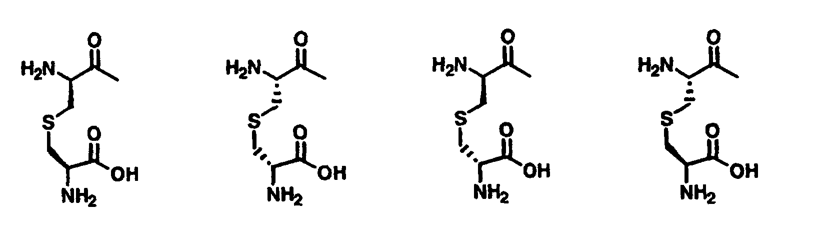

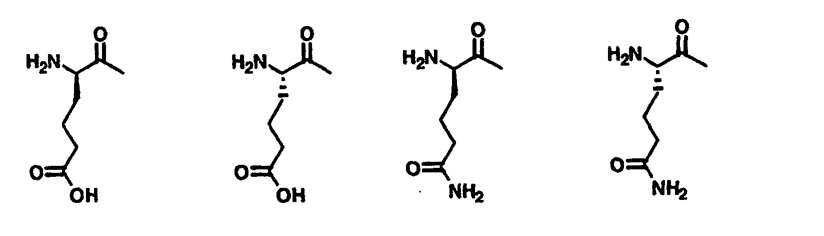

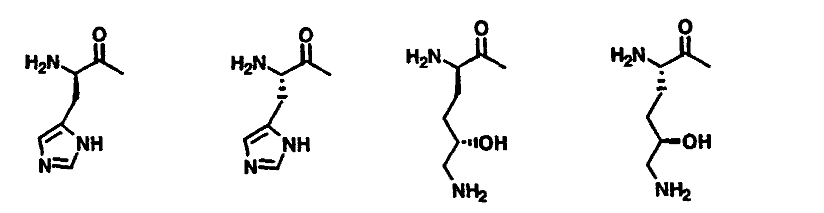

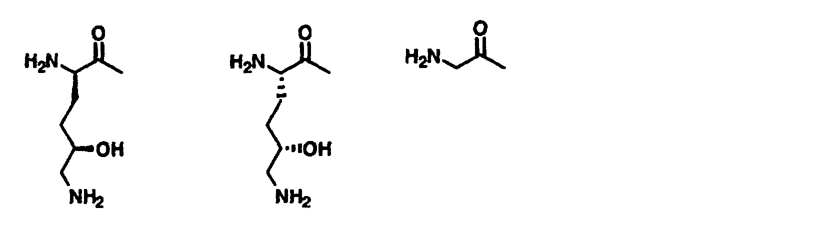

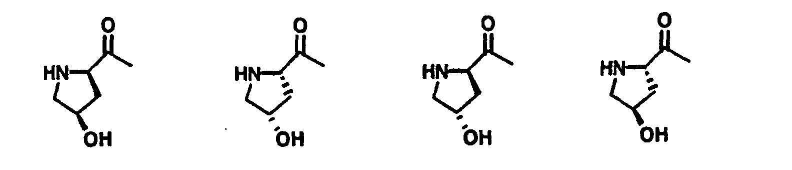

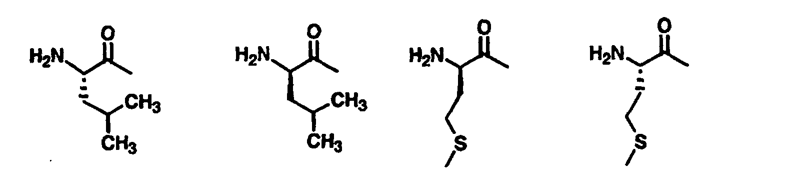

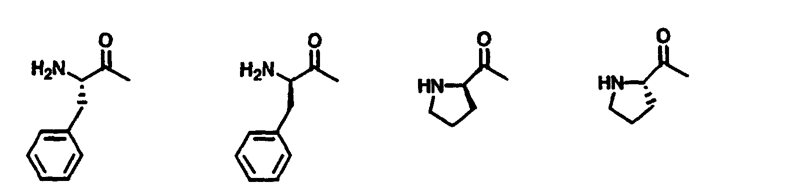

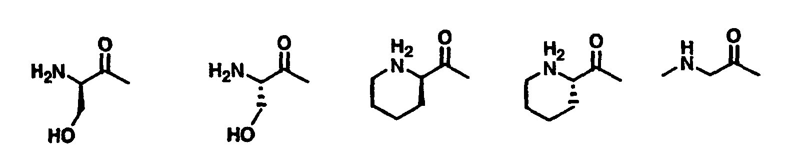

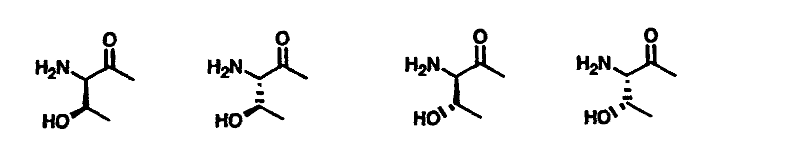

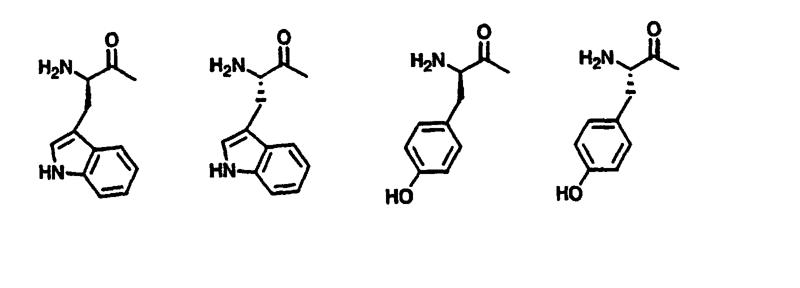

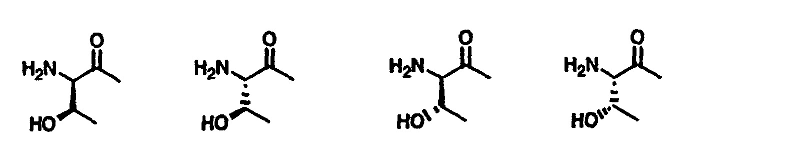

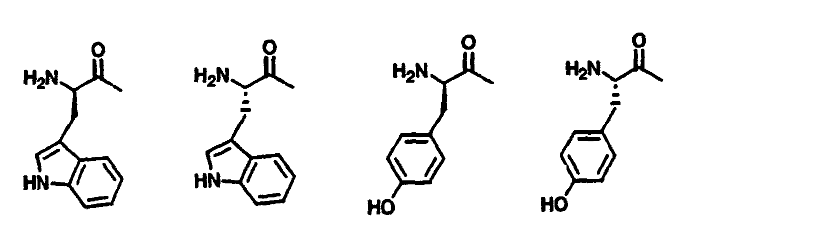

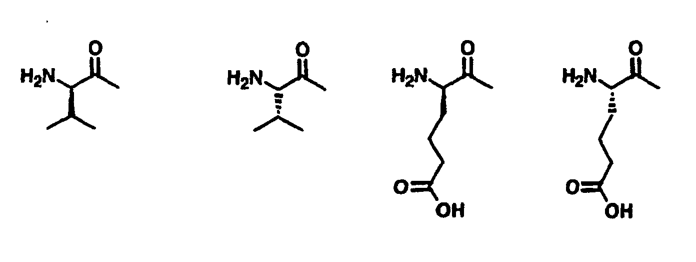

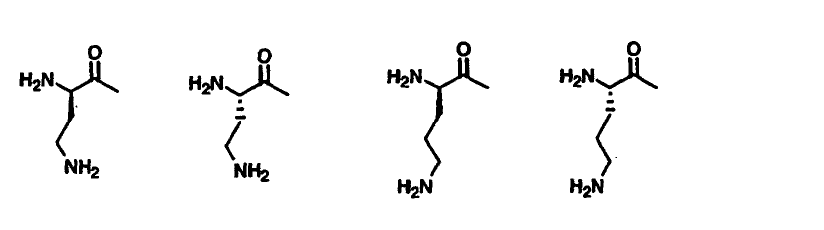

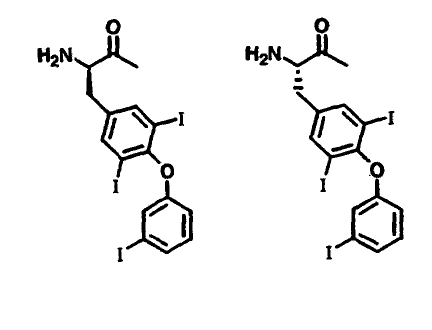

The present invention also provides a compound represented by formula VII 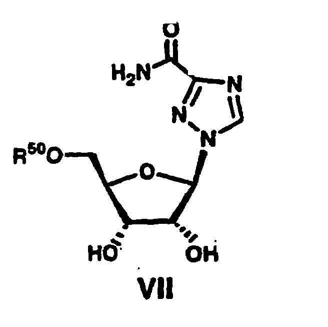 wherein R⁵⁰ is CH₃CH(NH₂)-CO-, CH₃CH₂(CH₃)CHCH(NH₂)-CO- or H₂N(CH(NH₂)-CO-;
or a pharmaceutically acceptable salt thereof.

The present invention also provides a compound represented by the formula VIII 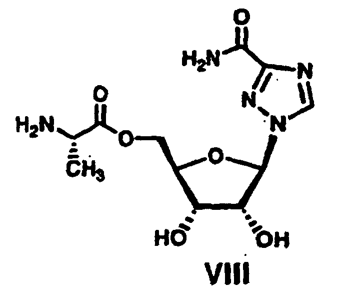 or a pharmaceutically acceptable salt thereof.

The present invention also provides pharmaceutical compositions for treating susceptible viral infections comprising a compound of any of formulae III, IV, V, VI, VII and VIII and at least one pharmaceutically acceptable carrier.

The present invention also provides the use of a compound of any of formulae III, IV, V, VI, VII and VIII for preparation of a medicament for treating a patient infected with chronic hepatitis C which comprises an effective amount of said compound in association with an effective amount of an interferon alfa sufficient to eradicate detectable HCV-RNA levels.

### DETAILED DESCRIPTION

The term "alkyl" as used herein means straight and branched carbon chains of one to twenty carbons, preferably one to six carbons.and more preferably one to three carbons.

The term "alkenyl" as used herein means straight and branched chain alkyl groups containing at least one carbon-carbon double bond and two to twenty carbons, preferably two to eight carbons.

The term "alkynyl" as used herein means straight and branched chain alkyl groups containing at least one carbon-carbon triple bond and two to twenty carbons, and preferably two to six carbons containing at least one carbon-carbon triple bond.

The term "cycloalkyl" as used herein means carbocyclic rings of three to twelve carbons, preferably three to seven carbons.and more preferably three to six carbons optionally substituted by one double bond.

The term "alkanoyl" as used herein means straight and branched chain alkanoyl groups of one to twenty carbons, preferably two to twelve, more preferably two to ten and most preferably two to six carbons.

The term "alkenoyl" as used herein means straight and branched chain alkenoyl groups of one to twenty carbons containing at least one carbon-carbon double bond, preferably two to twelve,or more preferably two to ten and most preferably two to six carbons containing at least one carbon-carbon double bond.

The term "halo" as used herein means fluroro, chloro or bromo, preferably fluroro or chloro.

The term "alkynoyl" as used herein means straight and branched chain alkenoyl groups of one to twenty carbons containing at least one carbon-carbon triple bond, preferably two to twelve,or more preferably two to ten and most preferably two to six carbons containing at least one carbon-carbon triple bond.

The term "alkoxy" as used herein means straight and branched chain alkyl groups containing one bond to oxygen at the one carbon and one to ten carbons. Typically suitable alkoxy includes methoxy, ethoxy and tert-butoxy.

The term "aryl" as used herein (including the aryl portion of aryloxy and aralkyl, e.g., benzyl)-represents a carbocyclic group containing from 6 to 15 carbon atoms and having at least one aromatic ring (e.g., aryl is a phenyl ring), or is a polycyclic aromatic containing one or more heteroatoms, e.g., N or S such as quinoyl, isoquinolyl with all available substitutable carbon atoms of the carbocyclic group being optionally substituted (e.g., 1 to 3) with one or more of halogen, alkyl, hydroxy, alkoxy, CN, phenoxy, CF₃, amino, alkylamino, dialkylamino, SH, S⁻ M⁺ or -NO₂; and the term "M⁺" represents an alkali metal cation such as Na⁺, K⁺ and Li⁺.

The term "arylalkyl" as used herein means an alkyl group substituted by an aryl group.

The term "heterocyclic" as used herein means a cyclic group represented by the formula; 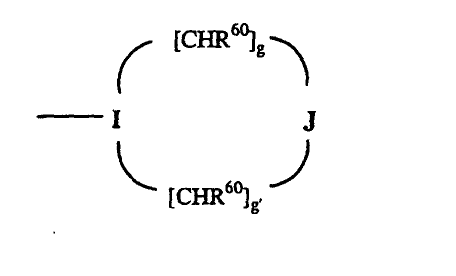 wherein **J** is -CHR⁶⁰-, -O-, -NR⁶⁰-, -S-, -SO- or -SO₂-, and I is -CR⁶⁰ or -N-; and R⁶⁰ is H, alkyl or aryl., and g ang' are indepedentlyb1 to 4 and g +g' are 2, 3, 4 or 5. Typically suitable heterocyclics include

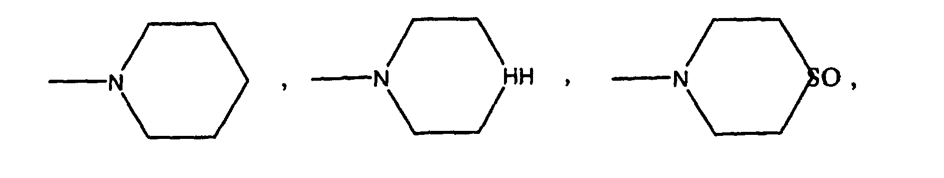

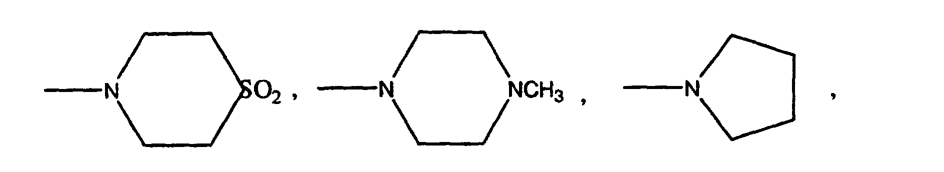

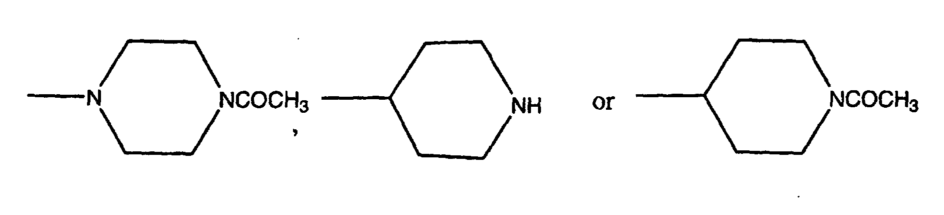

The term "halo" as used herein means fluoro, chloro, bromo or iodo,. preferably fluroro or chloro.

In a preferred embodiment of the present invention, the compounds of formula I have one or two of R², R³ and R⁵ equal to
R⁶ (W)ₓCO-, (HO)₂PO- or R⁶(W)ₓPO(OH) - and at least one of R², R³ and R⁵ equal to H. Most preferred compounds of formula I have R²=R³=H and R⁵ equal to R⁶(W)ₓCO-, (HO)₂PO- or R⁶(W)ₓPO(OH)-. In the preferred embodiments of R⁶-(W)ₓCO- and R⁶(W)ₓ PO(OH) -, W=O and x=0 or 1. In the preferred embodiments of R⁶-(W)ₓCO- and R⁶(W)ₓP(OH)O- include R¹⁷(CH₂)ₘ-NR^{7b}R^{7a}-(CH₂)ₙOCO- and
R¹⁷(CH₂)ₘ-NR^{7b}R^{7a}-(CH₂)ₙ-O-PO(OH), wherein m=0 to 4 and n= 0 to 4; and R¹⁷ is H, Me, MeCO- or Me₂N-
and R^{7a} R^{7b}N (CH₂)- (CHR^{7a})ₑOCO- or R^{7a} R^{7b}N (CH₂),- (CHR^{7a})ₑCO-
wherein f= 0 to 4 and e= 1 to 5, and R^{7a} R^{7b}N is Me₂N- , MeHN- or MeCONH-. In most preferred embodiments of R⁶(W)ₓCO-, x=0 and R⁶CO is one of 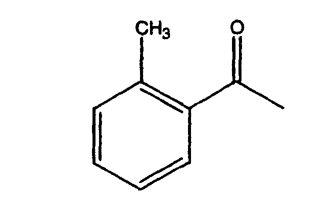 HOCH(CH₃)CO-, HOCH(C₆H₅)CO-, C₂H₅CH(OH)CO- or
CH₃CO₂(CH₂)₂CO-
or R⁶ in R⁶CO is one of 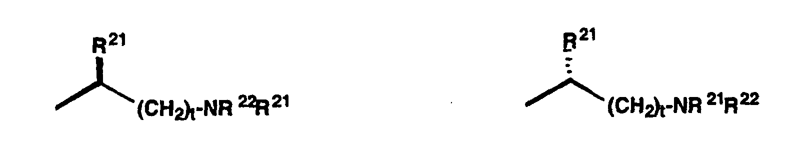 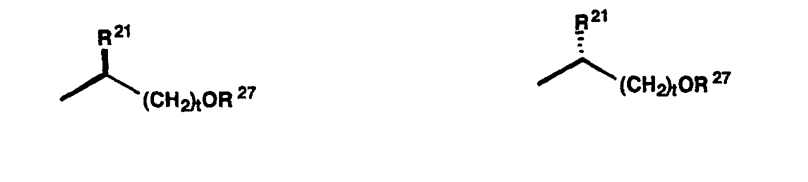 or R⁶CO is a natural or unnatural α-amino acid residue - 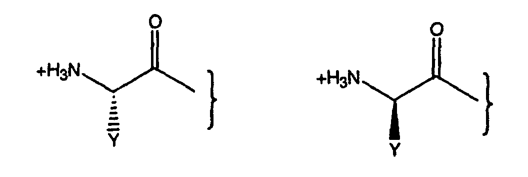 wherein Y = H, CH₃; CH₃CH₂-; CH₃CH₂CH₂-; Me₂CH-; Me₂CH₂CH₂-; CH₃CH₂CH(Me)-PhCH₂-; HOOCCH₂CH₂-; HSCH₂-; HOOCCH₂-; MeSCH₂CH₂-; HOCH₂-; 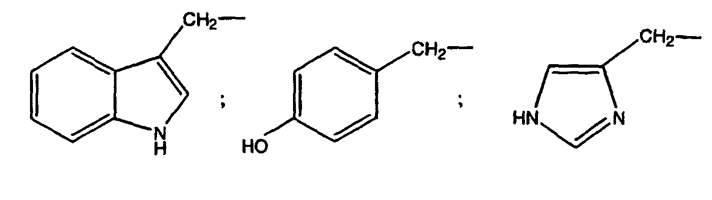 or Y is H₂N(CH₂)₄- or CH₃CH(OH)-; or a pharmaceutically acceptable salt thereof; or Y taken together with the α carbon and N form 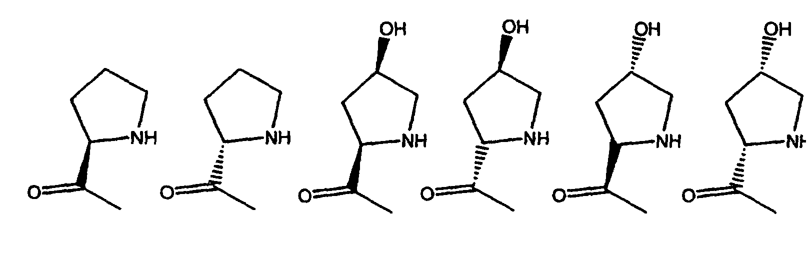 or a pharmaceutically acceptable salt thereof.
or 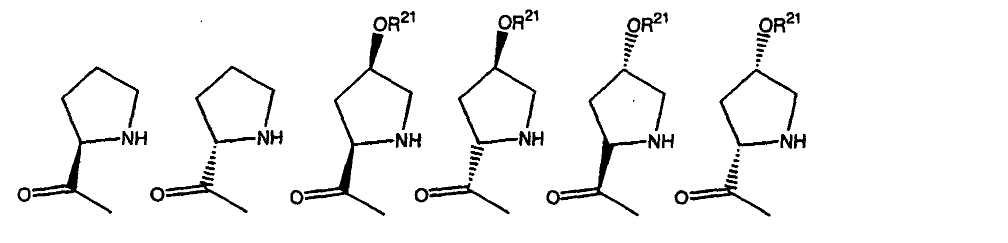 wherein R²¹ is as defined hereinabove; or a pharmaceutically acceptable salt thereof.

Other preferred embodiments for R⁶CO in the compounds of formula I include 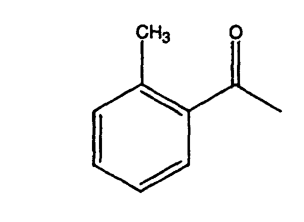 (CH₃)₃CO-, C₆H₅CO-,(HO)₂PO - and
L-C₆H₅CH₂OCONHCH(CH₃)CO-, i.e., C₆H₅CH₂OCONHCH

In a preferred embodiment of the present invention, the compounds of formula II have one or two of R², R³ and R⁵ equal to
R²⁰ (W)ₓCO- or R²⁰(W)ₓPO(OH) - and at least one of R², R³ and R⁵ equal to H. Most preferred compounds of formula II have R²=R³=H and R⁵ equal to R20⁶(W)ₓCO- or R²⁰(W)ₓP(OH)O-. In the preferred embodiments of R²⁰(W)ₓCO- and R²⁰(W)ₓP(OH)O-, W=O and x=O or 1. In the preferred embodiments of R²⁰(W)ₓCO- and R²⁰(W)ₓP(OH)O- include R²⁷(CH₂)ₘ-NR²¹R²²-(CH₂)ₙOCO- and R²⁷(CH₂)ₘ-NR²¹R²²-(CH₂)ₙ CO-, R²⁷(CH₂)ₘ-NR²¹R²²-(CH₂)ₙO PO(OH)- wherein m=0 to 4 and n= 1 to 5; R²⁷ is H, Me, MeCO- or Me₂N-, R²¹ is H, Me, or MeCO-R²² is H, Me, or MeCH₂. and R²¹ R²²N is Me₂N- , MeHN- or MeCONH-.

In most preferred embodiments of R²⁰(W)ₓCO-, x=0 and R²⁰CO is
HOCH(CH₃)CO-, HOCH(C₆H₅)CO-, C₂H₅CH(OH)CO- or
CH₃CO₂(CH₂)₂CO- or R²⁰CO is a natural α-amino acid residue 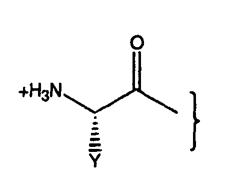 Y = H, CH₃; CH₃CH₂-; CH₃CH₂CH₂-; Me₂CH-; Me₂CH₂CH₂-; CH₃CH₂CH(Me)-PhCH₂-; HOOCCH₂CH₂-; HSCH₂-; HOOCCH₂-; MeSCH₂CH₂-; HOCH₂-; 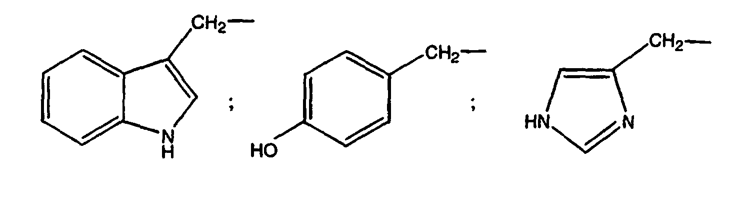 or Y is H₂N(CH₂)₄- or CH₃CH(OH)-;
or Y taken together with the α carbon and N form 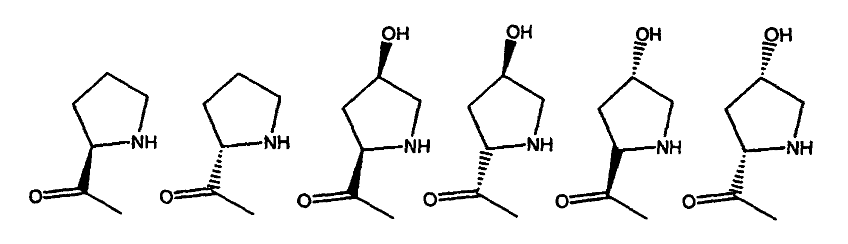 or 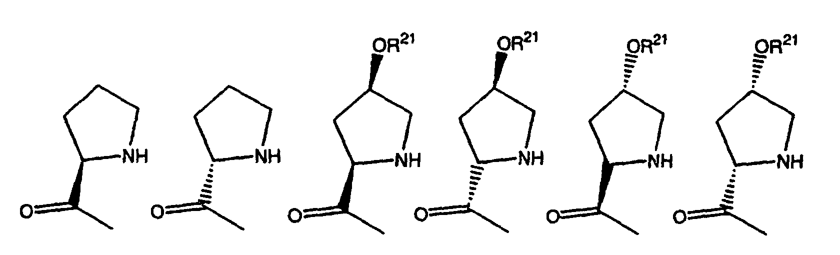 wherein R²¹ is as defined hereinabove;
or a pharmaceutically acceptable salt thereof.
or R²⁰CO is 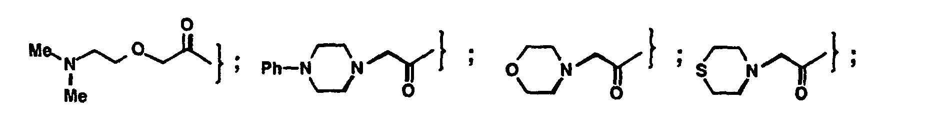 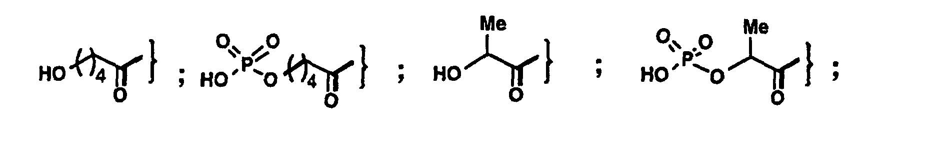 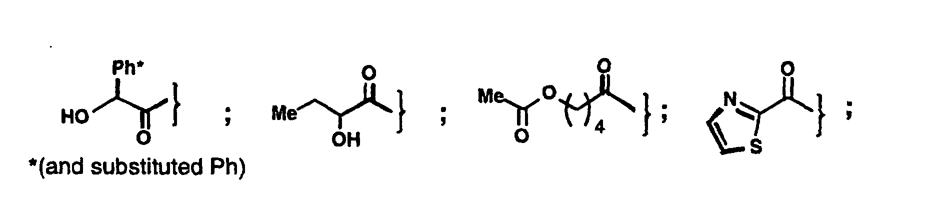 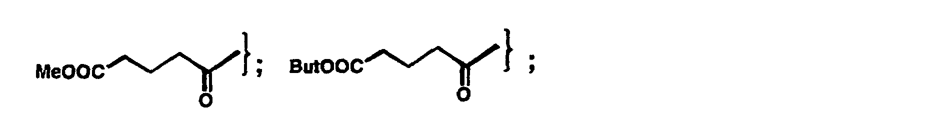 or a pharmaceutically acceptable salt thereof;
wherein Ph is phenyl and phenyl substituted by halo, CN, NO₂, OH, CO₂H, or alkoxy.

The compounds of formulas I to VIII metabolize *in vivo* into ribavirin and are useful for treating susceptible viral infections treatable with ribavirin, alone, or in combination with other ant-viral therapies .eg., interferon-alfa, and so- called Highly Active Antiretroviral Therapy ("HAART"). A-M. Vandamme et al., Antiviral Chemistry & Chemotherapy, 9:187-203 (1998) disclose current clinical treatments of HIV-1 infections in man including at least triple drug combinations or so-called Highly Active Antiretroviral Therapy ("HAART"); HAART involves various combinations of nucleoside reverse transcriptase inhibitors ("NRTI"), non-nucleoside reverse trans-criptase inhibitors ("NNRTI") and HIV protease inhibitors ("PI"). The treating of patients having chronic hepatitis C with the compounds of formulas I-VIII is performed as part of a combination therapy with interferon-alfa, including interferon alfa-2a, interferon alfa-2b, consensus interferon especially interferon alfa-2b as well as pegylatyed interferon alfa-2a and pegylatyed interferon alfa-2b.

The present invention provides methods and pharmaceutical compositions containing a compound of formulas II-VIII for treating susceptible viral infections, especially hepatitis C viral infections.

The term "susceptible viral infections" as used herein means viral infections caused by a wide range of RNA and DNA viruses, including, but not limited to, the families of viruses such as flaviruses-including the genus flavirus, pestivirus of which Kunjin virus is a member, and hepavirus of which hepatitis C virus is a member, and arbovirus of which the West Nile virus is a member- orthomyxoviruses, paramyxoviruses, arenaviruses, bunyaviruses, herpes viruses, adenoviruses, poxviruses, and retroviruses.

Typical suitable "susceptible viral infections" include influenza A and B viral infections; parainfluenza viral infections, respiratory syncytial virus("RSV") infections such as RSV bronchiolitis and RSV pneumonia especially such RSV infections in children and infants as well as RSV pneumonia in patients with preexisting cardiopulmonary disease, measles viral infections, Lassa fever viral infections, Korean Haemorrhagic fever infections, hepatitis B viral (HBV) infections, Crimean Congo-Haemorrhagic and HCV infections and HIV-1 infections, encephalitis infections such as caused by West Nile virus or Kunjin virus or the St. Louis encephalitis infections as well as viral infections found in immunocompromised patients. Other susceptible viral infections are disclosed in USP 4,211,771 at column 2, line 21 to column 3 line 37; doses and dose regimens and formulations are disclosed at column 3, line 4 to column 9, line 5; see also Canadian Patent No. 1,261, 265. Sidwell, R.W., et al. Pharmacol. Ther., 1979, Vol 6 pp 123-146 discloses that the in vivo antiviral experiments conducted with ribavirin generally confirm one broad-spectrum antiviral activity seen in vitro and states that the efficacy of ribavirin is quite dependent upon the site of infection; the manner of treatment; the age of the animal and the virus dosage utilized. Tables 4 and 5 on page 127 list the RNA and DNA virus infections significantly inhibited in vivo by ribavirin.

The *in vitro* inhibitory concentrations of ribavirin are disclosed in Goodman & Gilman's "The Pharmacological Basis of Therapeutics", Ninth Edition, (1996) McGraw Hill, NY, at pages 1214-1215. The Virazole product information discloses a dose of 20 mg/mL of Virazole aerosol for 18 hours exposure in the 1999 Physicians Desk Reference at pages 1382 -1384.

Ribavirin dosage and dosage regimens are also disclosed by Sidwell, R.W., et al. Pharmacol. Ther 1979 Vol 6. pp123-146 in section 2.2 pp 126-130. Fernandes, H., et al., Eur. J. Epidemiol., 1986, Vol 2(1) pp1-14 at pages 4-9 disclose dosage and dosage regimens for oral, parenteral and aerosol administration of ribavirin in various preclinical and clinical studies.

The term "patients having hepatitis C infections" as used herein means any patient-including a pediatric patient- having hepatitis C and includes treatment-naive patients having hepatitis C infections and treatment-experienced patients having hepatitis C infections as well as those pediatric, treatment-naive and treatment-experienced patients having chronic hepatitis C infections.

These patients having hepatitis C include those who are infected with multiple HCV genotypes including type 1 as well as those infected with, e.g., HCV genotypes 2, 3, 4, 5 and/or 6 and other possible HCV genotypes.

The term "treatment-naive patients having hepatitis C infections" as used herein means patients with hepatitis C who have never been treated with ribavirin or any interferon, including but not limited to interferon-alfa, or pegylated interferon alfa.

The term" treatment-experienced patients having hepatitis C infections" as used herein means patients with hepatitis C who have been treated with ribavirin or any interferon, including but not limited to interferon-alfa, or pegylated interferon alfa, including relapsers and non-responder.

The term "relkapsers" as used herein means treatment-experienced patients with hepatitis C who have relapsed after initial response to previous treatment with interferon alone, or in combination with ribavirin.

The term "non-responders" as used herein means treatment-experienced patients with hepatitis C who have not responded to prior treatment with any interferon alone, or in combination with ribavirin.

When the pegylated interferon-alfa administered is a pegylated interferon alfa-2b, the therapeutically effective amount of pegylated interferon alfa-2b administered during the treatment in accordance with the present invention, including in first and second treatment time periods, is in the range of about 0.1 to 9.0 micrograms per kilogram of pegylated interferon alfa-2b administered per week, in single or divided doses, preferably once a week (QW) or twice a week(BIW), preferably in the range of about 0.1 to about 9.0 micrograms per kilogram of pegylated interferon alfa-2b administered once a week (QW) or in the range of about 0.05 to about 4.5 micrograms per kilogram of pegylated interferon alfa-2b administered twice a week(BIW), or is in the range of about 0.5 to about 3.0 micrograms per kilogram of pegylated interferon alfa-2b administered per week, preferably in the range of about 0.5 to about 3.0 micrograms per kilogram of pegylated interferon alfa-2b administered once a week (QW) or in the range of about 0.25 to about 1.5 micrograms per kilogram of pegylated interferon alfa-2b administered twice a week, or is in the range of about 0.75 to about 1.5 micrograms per kilogram of pegylated interferon alfa-2b administered per week, most preferably is in the range of about 0.75 to about 1.5 micrograms per kilogram of pegylated interferon alfa-2b administered once a week or about 0.375 to about 0.75 micrograms per kilogram of pegylated interferon alfa-2b administered twice a week.

When the pegylated interferon-alfa administered to pediatric patients is a pegylated interferon alfa-2b, the therapeutically effective amount of pegylated interferon alfa-2b administered during the treatment in accordance with the present invention is in the range of about 0.1 to 9.0 micrograms per kilogram of pegylated interferon alfa-2b administered per week, in single or divided doses, preferably once a week (QW) or twice a week(BIW), more preferably about 0.1 to about 9.0 micrograms per kilogram of pegylated interferon alfa-2b administered once a week (QW), or about 0.05 to about 4.5 micrograms per kilogram of pegylated interferon alfa-2b administered per week, in single or divided doses, preferably once a week (QW) or twice a week(BIW), more preferably about 0.05 to about 4.5 micrograms per kilogram of pegylated interferon alfa-2b administered once a week, or preferably about 0.75 to about 3.0 micrograms per kilogram of pegylated interferon alfa-2b administered in single or divided doses, preferably once a week (QW) or twice a week(BIW), more preferably about 0.75 to about 3.0 micrograms per kilogram of pegylated interferon alfa-2b administered once a week or about 0.375 to about 1.5 micrograms per kilogram of pegylated interferon alfa-2b administered twice a week, and most preferably about 2.25 to about 2.6 micrograms per kilogram of pegylated interferon alfa-2b administered once a week or about 1.1 to about 1.3 micrograms per kilogram of pegylated interferon alfa-2b administered twice a week(BIW).

When the pegylated interferon-alfa administered is a pegylated interferon alfa-2a, the therapeutically effective amount of pegylated interferon alfa-2a administered in accordance with the present invention, is in the range of about 50 micrograms to about 500 micrograms once a week("QW"), preferably about 150 micrograms to about 250 micrograms QW or the effective amount is in the range of about 50 micrograms to about 250 micrograms twice a week, preferably about 100 micrograms to about 125 micrograms twice a week.

When the pegylated interferon-alfa administered to a pediatric patient is a pegylated interferon alfa-2a, the therapeutically effective amount of pegylated interferon alfa-2a administered in accordance with the present invention, is in the range of about 50 micrograms to about 500 micrograms once a week("QW"), preferably about 300 micrograms to about 375 micrograms QW or the therapeutically effective amount of pegylated interferon alfa-2a administered to a pediatric patient is in the range of about 50 micrograms to about 250 micrograms twice a week, preferably about 150 micrograms to about 190 micrograms once a week

The esters of ribavirin represented by formulas I-VIII are administered to the patient having chronic HCV in association with pegylated interferon-alfa, that is, before, after or concurrently with the administration of the pegylated interferon alfa. The pegylated interferon-alfa dose is preferably administered during the same period of time that the patient receives doses of esters of ribavirin represented by formulas I-VIII. The amount of esters of ribavirin represented by formulas I-VIII administered concurrently with the pegylated interferon-alfa is from about 200 to about 1600 mg per day, preferrably about 300 to about 1200 mg/day or about 400 to about 800 mg day and most preferably about 400 to about 600 mg a day. The pegylated interferon-alfa dose is also preferably administered to the pediatric patient during the same period of time that such patient receives doses of the esters of ribavirin represented by formulas I-VIII. The amount of the 5'-amino acid esters of ribavirin represented by formulas III-VIII administered to the pediatric patient having chronic HCV concurrently with the interferon-alfa is from about 1 to about 30 mg per kilogram per day, preferrably from about 4 to about 15 mg per kilogram per day, more preferrably about 6, 8 or 15 mg per kilogram per day, most preferrably about 8 to 10 mg per kilogram per day in divided doses.

Pegylated interferon-alfa formulations are not effective when administered orally, so the preferred method of administering the pegylated interferon-alfa is parenterally, preferably by sub-cutaneous(SC), intravenous(IV), or intramuscular(IM) injection. The 5'-amino acid esters of ribavirin represented by formula I may be administered orally in capsule, tablet, or liquid form, intranasally as an aerosol by nasal spray or parenterally, preferably by SC, IV, or IM injection. The esters of ribavirin represented by formulas I-VIII may be orally administered in association with the parenteral administration of pegylated interferon-alfa Of course, other types of administration of both medicaments, as they become available, are contemplated, such as transdermally, by suppository, by sustained release dosage form, and by pulmonary inhalation. Any form of administration will work so long as the proper dosages are delivered without destroying the active ingredient

The term " interferon-alfa " as used herein means the family of highly homologous species-specific proteins that inhibit viral replication and cellular proliferation and modulate immune response. Typical suitable interferon-alfas include, but are not limited to, recombinant interferon alfa-2b, such as Intron-A interferon available from Schering Corporation, Kenilworth, N.J., recombinant interferon alfa-2a, such as Roferon interferon available from Hoffmann-La Roche, Nutley, N.J., recombinant interferon alpha-2c, such as Berofor alpha 2 interferon available from Boehringer Ingelheim Pharmaceutical, Inc., Ridgefield, CT., interferon alpha-n1, a purified blend of natural alfa interferons, such as Sumiferon available from Sumitomo, Japan or as Wellferon interferon alpha-n1 (INS) available from the Glaxo-Wellcome Ltd., London, Great Britain, or a consensus alpha interferon, such as those described in U.S. Patent Nos. 4,897,471 and 4,695,623 (especially Examples 7, 8 or 9 thereof) and the specific product available from Amgen, Inc., Newbury Park, CA, or interferon alfa-n3 a mixture of natural alfa interferons made by Interferon Sciences and available from the Purdue Frederick Co., Norwalk, CT., under the Alferon Tradename. The use of interferon alfa-2a or alpha 2b is preferred. Since interferon alpha 2b, among all interferons, has the broadest approval throughout the world for treating chronic hepatitis C infection, it is most preferred. The manufacture of interferon alpha 2b is described in U.S. Patent No. 4,530,901.

The term "pegylated interferon alfa" as used herein means polyethylene glycol modified conjugates of interferon alfa, preferably interferon alfa-2a and -2b. The preferred polyethylene-glycol-interferon alfa -2b conjugate is PEG₁₂₀₀₀-interferon alfa 2b. The phrases "12,000 molecular weight polyethylene glycol conjugated interferon alpha" and "PEG₁₂₀₀₀-IFN alfa" as used herein mean conjugates such as are prepared according to the methods of International Application No. WO 95/13090 and containing urethane linkages between the interferon alfa-2a or -2b amino groups and polyethylene glycol having an average molecular weight of 12000.

The preferred PEG₁₂₀₀₀₋interferon alfa-2b is prepared by attaching a PEG polymer to the epsilon amino group of a lysine residue in the IFN alfa-2b molecule. A single PEG₁₂₀₀₀ molecule is conjugated to free amino groups on an IFN alfa-2b molecule via a urethane linkage. This conjugate is characterized by the molecular weight of PEG₁₂₀₀₀ attached. The PEG12000-IFN alfa-2b conjugate is formulated as a lyophilized powder for injection. The objective of conjugation of IFN alfa with PEG is to improve the delivery of the protein by significantly prolonging its plasma half-life, and thereby provide protracted activity of IFN alfa.

Other pegylated interferon alfa conjugates can be prepared by coupling an interferon alfa to a water-soluble polymer. A non-limiting list of such polymers include other polyalkylene oxide homopolymers such as polypropylene glycols, polyoxyethylenated polyols, copolymers thereof and block copolymers thereof. As an alternative to polyalkylene oxide-based polymers, effectively non-antigenic materials such as dextran, polyvinyl pyrrolidones, polyacrylamides, polyvinyl alcohols, carbohydrate-based polymers and the like can be used. Such interferon alta-polymer conjugates are described in U.S. Patent No. 4,766,106, U.S. Patent No. 4,917,888, European Patent Application No. 0 236 987, European Patent Application Nos. 0510 356, 0 593 868 and 0 809 996 (pegylated interferon alfa-2a) and International Publication No. WO 95/13090.

Pharmaceutical composition of pegylated interferon alfa suitable for parenteral administration may be formulated with a suitable buffer, e.g., Tris-HCl, acetate or phosphate such as dibasic sodium phosphate/monobasic sodium phosphate buffer, and pharmaceutically acceptable excipients, e.g., sucrose, carriers, e.g., humanor recombinant plasma albumin, tonicity agents, e.g. NaCl, preservatives, e.g., thimerosol, cresol or benyl alcohol, and surfactants, e.g., tweens or polysorabates in sterile water for injection. The pegylated interferon alfa-may be stored as lyophilized powders under a refrigeration at 2°-8°C. The reconstituted aqueous solutions are stable when stored between 2° and 8°C and used within 24 hours of reconstitution. See for example U.S. Patent Nos, 4,492,537; 5,762,923 and 5,766,582.The reconstituted aqueous solutions may also be stored in prefilled, multi-dose syringes such as those useful for delivery of drugs such as insulin. Typical suitable syringes include systems comprising a prefilled vial attached to a pen-type syringe such as the NOVOLET Novo Pen available from Novo Nordisk, as well as prefilled, pen-type syringes which allow easy self-injection by the user. Other syringe systems include a pen-type syringe comprising a glass cartridge containing a diluent and lyophilized pegylated interferon alfa powder in a separate compartment.

A person suffering from chronic hepatitis C infection may exhibit one or more of the following signs or symptoms:
(a) elevated ALT,
(b) positive test for anti-HCV antibodies,
(c) presence of HCV as demonstrated by a positive test for the presence of HCV-RNA in the serum,
(d) clinical stigmata of chronic liver disease,
(e) hepatocelluar damage.

The combination therapy of pegylated interferon-alfa and the esters of ribavirin represented by formulas I-VIII and preferably the 5'-amino acid esters of ribavirin represented by formulas III-VIII may also be administered in association with anti-retroviral therapy, e.g., HAART, to the patient co-infected with the HIV-1 and HCV infection and exhibiting one or more of the above signs or symptoms in amounts sufficient to eliminate or at least alleviate one or more of the signs or symptoms of hte HCV infection, and to lower the HIV-1- RNA and HCV-RNA serum levels each by at least a power of ten, and preferably to eradicate detectable HCV-RNA at least by the end of about 20 to about 50 weeks, preferably at least 24 weeks to 48 weeks and to maintain no detectable HCV-RNA for at least 24 weeks after the end of the about 20 to about 50 weeks. Administration of the 5'-amino acid esters of ribavirin represented by formula I may be discontinued after the end of the second time period depending upon the judgment of the attending clinician.

The term " no detectable HCV-RNA" in the context of the present invention means that there are fewer than 100 copies of HCV-RNA per ml of serum of the patient as measured by quantitative, multi-cycle reverse transcriptase PCR methodology. HCV-RNA is preferably measured in the present invention by research-based RT-PCR methodology well known to the skilled clinician. This methodology is referred to herein as HCV-RNA/qPCR. The lower limit of detection of HCV-RNA is 100 copies/mL. Serum HCV-RNA/qPCR testing and HCV genotype testing will be performed by a central laboratory. See also J. G. McHutchinson et al. (N. Engl. J. Med., 1998, 339:1485-1492), and G. L. Davis et al. (N. Engl. J. Med. 339:1493-1499).

### Biological Activity

The compounds of formulas I -VIII are useful for treating patients having susceptible viral infrections such as chronic hepatitis C as part of a Combination Therapy with interferon-alfa, especially interferon alfa-2b. The compounds of formulas I and II wherein R⁵ is R⁶(W)ₓCO-,R⁶(W)ₓCS, (HO)₂PO-, R⁶(W)ₓ P(OH)O-or HOSO₂-metabolizes *in vivo* into ribavirin.

Compounds of formula I wherein R²= R³=H and R⁵=(HO)₂PO- ,or 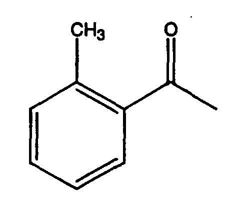 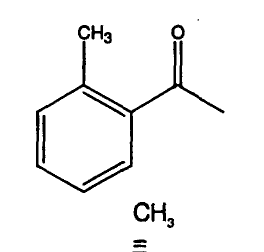 or (CH₃)₃CO-, or C₆H₅CO- or C₆H₅ CH₂OC(O)NH CCO- have produced higher plasma concentration of ribavirin after administration to rats.

**TABLE 1**

| Ribavirin Concentrations of pooled Rat¹ Plasma (2 rats/pool) following **oral administration** of 40 mpk of the compounds of formula in 20% hydroxypropyl-beta-cyclodextrin | | | | |
|---|---|---|---|---|
| Compound² | AUC (pooled)³(ng.hr/mL) | C.V.⁴ (%) | Conc. (6 hr) (ng/mL) | C.V.⁴ (%) |
| A | 1523.65 | 23.56 | 142.54 | 49.37 |
| B | 1309.76 | 1.68 | 241.83 | 19.61 |
| C | 1303.34 | 67.42 | 170.42 | 37.78 |
| D | 1065.88 | 29.12 | 116.33 | 10.73 |
| E | 754.77 | 74.42 | 122.71 | 44.68 |

| | | | | |
|---|---|---|---|---|
| ¹ Male Charles River rats obtained from Charles River, Wilmington, MA 01887 | | | | |
| ² (a) Compound A is the cmpound of formula I wherein R⁵ is (CH₃)₃C CO- (b) Compound B is the compound of formula I wherein R⁵ is (HO)₂P(O)- (c) Compound C is the compound of formula I wherein R⁵ is C₆H₅CO- (d) Compound D is the compound of formula I wherein R⁵ is C₈H₆CH₂OCONH CH(CH₃ )CO- (e) Compound E is Ribavirin | | | | |
| ³ Area under the curve measured from 0 to6 hrs. after oral administration to 2 rats of an aqueous solution of compounds of formula I in 20% HPBCD | | | | |
| ⁴ %CV is percent coefficient of variation which is a relative measure of variability. See Steele and Torrie, "Principles and Procedures of Statistics". (1980) 2nd Edition, McGraw-Hill, NY, atg page 27. | | | | |

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient. Suitable solid carriers are known in the art, e.g. magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington's Pharmaceutical Sciences, 18th Edition, (1990), Mack Publishing Co., Easton, Pennsylvania.

Liquid form preparations include solutions, suspensions and emulsions. As an example may be mentioned water or water-propylene glycol solutions for parenteral injection or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, e.g. nitrogen.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is administered orally.

Preferably, the pharmaceutical preparation is in a unit dosage form. In such form, the preparation is subdivided into suitably sized unit doses containing appropriate quantities of the active component, e.g., an effective amount to achieve the desired purpose.

The quantity of active compound in a unit dose of preparation may be varied or adjusted from about 0.01 mg to about 1000 mg, preferably from about 0.01 mg to about 750 mg, more preferably from about 0.01 mg to about 500 mg, and most preferably from about 0.01 mg to about 250 mg, according to the particular compound and particular application.

The actual dosage employed may be varied depending upon the requirements of the patient and the severity of the condition being treated. Determination of the proper dosage regimen for a particular situation is within the skill of the art. For convenience, the total daily dosage may be divided and administered in portions during the day as required.

The amount and frequency of administration of the compounds of the invention and/or the pharmaceutically acceptable salts thereof will be regulated according to the judgment of the attending clinician considering such factors as age, condition and size of the patient as well as severity of the symptoms being treated. A typical recommended daily dosage regimen for oral administration can range from about 1 mg/kg/day to about 100 mg/kg/day, in two to four divided doses.

In a preferred embodiment of the present invention, those patients co-infected with HIV-1 and HCV infections are treated with pegylated interferon alfa in combination with the preferred 5'-amino acid esters of ribavirin represented by formulas III to VIII and a HAART combination considered appropriate by the attending clinician and the patient.. See also J. G. McHutchinson et al. (N. Engl. J. Med., 1998, 339:1485-1492), and G. L. Davis et al. (N. Engl. J. Med. 1998, 339:1493-1499).

The preferred compounds of formulas III-VIII are useful for treating patients having suceptible viral infections, e.g., chronic hepatitis C. The compounds of formulas III-VIII metabolize *in vivo* into ribavirin and are useful for treating susceptible viral infections treatable with ribavirin, alone, or in combination with other anti-viral therapies, e.g., interferon-alfa and HAART. The treating of patients having chronic hepatitis C with the compounds of formulas III-VIII is performed as part of a combination therapy with interferon-alfa, especially interferon alfa-2b.

Compounds of formulas III VIII metabolize *in vivo* into ribavirin and have produced higher plasma concentrations of ribavirin after oral administration of a compound of formula VII to rats and monkeys compared to administration of ribavirin. The pharmacokinetics of the prefered compounds of formulas VII are presented in Tables 2 & 3

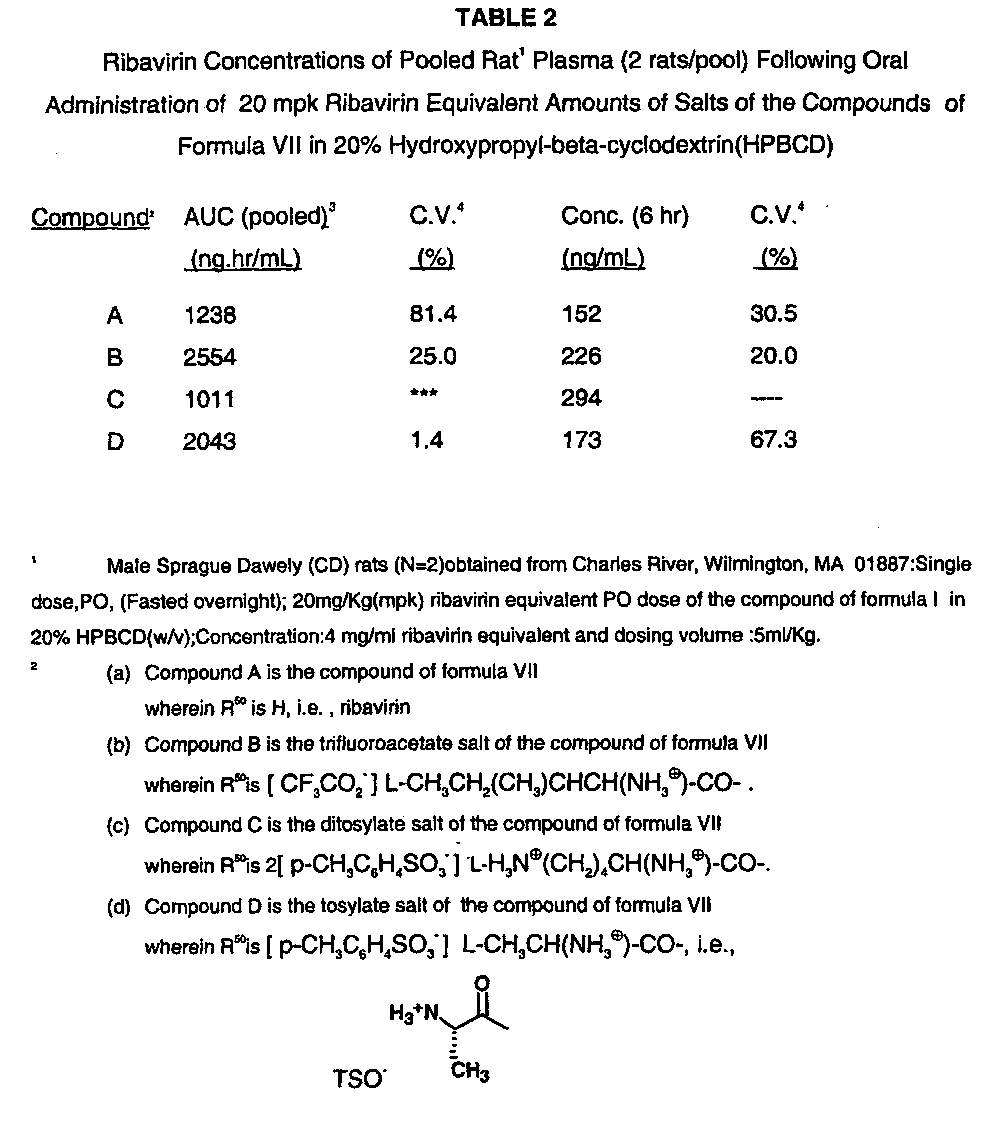

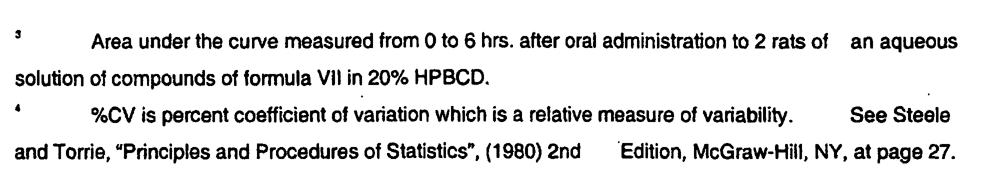

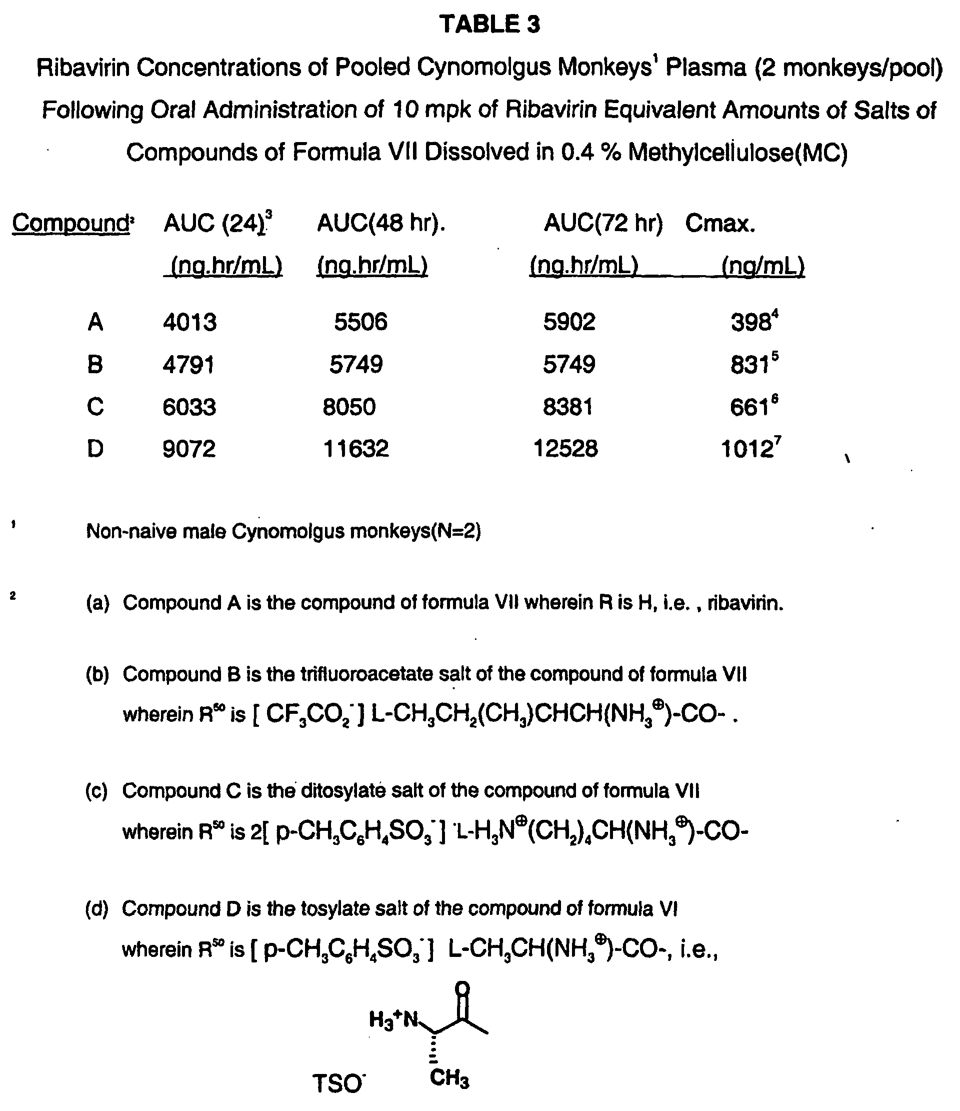

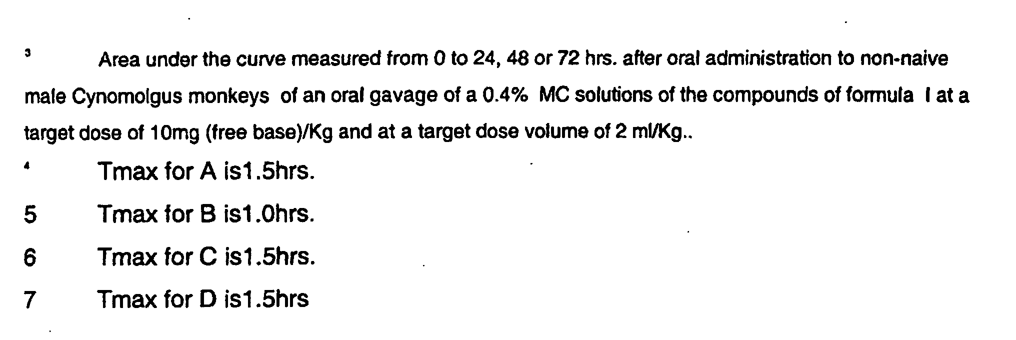

The pharmaceutical compositions of the preferred 5'-amino acid esters of ribavirin of the present invention (represented by formulas III-VIII) may be adapted for any mode of administration e.g., for oral, parenteral, e.g., subcutaneous ('SC"), intramuscular ("IM"), intravenous ("IV") and intraperitoneal ("IP"), topical or vaginal administration or by inhalation (orally or intranasally). Preferably the ribavirin compounds represented by formula I are administered orally.

Such compositions may be formulated by combining a compound of formulas III-VIII or an equivalent amount of a pharmaceutically acceptable salt of compound I with an suitable, inert, pharmaceutically acceptable carrier or diluent which may be either solid or liquid. The compounds of formulas III-VIII are preferably converted into the pharmaceutically acceptable acid addition salts by adding to compounds of formulas III-VIII an equivalent amount (or two equivalents in the case of for example the lysine ester) of a pharmaceutically acceptable acid. Typically suitable pharmaceutically aceptable acids include the mineral acids, e.g.,HNO₃ H₂SO₄, H₃PO₄, HCl, HBr, organic acids,including, but not limited to, acetic, trifluoroacetic, propionic, lactic, maleic, succinic, tartaric, glucuronic and citric acids as well as alkyl or arylsulfonic acids, such as p-toluenesulfonic acid, 2-naphthalenesulfonic acid, or methanesulfonic acid.

Typically suitable pharmaceutically acceptable salts include the following anions: acetate, adipate, besylate (benzenesulfonate), bromide, camsylate[(+)-7,7-dimethyl-2-oxobicyclo[2.2.1]hepatane-1-methanesulfonate], chloride, citrate, edisylate(1,2-ethanedisulfonate), estolate(dodecyl sulfate), fumarate, gluceptate(glucoheptonate), gluconate, glucuronate, hippurate, hyclate(hydrochloride, hemiethanolate), hydrobromide, hydrochloride, iodide, isethionate (2-hydroxyethanesulfonate), lactate, lactobionate, maleate, mesylate (methanesulfonate), methylbromide, methylsulfate, napsylate, nitrate, oleate, pamoate [4,4'-methylenebis[3-hydroxy-2-napthalenecarboxylate]], phosphate, polygalacturonate, stearate, succinate, sulfate, sulfosalicylate, tannate, tartrate, terephthalate, tosylate(p-toluenesulfonate), triethiodide.;
and the following catrons:
benzathine (N,N-bis(phenylmethyl)-1,2-ethanediamine), calcium, diolamine (2,2-iminobis(ethanol), megiumine[1-deoxy-1-(methylamino)-D-glucitol], olamine(2-aminoethanol), potassium, procaine, sodium tromethamine [2-amino-2-(hydroxymethyl)-1,3 propanediol,] and zinc.

The preferred pharmaceutically acceptable salts are trifluoroacetate, tosylate, mesylate, and chloride.

Solid form preparations include powders, tablets, dispersible granules, capsules, cachets and suppositories. The powders and tablets may be comprised of from about 5 to about 95 percent active ingredient. Suitable solid carriers are known in the art, e.g. magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington's Pharmaceutical Sciences, 18th Edition, (1990), Mack Publishing Co., Easton, Pennsylvania.

Liquid form preparations include solutions, suspensions and emulsions. As an example may be mentioned water or water-propylene glycol solutions for parenteral injection..Solid form preparations may be converted into liquid preparations shortly before use for either oral or administration. Parenteral forms to be injected intraveneously, intramuscularly or subcutaneously are usually in the form of sterile solutions and may contain tonicity agents (salts or glucose), and buffers. Opacifiers may be included in oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier, such as an inert compressed gas, e.g., nitrogen.

Also included are solid form preparations that are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably, the pharmaceutical preparation is in a unit dosage form. In such form, the preparation is subdivided into suitably sized unit doses containing appropriate quantities of the active component, e.g., an effective amount to achieve the desired purpose.

The effective amount or therapeutically effective amount of active compound of the present invention (of formulas I- VIII) in a unit dose of preparation may be varied or adjusted from about 1 mg to about 1600 mg per day, preferably from about 1 mg to about 1200 mg per day, or about 300 mg to about 1200 mg per day, more preferably from about 1 mg to about 800 mg per day, or about 400 mg to about 800 mg per day and most preferably from about 1 mg to about 100 mg per day from about 400 mg to about 600 mg per day, in single or divided doses, according to the particular compound and particular application.

The actual dosage employed may be varied depending upon the requirements of the patient and the severity of the condition being treated. Determination of the proper dosage regimen for a particular situation is within the skill of the art. For convenience, the total daily dosage may be divided and administered in portions during the day as required.

The amount and frequency of administration of the compounds of the invention and/or the pharmaceutically acceptable salts thereof will be regulated according to the judgment of the attending clinician considering such factors as age, condition and size of the patient as well as severity of the symptoms being treated. The dose of the preferred compounds of formulas III to VIII should be chosen to provide steady state plasma concentrations of ribavirin in the range of about 0.1 ug/mL to about 100 ug/mL, preferably in the range of about 0.1 ug/mL to about 50 ug/mL, more preferably in the range of about 1 ug/mL to about 3 ug/mL., and most preferably in the range of about 1.8 ug/mL to about 2.6 ug/mL. Plasma ribavirin concentrations may be determined using high pressure liquid chromatographic material with tandem mass spectrometric detection. The method was validated with respect to linearity, selectivity, precision, accuracy and has a limit of quantitation of 50 microg/mL. A typical recommended daily dosage regimen for oral administration can range from about 1 mg/kg/day to about 100 mg/kg/day, in two to four divided doses.
A compound represented by the formula VI 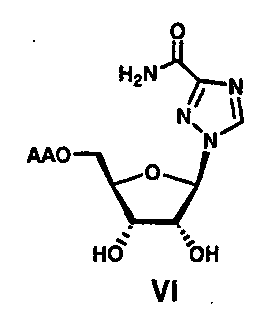 or a pharmaceutically acceptable salt thereof;
wherein AA is a natural or unnatural amino acid moiety represented by the formulas in Table AA herein below:

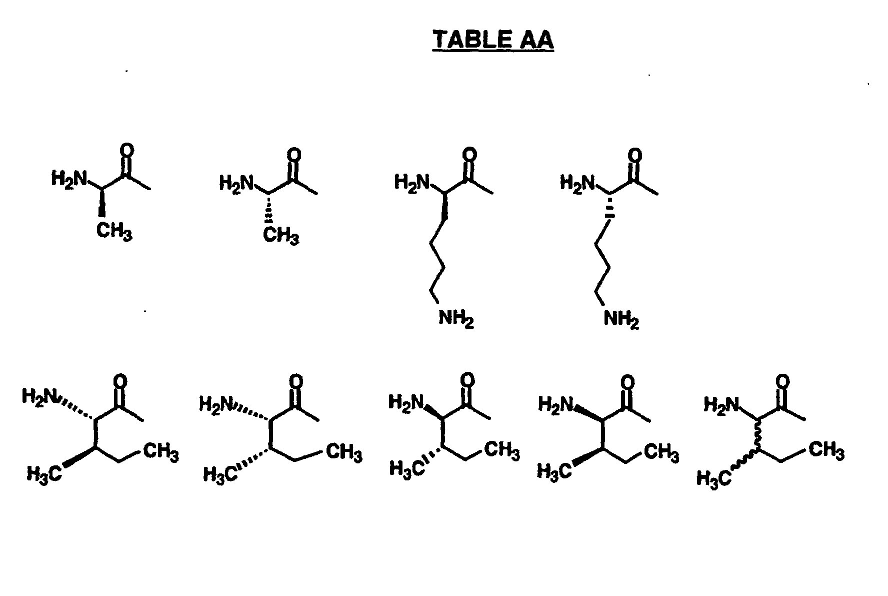

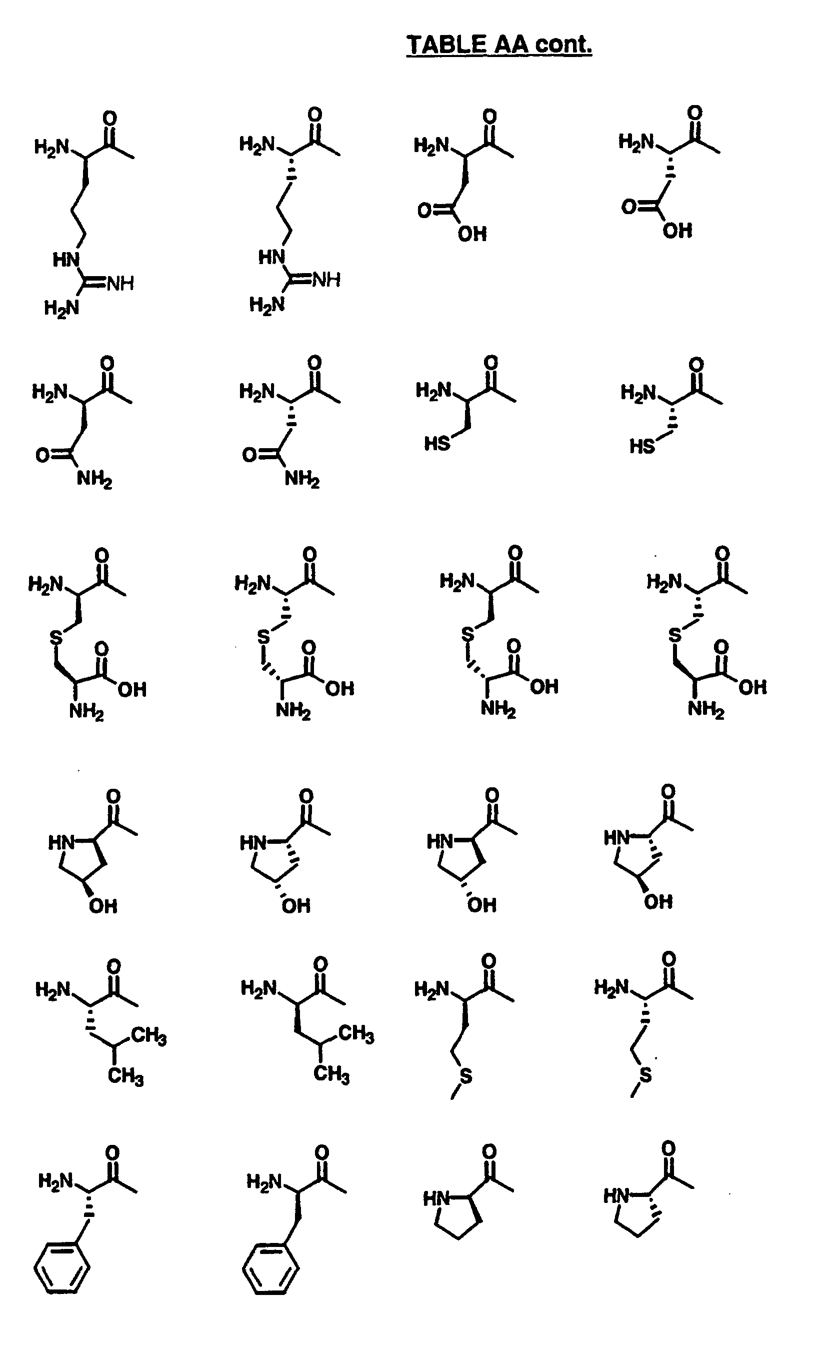

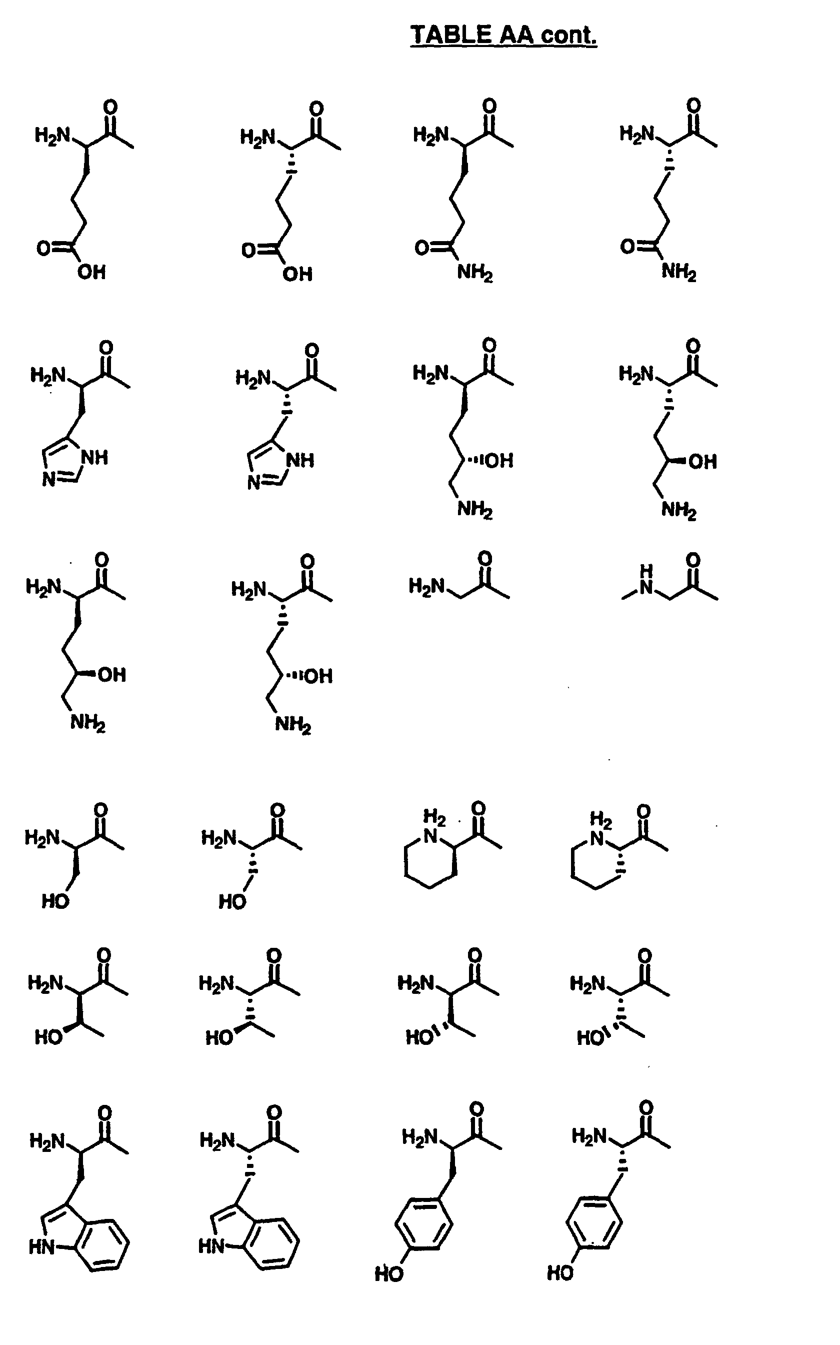

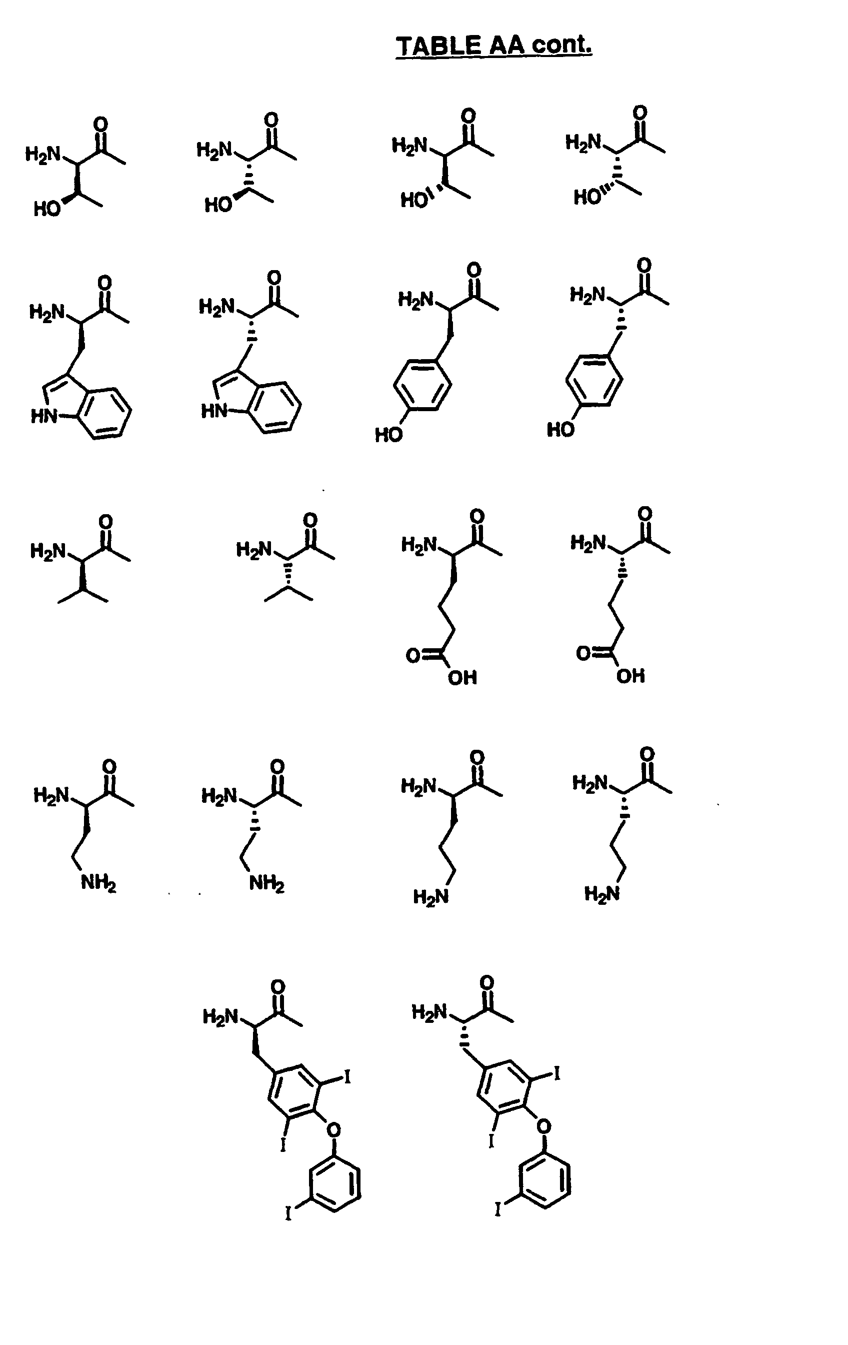

The present invention concerns compounds represented by the formula VII: 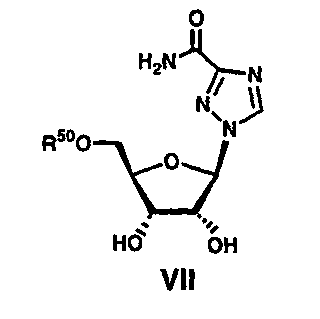 wherein R⁵⁰ is CH₃CH(NH₂)-CO-, CH₃CH₂(CH₃)CHCH(NH₂)-CO- or H₂N(CH₂)₄CH(NH₂)-CO- ; or stereoisomers thereof ,or a pharmaceutically acceptable salt thereof.
R may be CH₃CH(NH₂)-CO-, i.e., α-aminopropanoyl, in the form of a stereoisomeric mixture, i.e. the DL-form of α-aminopropanoyl - or as one of the enantiomers ,i.e., 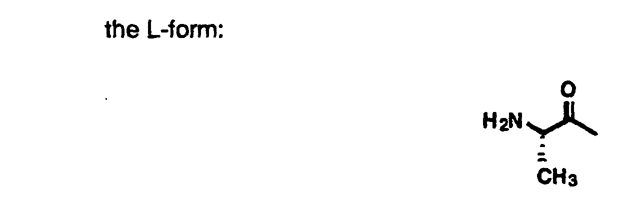 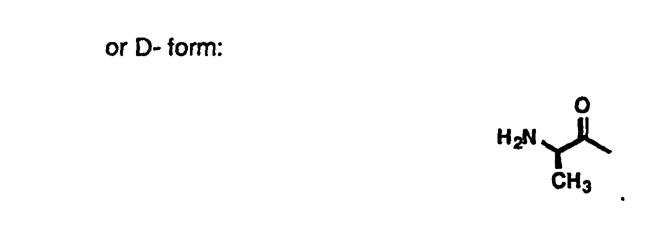 or mixtures thereof.
R may be CH₃CH₂(CH₃)CHCH(NH₂)-CO-, i.e., 2-amino-3-methylpentanoyl, -
(a) as a stereoisomeric mixture, i.e., the DL-Form-DL-(-+) erythro-2 -amino-3-methylvaleroyl- of the formula: 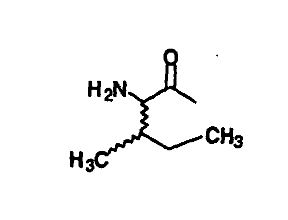
or (b) as one of the diastereomers of the formula, i.e.,
   the L-allo- form of the formula: 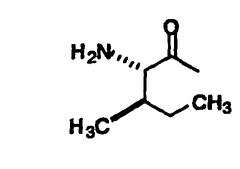 [ ( +)-threo-2-amino-3-methylpentanoyl];
   or the L-form of the formula: 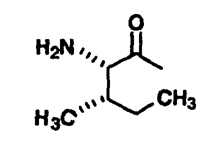 [ L-(+)-amino-3-methylpentanoyl, or (2S,3S)-2-- amino-3-methylpentanoyl ];
   or mixtures thereof;
or (c) as one of the diastereomers of the formula: 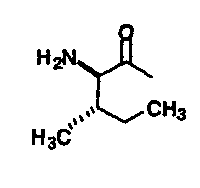 [ (2R,3S)-2--amino-3-methylpentanoyl ]; or 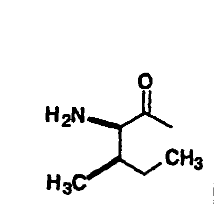 [ (2R,3R)-2--amino-3-methylpentanoyl ]; or mixtures thereof.

R may be H₂N(CH₂)₄CH(NH₂)-CO- in the form of a stereoisomeric mixture, or as one of the enantiomers ,i.e., the L-form of the formula: 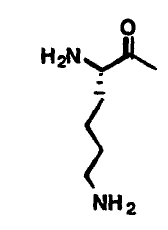 or, the D- form of the formula:

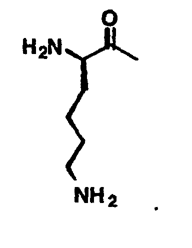

Preferably, R is L-α aminopropanoyl of the formula:

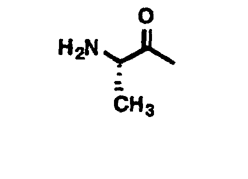

### General Synthetic Preparation

Ribavirin, 1-β-D-ribofuranosyl-1H-1, 2,4-triazole-3-carboxamide, available from ICN Pharmaceuticals, Inc., Costa Mesa, California, is described in the Merck Index, compound No. 8199, Eleventh Edition. Its manufacture and formulation is described in U.S. Patent No. 4,211,771.

The ribavirin derivatives of formulas I -VIII may be prepared by use of the sequences of steps illustrated in the following Schemes, and in the Examples using the compounds listed hereinafter the Schemes.

In Scheme I, compounds of formula I wherein R⁵=R^{5a}CO-,R³=R^{3a}CO and R²=H and R⁵=R^{5a}CO-and R²=R^{2a}CO-and R³=H and R⁵=R^{5a}CO-and R³=R²=H, are prepared. Compound 110 (ribavirin) and benzaldehyde are treated with ZnCl₂ in excess benzaldehyde as solvent at ambient temperature (20 to 25°C) for 24 hrs. to give compound 111. Treatment of 111 with the alkanoyl chloride R^{5a}COCl in the presence of base e.g. triethylamine ("TEA"); or with the carboxylic acid (R^{5a}COOH) and triethylamine and coupling reagent such as dicyclohexylcarbodiimide ("DCC") produces compound 112. Removal of the acetal protecting group with trifluoroacetic acid:water (9:1 ,v/v) at ambient temperature for 0.25-2 hrs, preferably about 0.05 hrs. provides compound 113. Compound 113 is converted into a mixture of compounds 114 and 115 by treatment of 113 with R^{3a} COOH, base and DCC or R^{3a}COCI and base, e.g., TEA.

Compounds 114 and 115 may be separated by standard chromatographic techniques to provide pure compounds 114 and 115.

In Scheme II, compounds of formula 1 wherein R⁵=R³=H and R²-R^{2a}CO-are prepared from compound 110 (ribavirin). Compound 110 is treated with 1,3-dichloro-1,1,3,3-tetraisopropyldisiloxane, i.e., [(i-Pr)₂SiCl]₂O, in DMF as solvent in the presence of imidazole for 1-4 hrs. at ambient temperature to give compound 116. Treatment of 116 with R^{2a}CO Cl and base e.g., TEA or R^{2a}COOH, base and a coupling reagent e.g., DCC for 12-48 hrs. at ambient temperature provides 117. Treatment of 17 with Bu₄NF in tetrahydrofuran ("THF") at ambient temperature for 1-10 hrs. provides compound 118.

Scheme III illustrates the preparation of the compounds of formula wherein R⁵=R²=H and R³=R^{3a}CO- and R⁵=R³=H and R²=R^{2a}CO- Ribavirin is treated with trityl chloride or [MeOC₆H₄(C₆H₅)₂]CCOCl and base, e.g., pyridine in a solvent DMF at ambient temperature for 6-24 hrs. to provide 119. Treatment of 119 with R^{2a} COCl and base or R^{2a} COOH, base and a coupling reagent, e.g., DCC, provides a mixture of compounds 120 and 121. The mixture is separated into the pure compounds by standard chromatographic techniques. Treatment of 120 or 121 with paratoluenesulfonic acid ("p-TsOH") in methanol in the presence of hydrogen and a palladium on charcoal catalyst at ambient temperature for 2-48 hrs. removes the protecting group to give 122 and 123, respectively. p-TsOH salt thereof.

Scheme IV illustrates preparation of the compounds of formula I wherein R⁵=R^{5a}CO- or R⁵=R^{5a} and R³=R²=H. Treatment of ribavirin 110 with R^{5a}COON=C(CH₃)₂ in the presence of an enzyme, such as Novo SP 435 lipase at 65°C in a solvent such as THF or dioxane for 12-48 hrs selectively adds R^{5a}CO to the form compound 124. See also Examples 9 to 14.

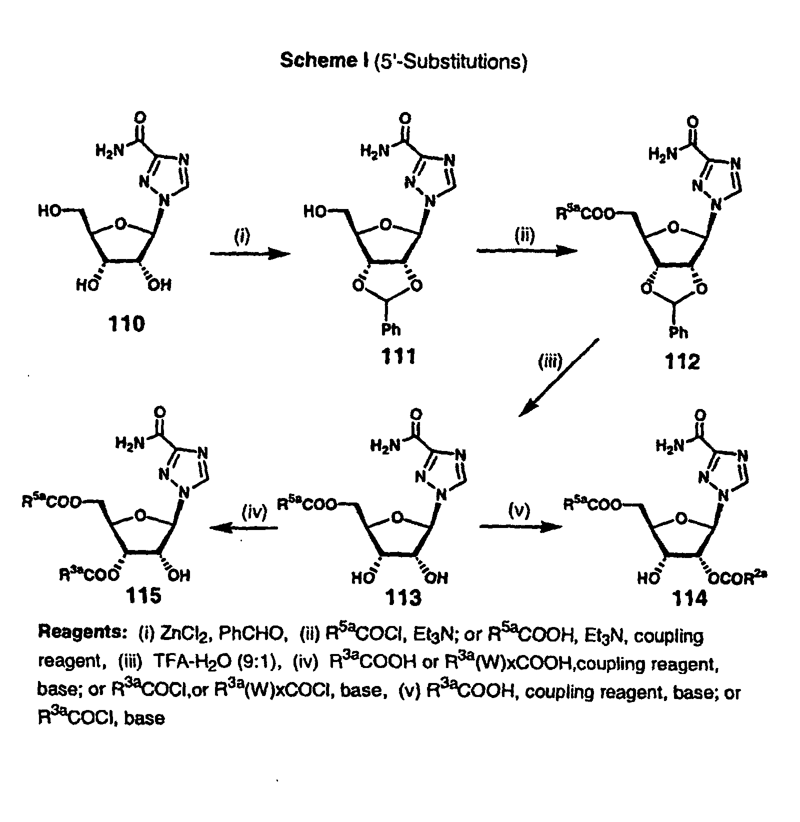

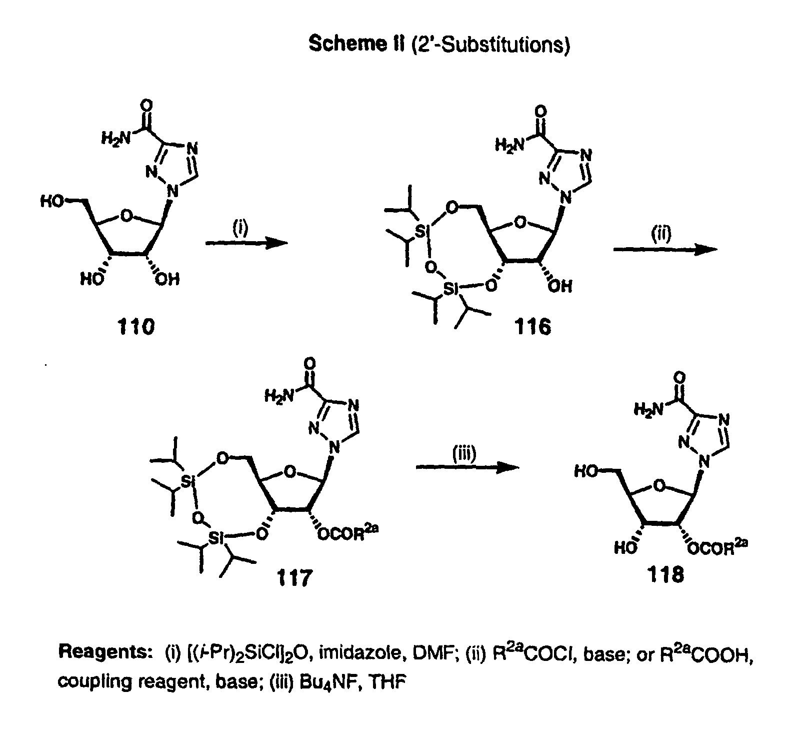

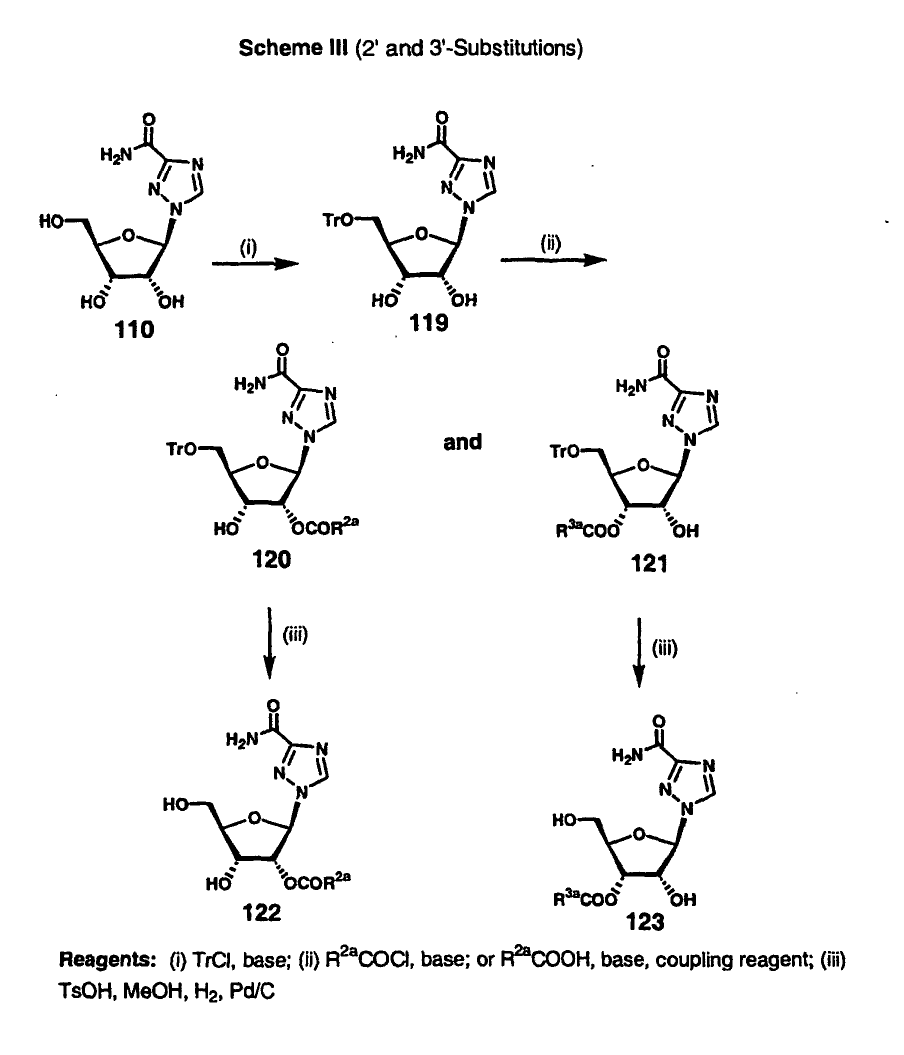

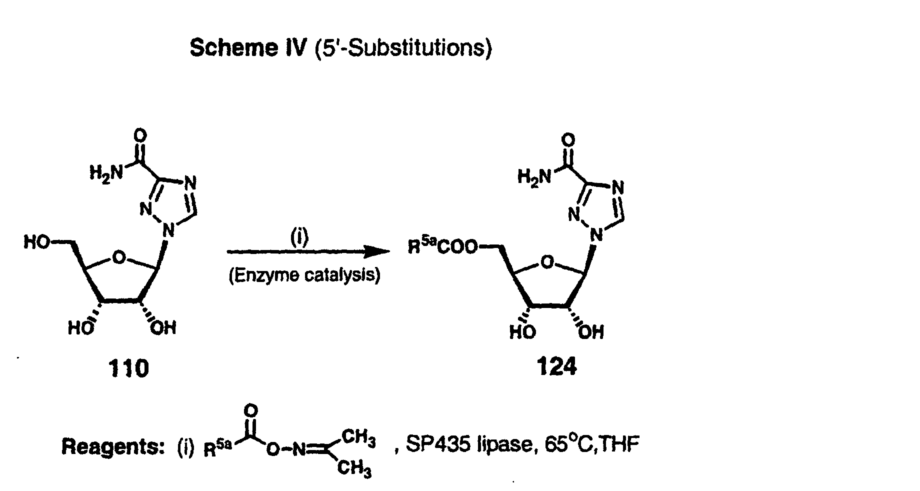

Examples for all schemes: 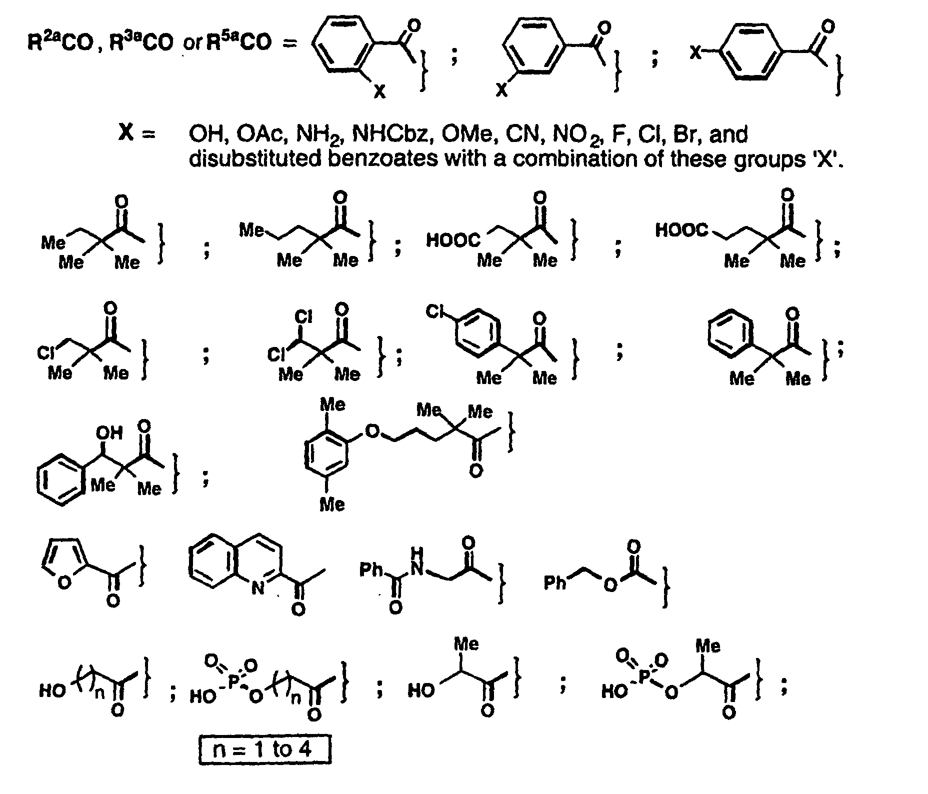 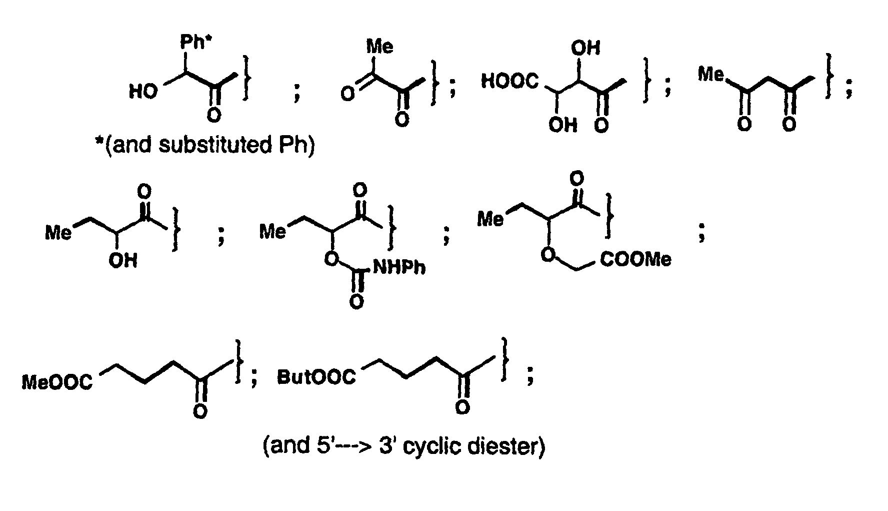 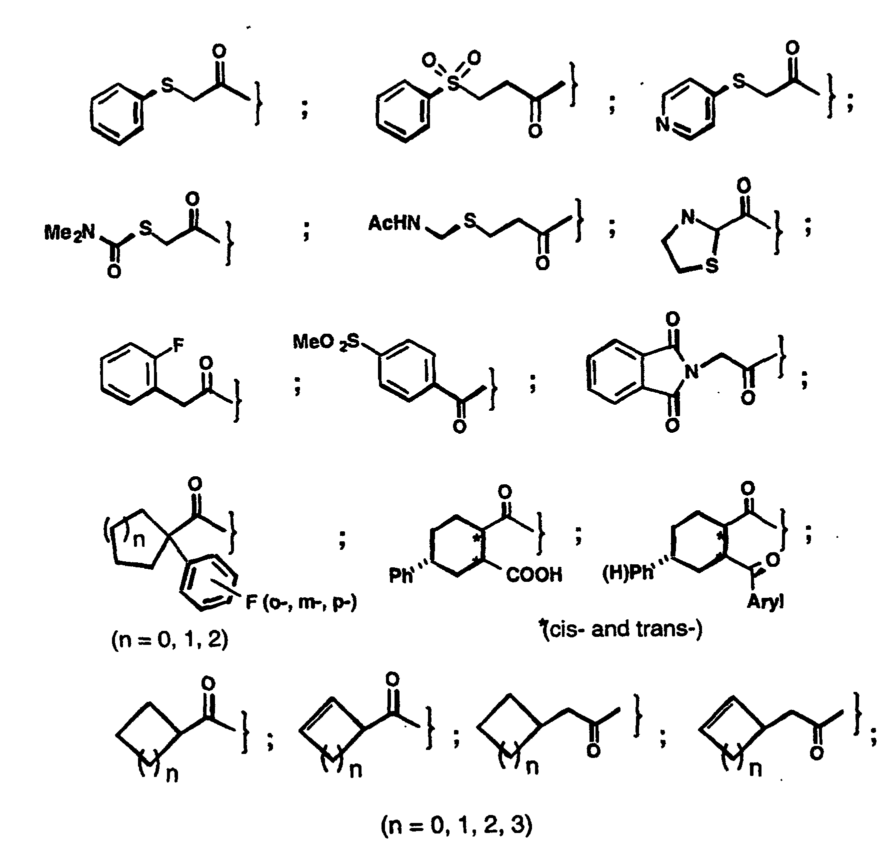 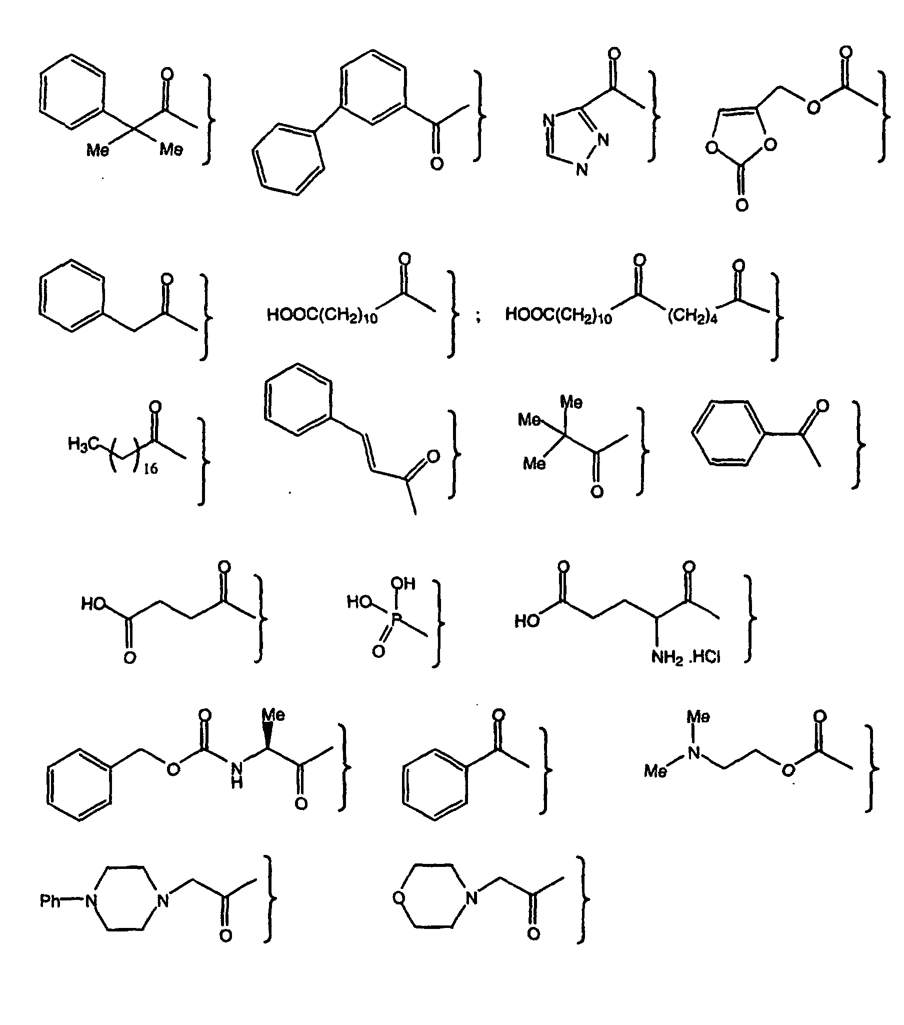 R^{2a}CO, R^{3a}CO, and R^{5a}CO may also be represented by the formula: 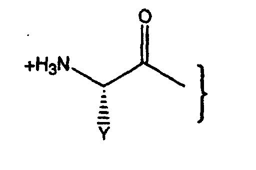 Y = H, CH₃; CH₃CH₂-; CH₃CH₂CH₂-; Me₂CH-; Me₂CH₂CH₂-; CH₃CH₂CH(Me)-PhCH₂-; HOOCCH₂CH₂-; HSCH₂-; HOOCCH₂-; MeSCH₂CH₂-; HOCH₂-; 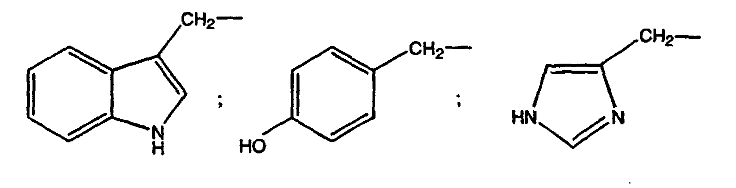 or Y is H₂N(CH₂)₄- or CH₃CH(OH)-;
or Y taken together with the α carbon and N form 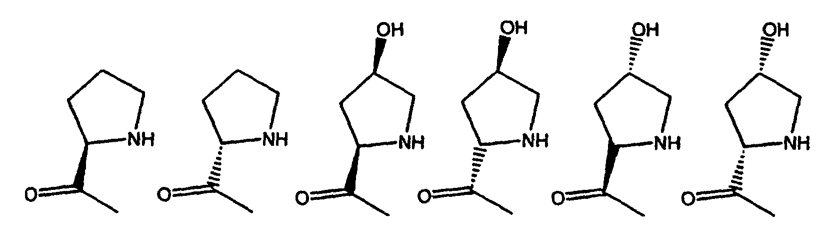 or a pharmaceutically acceptable salt thereof.

Examplary 5'- heteroaryl esters of Formulas I& II wherein R⁵ = 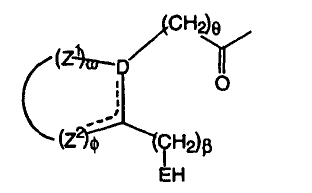 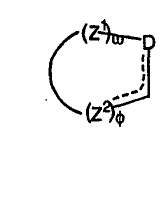 represents a heteroaryl ring of 5-7 total atoms at maximum unsaturation wherein:
- D =: C, or when θ > 0 is N or S
- E=: O or NH
- Z¹ and Z² =: independently -C(H)=, -C(H)=N-, -N=, -N(H)-, S, S-C(H)= or S-N=
- θ and β =: independently 0, 1, or
- ω and φ =: independently 1, 2, or 3. The 5'-ester of formula is represented by the formula:

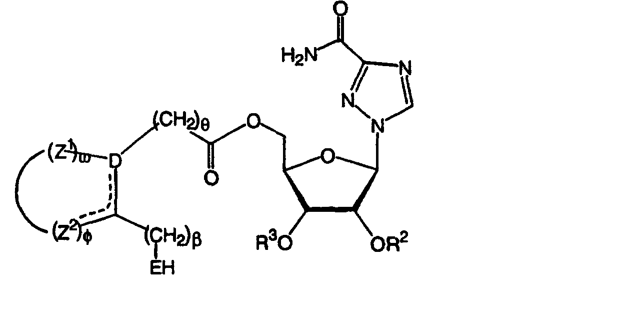

The following 5' esters may be prepared as described in Schemes hereinabive, using appropriately protected acids which are readily obtained by procedures well known to one skilled in the art.

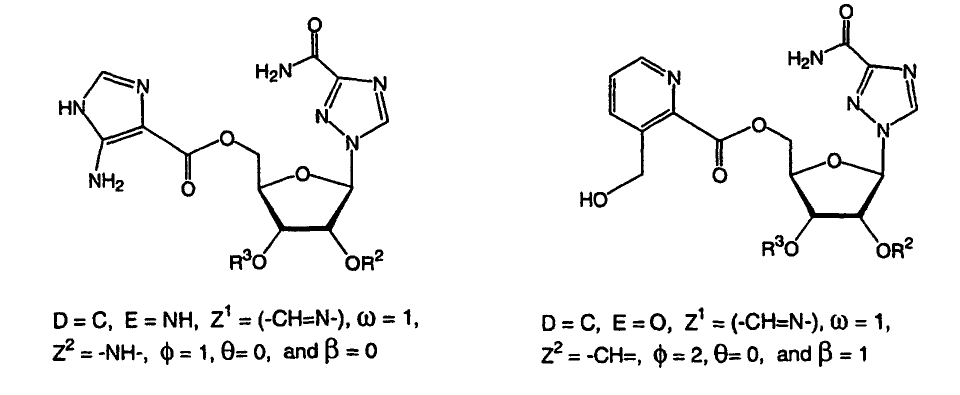

The folowing 5'-3' cyclic ester (Formula I wherin R²=H)may be prepared using the Schemes herein above and readily available starting materials. 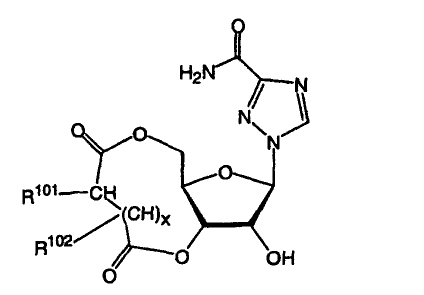 wherein R¹⁰¹ and R¹⁰² are
independently H,
alkanoyloxy,OR^{7b} or NR⁶R^{7b}and
x=1 or 2

The following 3'-2' cyclic esters(formula I wherein R⁵=H) may be made using the Schemes herein above and readily available starting materials.

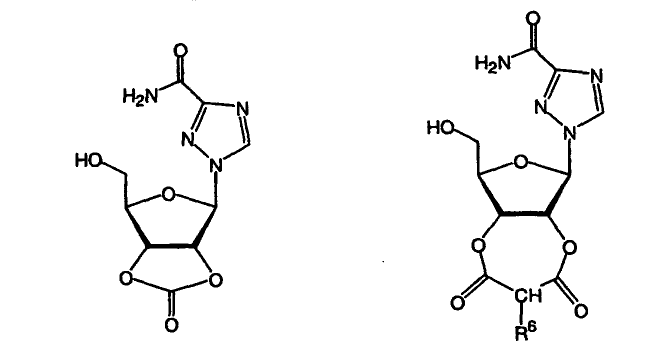

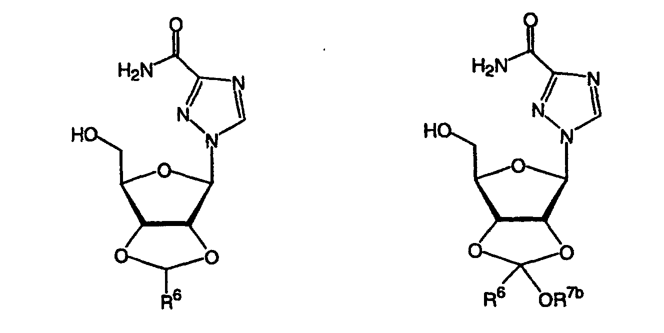

### Example I

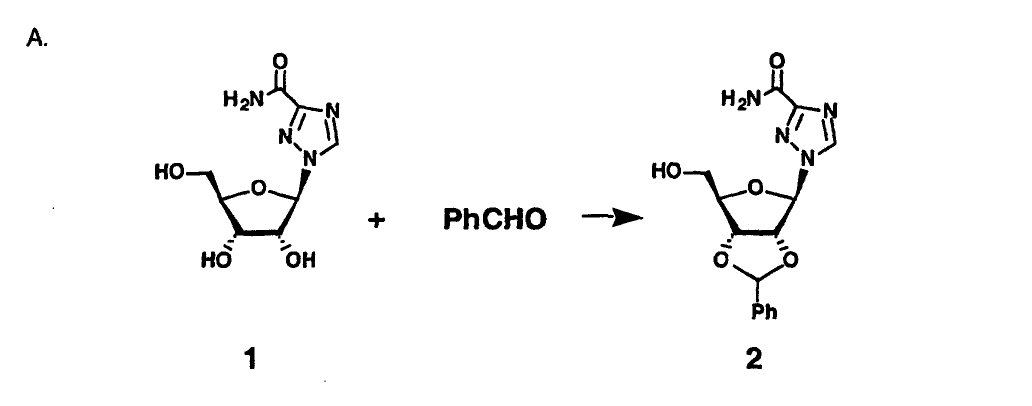

### A.-Benzylidene ribavirin

Combine 20 g ribavirin (1, 87 mmol), 200 ml of benzaldehyde, and 20 g of ZnCl₂. Stir the so-formed reaction mixture at ambient temperature for 24 hours. Pour the resulting solution, with stirring, into 2.5 L of ethyl ether (Et₂O). Suction-filter the resulting mixture and dry the solid precipitate. Mix the solid precipitate with 1.2 L of ice-cold 2N sodium hydroxide (NaOH) solution. Extract the mixture with 2×0.75L of cold ethyl acetate (EtOAc) and wash the organic layer with brine. Gravity-filter the organic layer through fluted filter paper, then concentrate it in vacuo to leave a solid. Triturate the solid thoroughly with 0.5 L of Et₂O, suction-filter and wash the so-formed preciptate with fresh Et₂O to leave 23 g of compound 2 as a solid; Calc. for C₁₅H₁₆N₄O₅ (332.32). MS(FAB) = 333.1.

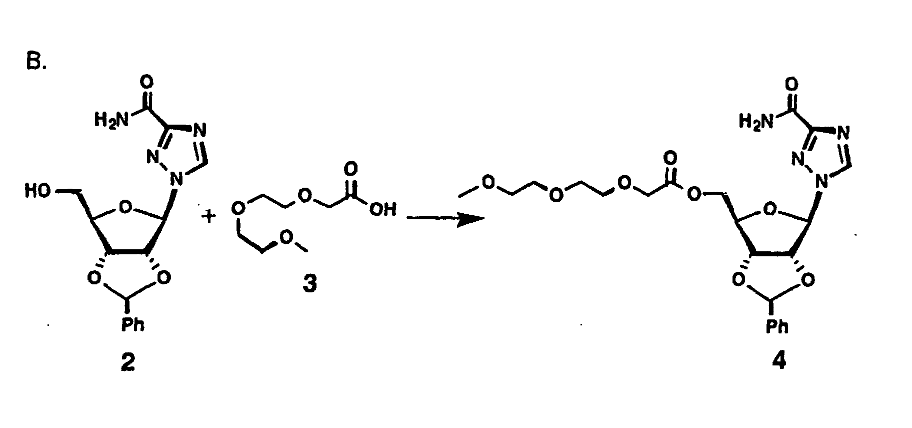

Combine 0.5g (2.8 mmol) of compound 2 from step A,0.80g(2.4 mmol) of compound 4 [MeO(CH₂CH₂O)₂CH₂CO₂H] and 0.8 g (1.2 mmmol) of 4-(N,N,dimethylamino)pyridine (DMAP) in 15 ml of N,N,-dimethylformamide (DMF). Add 2.5 ml of a 1M solution of dicyclohexylcarbodiimide (DCC) in dichloromethane (CH₂Cl₂), and stir the resulting mixture at ambient temperature for 0.5 hour. Heat the so-formed reaction mixture at 100 °C for 1 hour. Quench the reaction mixture with aqueous 5% KH₂PO₄, and extract it with 2x100 ml of ethyl acetate (EtOAc). Wash the organic extract with 20 ml cold water, then with saturated brine. Gravity-filter the organic extract through fluted filter paper, then concentrate the filtrate *in vacuo* to leave a gum residue. Purify the residue by column chromatography (silica gel, gradient of 1% to 6% methanol-CH₂Cl₂) to give 0.48 g of compound **4**; Calc. for C₂₂H₂₈N₄O₉ (492.49); MS(FAB) = 493.1.

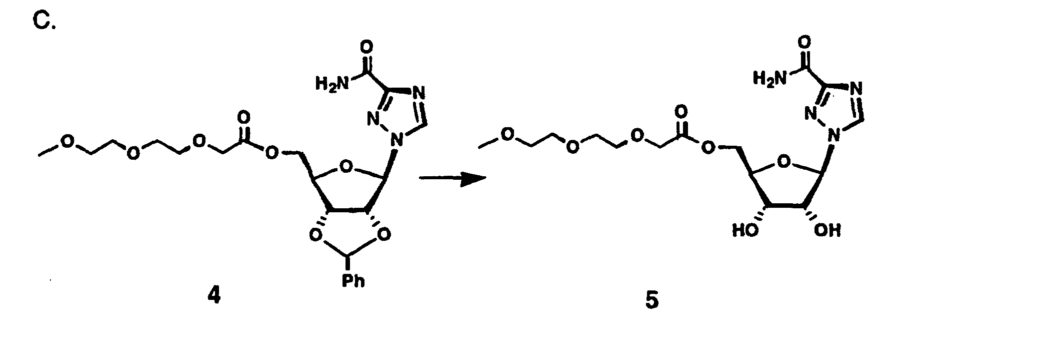

C.Treat 0.45 g 4 (0.91 mmol) with 10 ml of trifluoroacetic acid (TFA)-water (9:1 v/v) at ambient temperature for 0.5 hour. Quench with 30 ml xylene, then concentrate the so-formed mixture *in vacuo* to leave a gum. Triturate the gum with EtOAc, dilute with Et₂O, then suction-filter and dry the gum to leave 0.23 g of compound 5 as a solid, Calc. C₁₅H₂₄N₄O₉ (404.38); MS(FAB) = 405.1.

### Example 2

Follow the procedures of Example 1A and 1 B except substitute an equivalent amount of the compound 6 (MeOCH₂CH₂OCHCO₂H) for compound 3 in step B to form compound 8. Follow the procedures of step C of Example 1 except substitute an equivalent amount of compound 7 for compound 4 to form the compound 8.

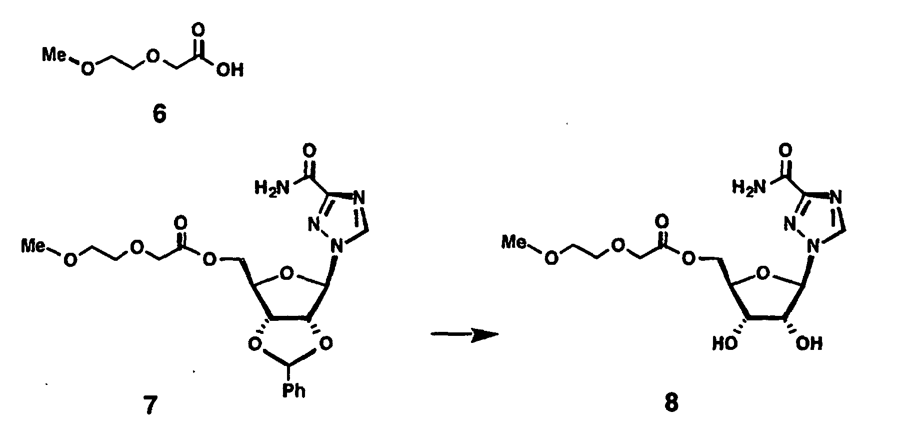

### Example 3

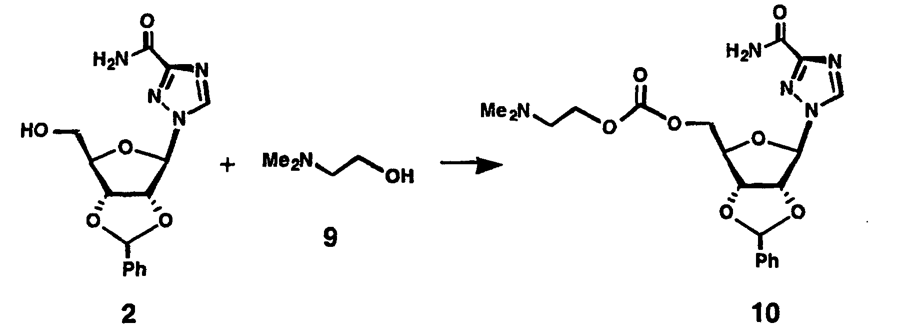

A.Treat a solution of 0.32 g (3.6 mmol) of compound 9 (2-(N,N'-dimethytamino)ethanol] in 10 mL of N,N,-dimethylformamide (DMF) at 5°C with 0.58 g carbonyldiimidazole (3.6 mmol), and allow the so-formed solution to warm to 20°C over 0.5 hr. Add to the resulting reaction mixture 0.8 g (2.4 mmol) of compound 2 prepared in accordance with Example 1 A and stir the so-formed reaction mixture at ambient temperature for 24 hr. Concentrate the mixture *in vacuo* add 50 mL of ethyl ether, and allow the so-formed mixture to set for 24 hr. Decant the supernatant solution, and purify the residue by column chromatography (silica gel, gradient of 10% to 20% methanol-tetrahydrofuran) to give 0.21 g of compound 10; calc. C₂₀H₂₅N₅O₇ (447.44). MS(FAB) = 448.1.

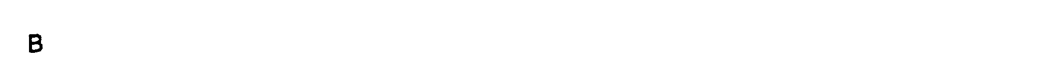

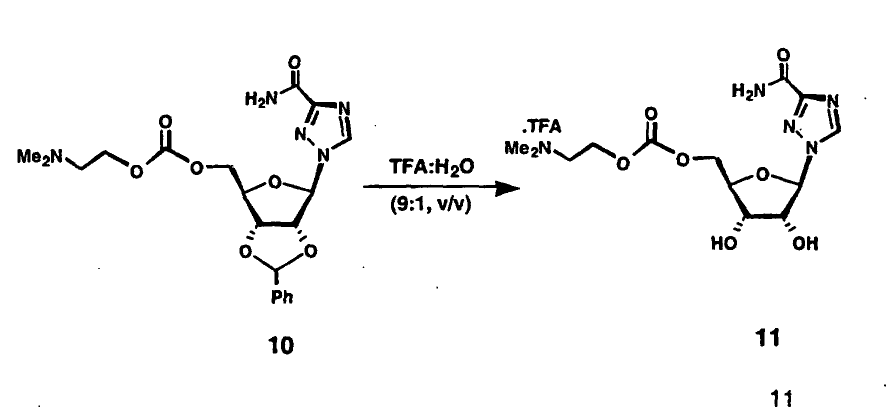

Follow the procedures of Example 1 C except substitute an equivalent amount of compound 10 for compound 4 to obtain compound 11.

### Example 4

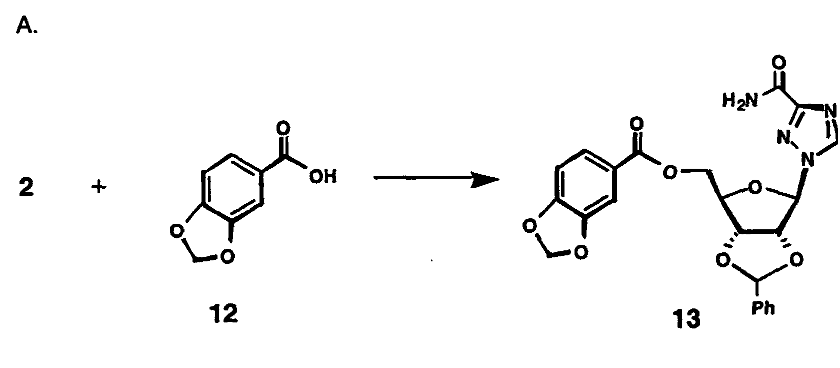

A stirred, dry DMF (5mL) solution of 500 mg (1.506 mmol) of compound 2 prepared in accordance with the procedures of Example 1A, 300 mg (1.2 eq) of piperonylic acid (compound 12) was treated at ambient temperature with 732 mg (1.1 eq) of benzotriazolyloxytris-(dimethylamino)phosphonium hexafluorophosphate ("BOP reagent") and 576µL (2.2 eq) of Hunig's base, i.e., -diisopropylethyl amine, [(i-Pr)₂NEt], under a nitrogen atmosphere. The so-formed reaction mixture was stirred overnight at ambient temperature. Thin layer chromatography (TLC) showed one major new spot. The reaction mixture was quenched with an aqueous NH₄Cl solution and diluted with EtOAc. The organic layer was washed with water and brine and then dried over Na₂SO₄. The organic solvent was evaporated to produce a crude purple solid. The crude solid was purified on a silica gel chromatography column with 2-3% MeOH/CH₂Cl₂ as an eluent. The appropriate fractions were combined to give 550 mg of compound 13 as a purple-tinted solid (76% yield). The 'H nmr spectrum (350 MHz in CDCl₃) was consistent with the structure of 13.

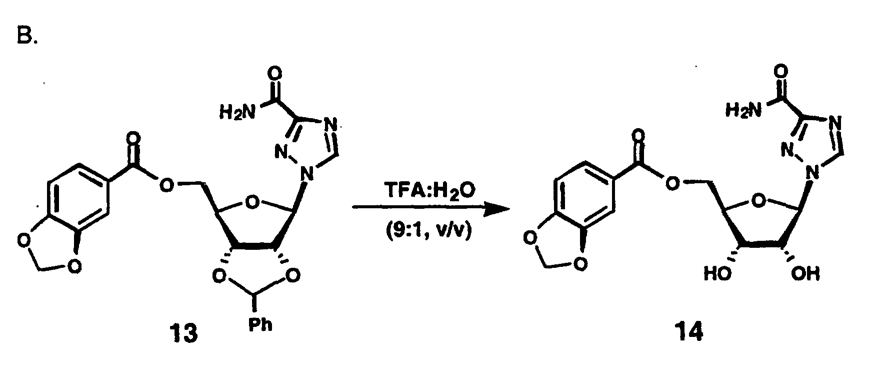

Compound 13 from step A was treated with 4 mL of TFA:H₂O (9:1,v/v) for 40 min. at ambient temperature. The solvents were evaporated to give a yellow solid. Water was added and evaporated. The crude solid was dried overnight. To the crude product was added 2 x 4 mL of MeOH. The MeOH layer was yellow. The insoluble material was collected and dried to give 342 mg of compound 14 as a white solid. The 'H nmr spectrum was consistent with the structure of 14.

### Example 5

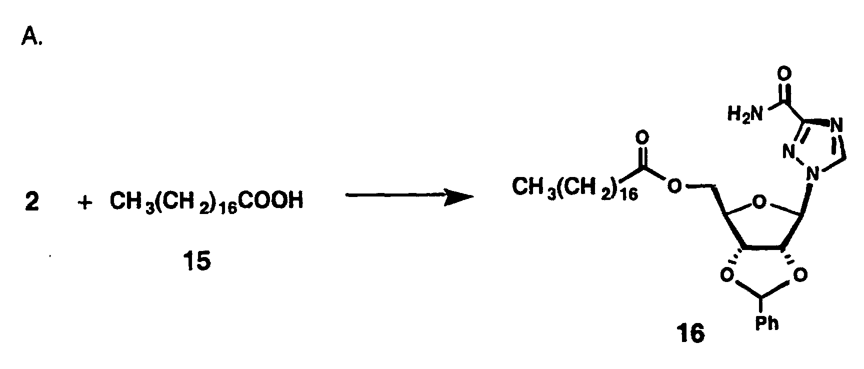

To a stirred solution of 500 mg (1.505 mmol) of 2 prepared in accordance with Example 1 A in 3 mL of dry DMF at ambient temperature was added 516 mg (1.2 eq) of stearic acid (compound 15), 670 mg (1.0 eq) of the BOP reagent and 792 µL (3.0 eq) of Hunig's base. The so-formed reaction mixture was stirred overnight at ambient temperature. The reaction mixture was isolated in accordance with the procedures of Example 4A to give 595.5 mg of compound 16 as a viscous white solid (66% yield). The 'H nmr spectrum was consistent with the structure of 16.

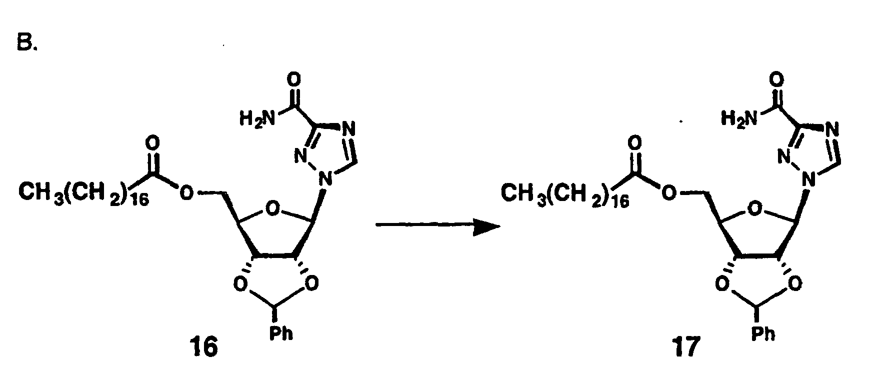

Compound 16 (417 mg, 0697 m mol) was treated in accordance with the procedures of Example 4B to give 330 mg of compound 17 as a white solid (93% yield). The 'H nmr spectrum and MS-FAB were consistent with the structure of 17.

### Example 6

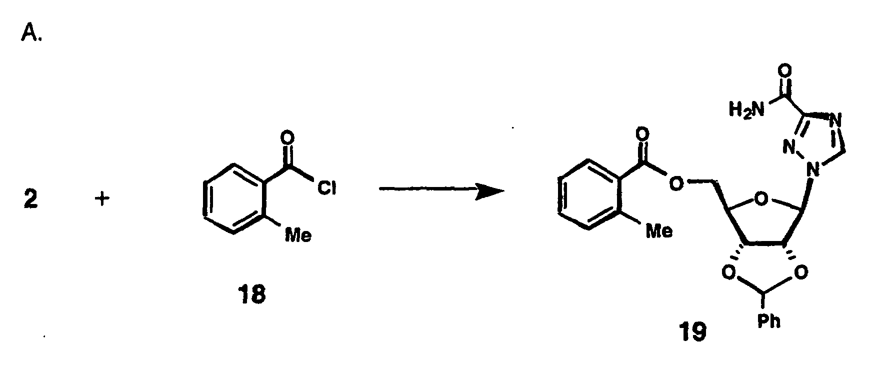

To a stirred suspension of 332 mg (1.0 mmol) of compound 2 prepared in accordance with Example 1A in 5 ml of dry CH₂Cl₂ was added 209 µL (1.5 eq) of triethylamine (Et₃N) and 167 µL (1.2 eq) of o-toluoyl chloride (compound18). TLC showed completion of the reaction after the reaction mixture had been stirred at ambient temperature for 4 hours. The so-formed clear reaction mixture was diluted with EtOAc and quenched with water. The separated organic layer was washed with water and brine, dried and evaporated to give a crude white solid. The crude solid was purified on silica gel column chromatography elueting with 2-3% MeOH to give 262 mg of compound 19 as a white solid (57% yield).

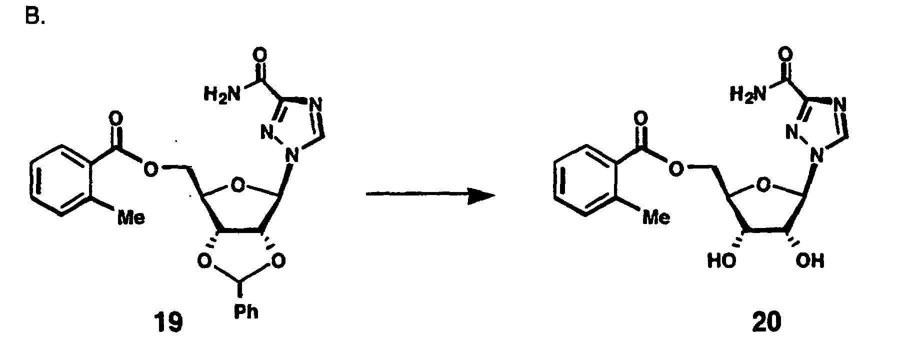

Compound 19 (262 mg) was treated with 3 mL of TFA:H₂O (9:1 v/v) in accordance with the procedure of Example 4A to give 210 mg (99% yield) of the compound 20. The 'H nmr spectrum was consistent with the structure of compound 20.

### Example 7

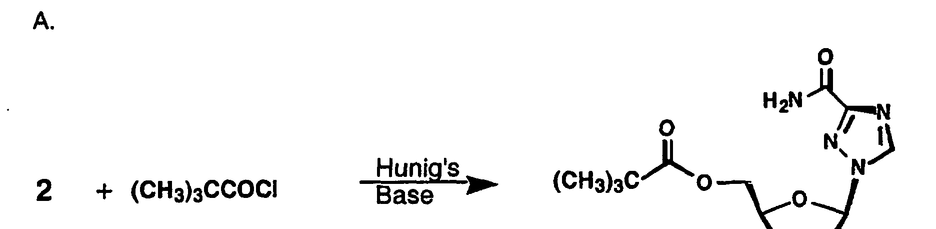

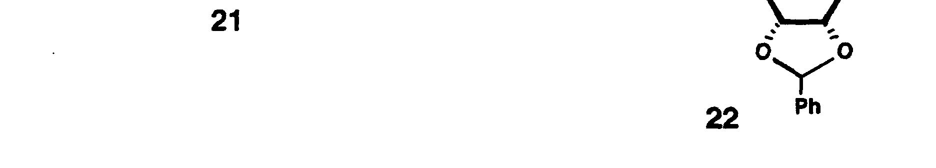

To a stirred suspension in a reaction flask immersed in an ice bath of 498 mg (1.5 m mol) of 2 prepared in accordance with Example 1 A in 10 mL of dry CH₂Cl₂ was added 146 µL (1.80 mmol, 1.2 eq) of pyridine and 203 µL (1.65 mmol, 1.1 eq) of trimethylacetyl chloride (compound 21) at 0°C. The ice bath was removed after 5 min. Stirring was continued for 30 min. and 1 mL of DMF was added; the mixture changed from a suspension to a cloudy solution. By TLC, there was a substantial amount of unreacted starting material (compound 2). The reaction mixture was stirred overnight, and an additional amount of compound 21 (203 µL,1.1 eq) and 180 mg (1.5 eq) of DMAP were added. The so-formed reaction mixture was stirred for 4 days at ambient temperature. There was still some unreacted starting material(compound2) by TLC. The reaction mixture was worked-up by routine extraction between H₂O/EtOAc. The crude product was purified by silica gel column chromatography eluting with 2-5% MeOH/CH₂Cl₂ to give 411.3 mg of compound 22 (66% yield) as a white solid.

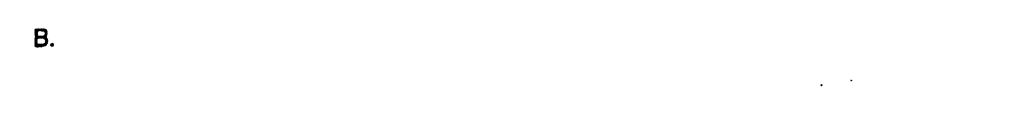

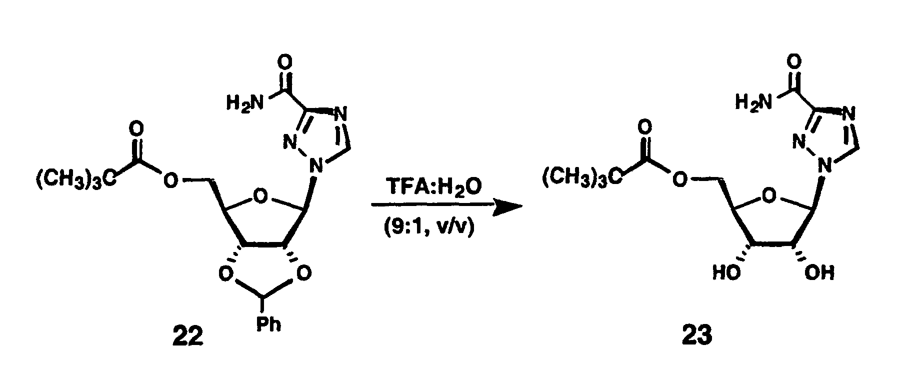

Compound 22 (316 mg) was treated with 4 mL of TFA:H₂O (9:1 v/v) in accordance with the procedures of Example 4B to give 265 mg of compound 23.

### Example 8

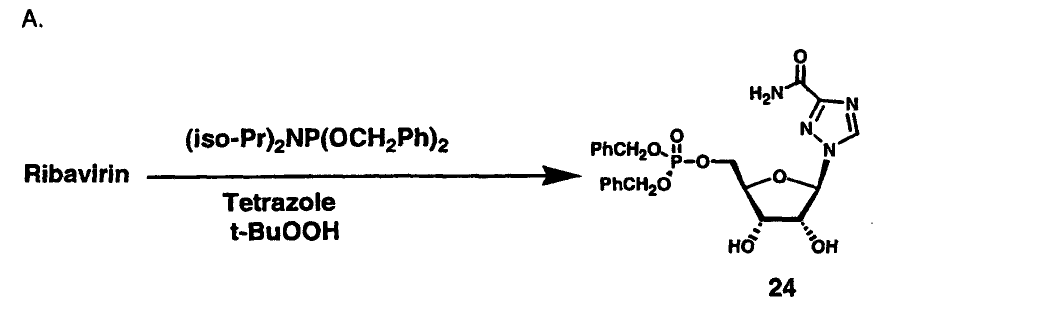

To a stirred mixture of 500 mg (2.1 m mol) of ribavirin and 2.07 g (1.04 mL, 1.5 eg, 3.1 mmol) of N,N-disopropyl-dibenzyl phosphoramide in 5 mL of DMF at room temperature, was added 433 mg (6.1 m mol, 3 eq) of tetrazole.The resulting reaction mixture was stirred at room temperature for 2 hrs., and then 1.11 mL (3 eq) of tert-butylperoxide (5.5M in decane) were added. The so-formed reaction mixture was stirred for 2 hrs. Water was added and the organic layer was washed, dried and evaporated to give a crude product. The crude product was purified on silica gel column chromatography using MeOH/CH₂Cl₂ as an eluent to give 493 mg of compound 24 as white solid. FAB:MS:MH⁺=505.2.

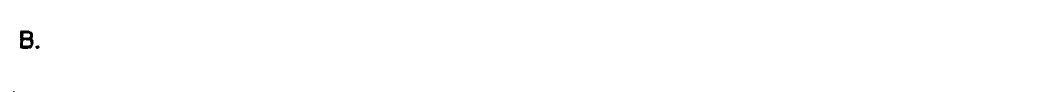

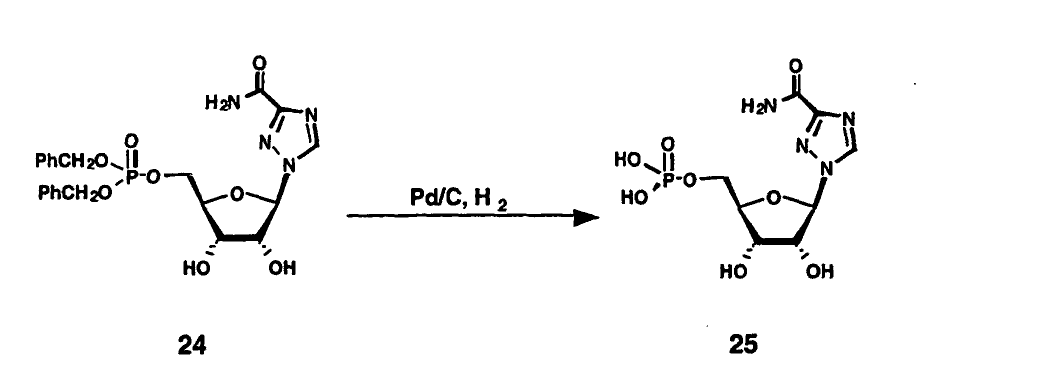

A suspension of compound 24 (278 mg) in a mixture of 3mL of MeOH and 3 mL of water and 150 mg of 10% Pd on carbon was placed under an atmosphere of hydrogen for 4 hrs. The suspension was filtered through a pad of celite and the solid was washed thoroughly with MeOH. The combined filtrates were concentrated under reduced pressure to give 169.3 mg of the compound 25 as a white solid. FAB-MS, MH*=325.1

### Example 9

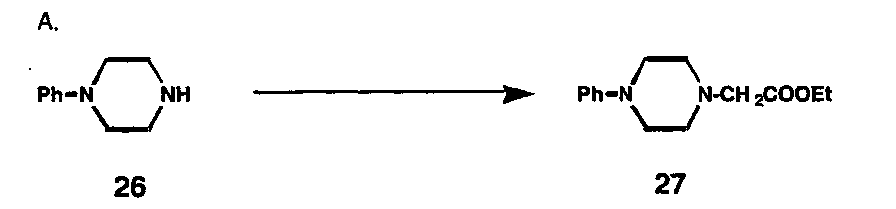

An oil dispersion of NaH (0.88g of NaH, 0.022 mmol, 1.1 eq) was washed once with dry hexane and then suspended in 10 mL of dry THF. Compound 26 (N-phenylpiperazine, 3.24g, 20mmol) was added to the reaction vessel containing NaH in dry THF immersed in an ice bath The ice bath was removed and the so-formed reaction mixture was stirred for 1 hr. at room temperature. To the stirred white reaction mixture was added 2.66 mL (0.024 m mol, 1.2 eq) of ethyl bromoacetate. The so-formed reaction mixture was stirred overnight. The reaction mixture was cooled to ice bath temperatuare and quenched with an aqueous NH₄Cl solution. The so-formed mixture was extracted with EtOAc and the organic layer was washed with aqueous NH₄Cl brine and dried. The solvent was removed at reduced pressure to provide a crude product which was purified on a silica gel chromatography column using 20% EtOAc/hexane (v/v) as an eluent to provide 2.10g of compound 27 (ethyl N-phenylpiperazinylacetic acid) as a clear oil. The 'H nmr spectrum was consistent with the structure of compound 27.

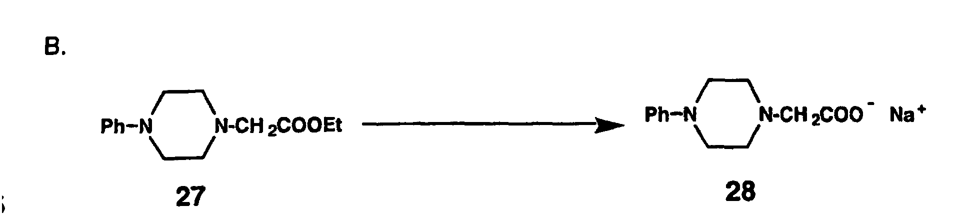

To a stirred solution of 2.10g (8.47 m mol) of compound 27 in 8.47 mL of MeOH was added 8.47 mL (1.0 eq) of a 1N NaOH solution. The so-formed reaction mixture was stirred for 3 hrs. at room temperature (no compound 27 was found by TLC). The solvents were removed under reduced pressure to provide a crude product. Water was added to the crude product and the solution was freeze dried overnight to provide 2.10 g of compound 28 as a white solid. The 'H nmr spectrum was consistent with the structure of compound 28.

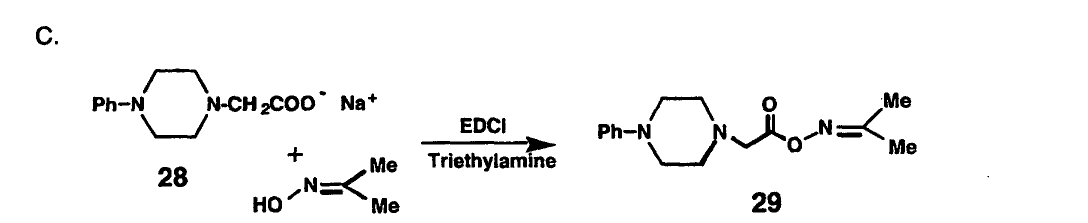

To a stirred solution of 1.21 g (5.0 m mol) of compound 28 in a mixture of 15 mL of CH₃CN and 5 mL of DMF was added 1.146g (6.0 m mol, 1.2 eq) of EDC. HCl followed by 837 µL (6.0 m mol, 1.2 eq) of Et₃N and 731 mg (10.0 mmol, 2 eq) of acetone oxime (compound 28). The so-formed reaction mixture was stirred overnight and then diluted with EtOAc. The organic layer was washed with water and brine, dried and concentrated to give a crude product. The crude product was purified on silica gel column chromatography using 20% EtOAc/hexane (v/v) as an eluant to give 237 mg of compound 29 as a white solid. The 'H nmr spectrum of the oxime ester was consistent with the structure of compound 29.

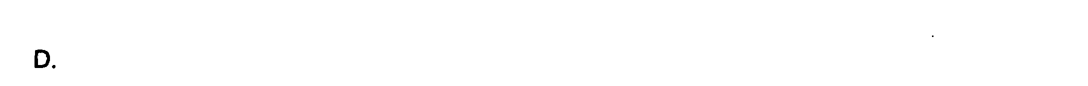

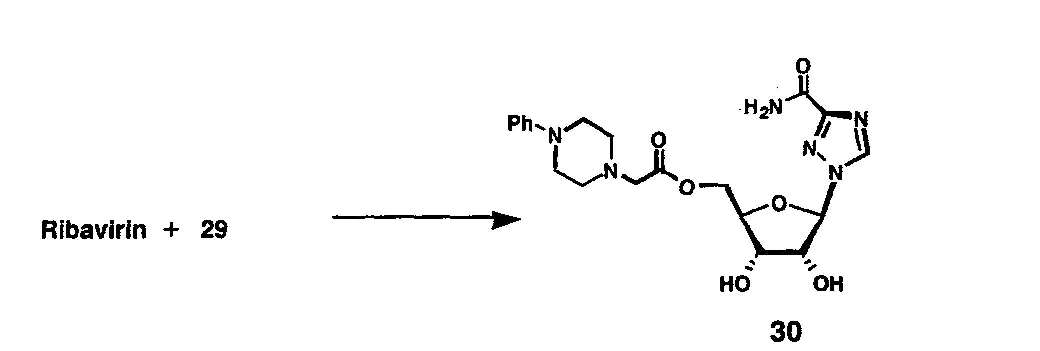

A suspension of 105.0 mg (0.431 m mol) of ribavirin 237 mg (0.862 m mol) of oxime ester 29 and 0.1 g of Novo SP435 lipase *(Candida antarctica)* in 5 mL of anhydrous THF was stirred at 65°C for 24 hrs. The so-formed reaction mixture was cooled to room temperature, filtered and washed with MeOH. The crude material was purified on silica gel column chromatography using 10% MeOH/CH₂Cl₂ (v/v) as eleunt to produce 66 mg of Compound 30 (a white solid) as a single product (in 42% yield). No other product was observed by TLC. The 'H nmr spectrum (300 MHz, DMSO-d₆) was conistent with the 5' ribavirin ester of structure 30.

### Example 10

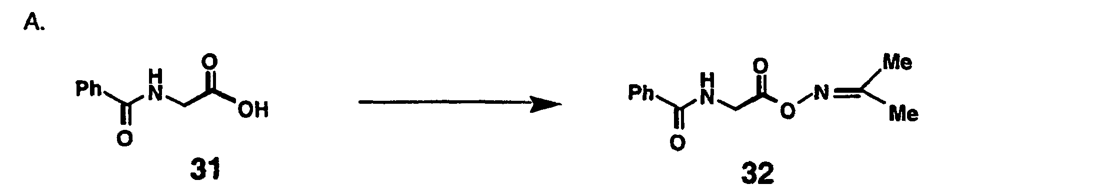

To a stirred solution of 2.45 g (1.4 mmol) of hippuric acid (compound 31) and 1.00 g (1.0 eq) of acetone oxime in 10 mL of CH₂Cl₂ at room temperatuare was added 2.83 g (1.0 eq) of DCC. The so-formed reaction mixture was stirred overnight and then filtered. The filtrate was concentrated under reduced pressure to produce a residue which was purified with EtOAc/hexane to give 2.36 g of the acetone oxine ester (32) as a colorless oil. The FAB-MS: MH⁺= 235.1.

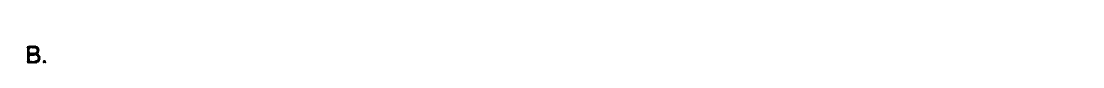

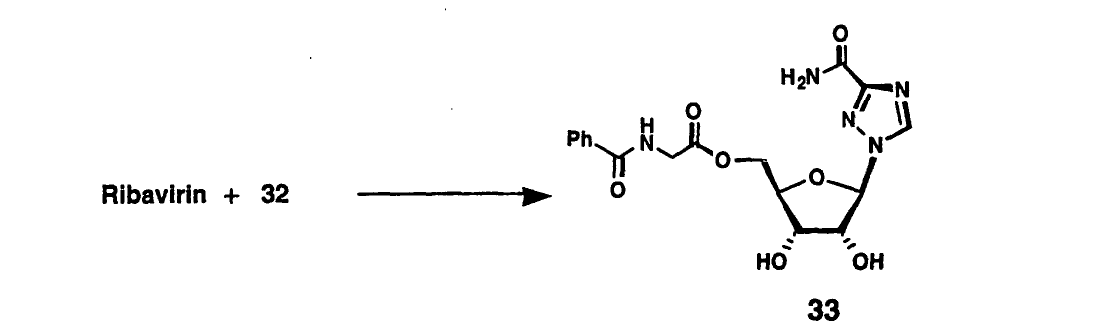

The procedure of Example 9, Step D was used except 2.13 g (9 mmol) of compound 32 was substituted for compound 29, 0.734 g (3 mmol, 1/3 eq) of ribavirin and 0.6 g of SP435 lipase were used in 25 ml of THF. The crude product was purified on silica gel column chromatography using CH₂Cl₂: MeOH (20:1, v/v) as an eluent to provide a crude product. The crude product was crystallized from MeOH: Et₂O to provide 0.936 g of compound 33. FAB:MS, MH⁺=406.1.

### Example 11

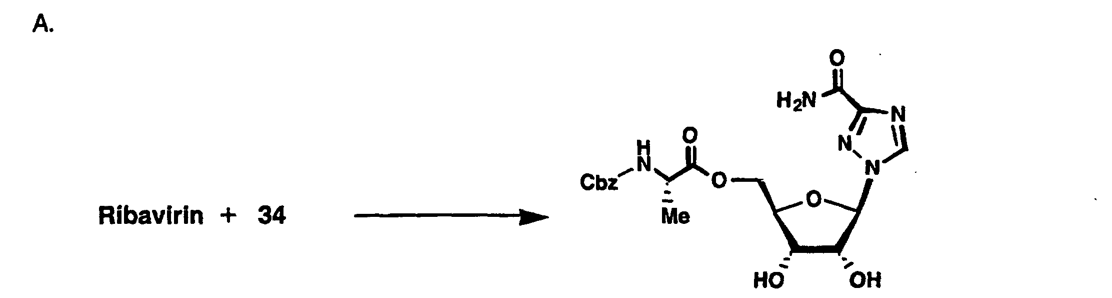

Compound 34, the acetone oxime ester of Cbz-L-alanine, was prepared in accordance with the procedures of F.Morris and V. Gotor, Tetrahedron, 1994, 50, 69-6934 at paragraph bridging 6932-6933.. Then, the procedure of Example 10, Step B, was followed using 1.00 g (3.6 mmol) of compound 34 in place of compound 32, 0.294 g (1.2 mmol) of ribavirin, and 0.48 g of Novo SP435 lipase in 12 ml of THF. The crude produce was purified on silica gel column chromatography using CH₂Cl₂:MeOH (20:1, v/v) as an eleunt to give 0.660 g of compound 35 as a white solid. The white solid was recrystallized from MeOH-EtOAc to give 0.532 g of compound 35.

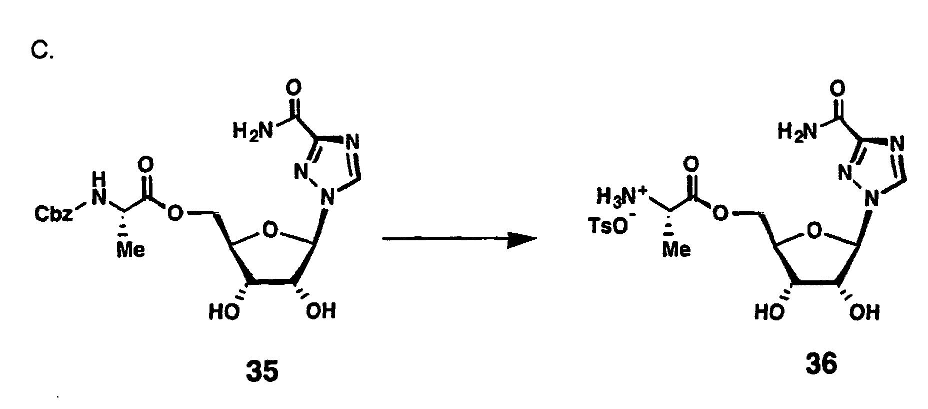

To a mixture of 0.100 g (0.222 mmol) of compound 35 and 42.3 mg (1 eq) of TSOH.H₂O in 3 ml of aqueous MEOH was added 50 mg of 10% Pd on carbon. The resulting black suspension was placed under a hydrogen atmosphere for 4 hours. The so-formed reaction mixture was filtered through a pad of celite and the solid was washed thoroughly with methanol. The combined filtrates were concentrated and the solvent removed to give 0.101 g of compound 36 as a white solid. FAB:MS MH⁺=316.1.

### Example 12

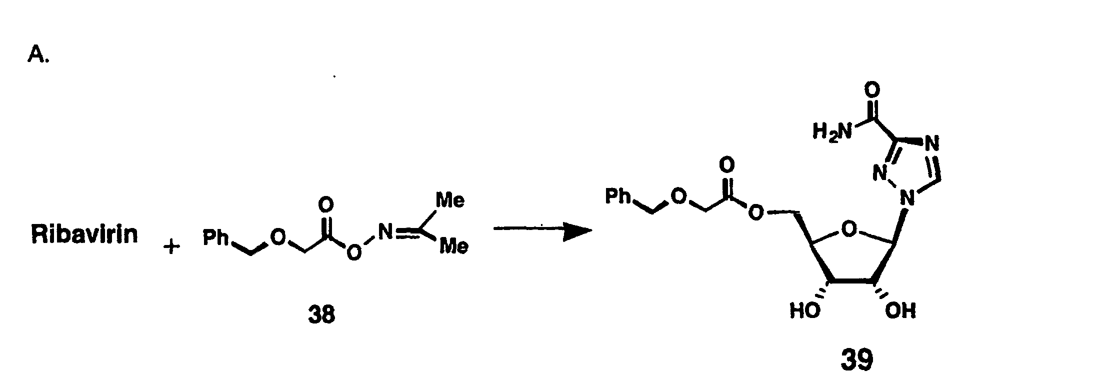

Compound 38 (PhCH₂OCH₂COO-N=C(CH₃)₂) was prepared from 2.17 ml (2.53 eq) benzyloxyacetylchloride (Compound 37) and 1.00 g of acetone oxine in 20 ml of CH₂Cl₂ containing 1.67 g (1 eq) of DMAP in accordance with the procedure of Example 10 Step A. Then, the procedure of Example 10, Step B was followed using 2.40 g (10.8 mmol) of compound 38 in place of compound 32 1.325 g (5.4 mmol) of ribavirin 2.18 g of Novo SP435 lipase in 70 ml of THF. The mixture was treated overnight at 70°C under a nitrogen atmosphere. The crude product was purified on silica gel column chromatography using CH₂Cl₂:MeOH (10:1, v/v) as an eluent to provide 0.511 g of a mixture of two compounds. The desired compound 39 was purified by crystallization from MeOH:Et₂O to give 0.367 g of compound 39 as a white crystalline product.

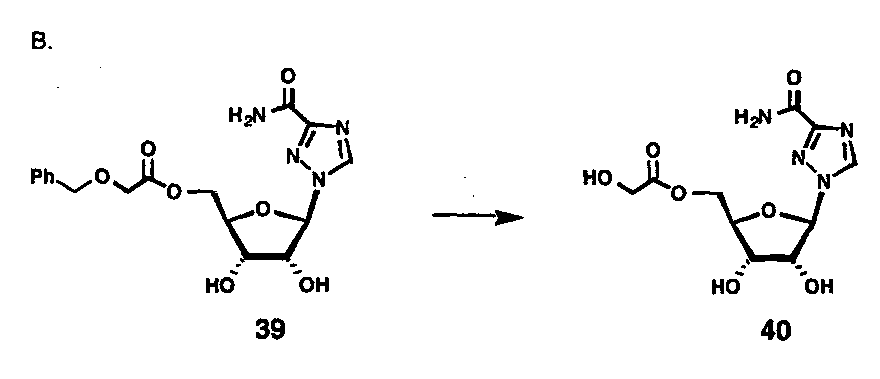

To a stirred suspension of 0.200 g of compound 39 in 5 ml of MeOH containing 50 mg of palladium black under an atmosphere of nitrogen was added 0.20 ml of formic acid. The resulting reaction mixture was heated to reflex for 1 hour. The resulting reaction mixture was cooled and filtered through a pad of cotton wool. The solids were thoroughly washed with methanol and then water. The combined filtrates were concentrated under reduced pressure to give a residue. Methanol was added to provide 99.2 mg of the compound 40. FAB-MS:MH⁺= 303.1.

### Example 13

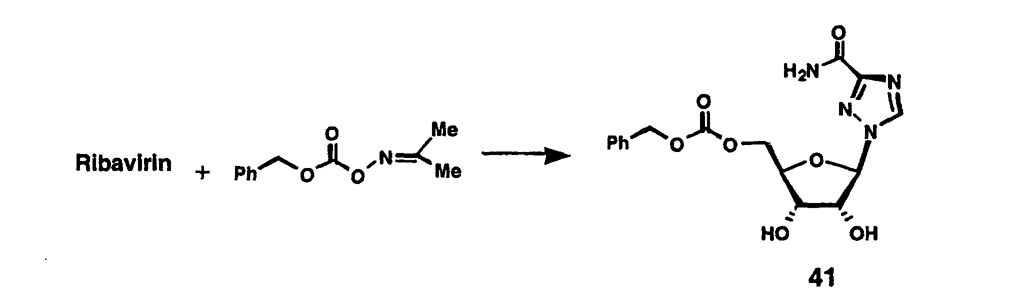

Compound 40 (PhCH₂OCOO-N=C(CH₃) was prepared in accordance with the procedure of Step A of Example 10. Then the procedure of Step B of Example 10 as followed using 500 mg (2.4 mmol) of compound 40 in place of compound 32, 295 mg (1.2 mmol) of ribavirin, 160 mg of Novo SP435 in 15 ml of THF. The so-formed reaction mixture was heated at 70°C for 3 days. The crude product dissolved in MeOH was purified on silica gel column chromatography using CH₂Cl₂:MeOH (20:1, v/v) as an eleunt to provide 156 mg of compound 41. FAB:MS, MH⁺=379.2.

### Example 14

A. The benzyl ether of methyl-L-lactate (compound 42) was prepared from methyl-L-lactate in accordance with the procedures of U. Widner Synthesis 1987, 568.
B. 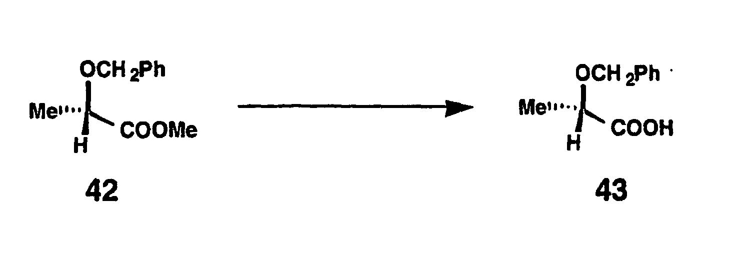
   To a stirred solution of 1.00 g of compound 42 in a mixture of 9 ml of MeOH and 3 ml of water was added 216 mg (1 eq) of LiOH.H₂O. The so-formed reaction mixture was stirred for 4 hours. The resulting mixture was partitioned between CH₂Cl₂, and water. The aqueous phase was separated, washed with CH₂Cl₂ and acidified with excess 10% aqueous HCl. The organics were extracted with EtOAc. The EtOAc layer was dried and concentrated to give 779 mg of compound 43 as a colorless oil.
C. 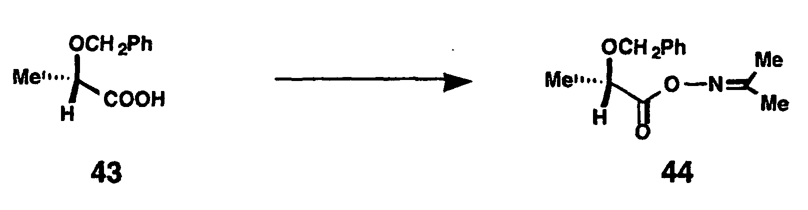
   The procedures of Step A of Example 10 were followed using 1.15 g (6.39 mmol) of compound 43 in place of compound 31, 513 mg, (7.03 mmol, 1.1 eq) of acetone oxime, 1.45 g of DCC in 5 ml of CH₂Cl₂ to produce 1.20 g of the acetone oxime ester, compound 44.
D.

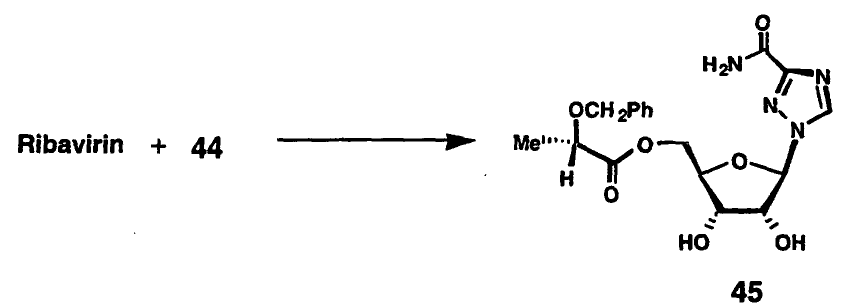

The procedure of Step B of Example 10 was followed using 1.2 g (5.1 mmol) of compound 44 in place of compound 32, 600 mg (2.5 mmol) of ribavirin and 500 mg of Novo SP435 lipase in 20 ml of THF. The mixture washed at 70°C until no starting found by TLC.

The crude reaction product was purified by silica gel column chromatography using CH₂Cl₂:MeOH (20:1, v/v) to produce 183 mg of compound 45 as a white solid.

### Example 15

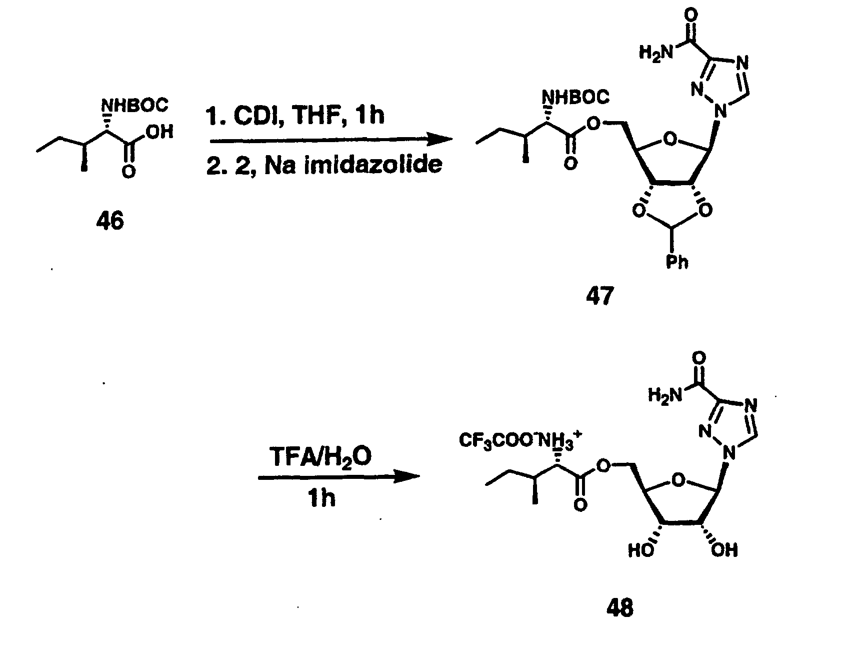

A solution of N-t-Boc-L-isoleucine (available fromSigma Chemical Co. St. Louis, Mo) (1.270 g, 5.5 mmol) in anhydrous THF (30mL) was treated with CDI, i.e., carbonyldiimidazole (981 mg, 6.05 mmol) at room temperature for 1 hr. The compound 2 of Example 1 (1.660 g, 5.00 mmol) and sodium imidazolide (150 mg, 1.5 mmol) were then added to the reaction mixture. This mixture was heated at 45°C for 20 hrs. The reaction was diluted with EtOAc and quenched with aqueous NH₄Cl. The organic layer was washed with water three times and once with brine, and dried over Na₂SO_{4.} The dried organic layer was filtered and the filtrate was concentrated to give an off white solid which was purified on silica gel column chromatography (5-10% by volume of MeOH in CH₂Cl₂) to afford 1.244 g of compound 47.

Compound 47 was treated with trifluoroacetic acid (TFA)/ water (9:1 v/v) at room temperature for 1 hr. All volatiles were evaporated. Water was added and evaporated again to afford 1.10 g of compound 48 as a soft solid. MS(FAB)=358 (MH⁺, 100%).

### Example 16

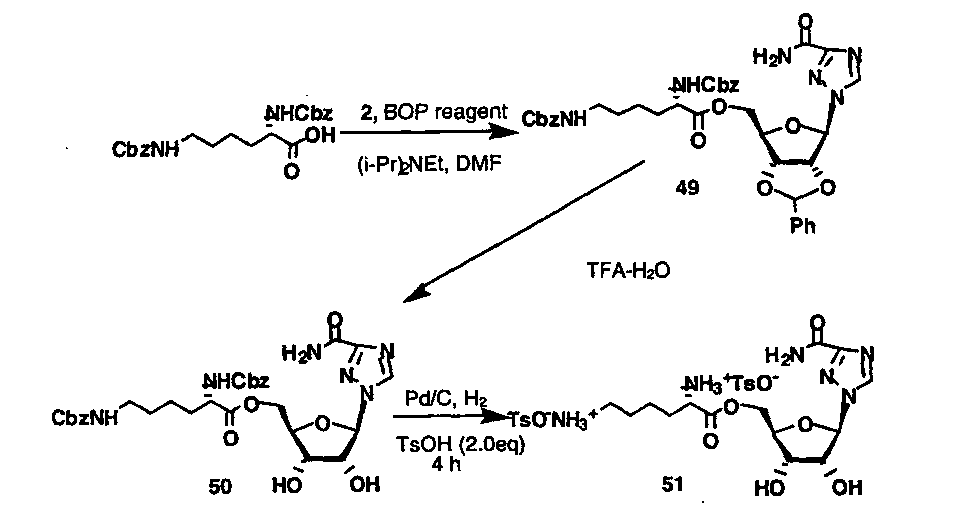

A solution of N,N-diCbz-L-lysine (available fromSigma Chemical Co. St. Louis, Mo) (749 mg, 1.81 mmol) and compound 2 (500 mg, 1.506 mmol) in anhydrous DMF (5 mL) was treated with (732 mg, 1.66 mmol) of benzotriazolyloxytris-(dimethylamino)phosphonium hexafluorophosphate ("BOP reagent" available fromSigma Chemical Co. St. Louis, Mo) followed by 576 mL, 3.31 mmol of Hunig's base, i.e., N,N-diisopropyl-ethyl amine, [ "(i-Pr)₂NEt " available from Aldrich Chemical Co., Milwaukee, WI ] under a nitrogen atmosphere at room temperature. The reaction mixture was stirred at room temperature overnight. The reaction mixture was diluted with EtOAc and quenched with aqueous NH₄Cl. The organic layer was washed three times with water, once with brine, and dried over Na₂SO₄ and. The dried organic layer was filtered and the filtrate was concentrated to give compound 49 as an off white solid.

Compound 49 was treated with trifluoroacetic acid/ water (9:1 v/v) at room temperature for 1 hr. All volatiles were evaporated. Water was added and evaporated again to afford the crude product which was purified on a silica gel column (10 % by volume of MeOH in CH₂Cl₂) to afford 520 mg of compound 50 as a white solid.

A solution containing compound 50(140 mg, 0.219 mmol) and p-toluenesulfonic acid monohydrate, TsOH H₂O (83 mg, 0.438 mmol) was hydrogenated over 10% Pd/C (50 mg) under H₂ at a pressure of 1 atm for 4 hr. The catalyst was filtered off through a bed of Celite and washed with methanol-water. The filtrate was evaporated to afford 157 mg of compound 51 as a white powder. MS(FAB)= 373 (MH⁺, 100%).

### Example 17

Follow the procedures of Examples 11, 15 or 16 except substitute an equivalent amount of the Cbz- or Boc-D- amino acid derivative of the amino acid listed in the left hand column in the Table 3 below for the Boc-L-isoleucine used in Example 15, or the diCbz-L-lysine used in Example 16, or the Cbz-L-alanine used in Example 11 to obtain the compounds of formula I or a pharmaceutical accepable salt thereof wherein the R is the moiety listed in the right hand column of the Table 4 below.

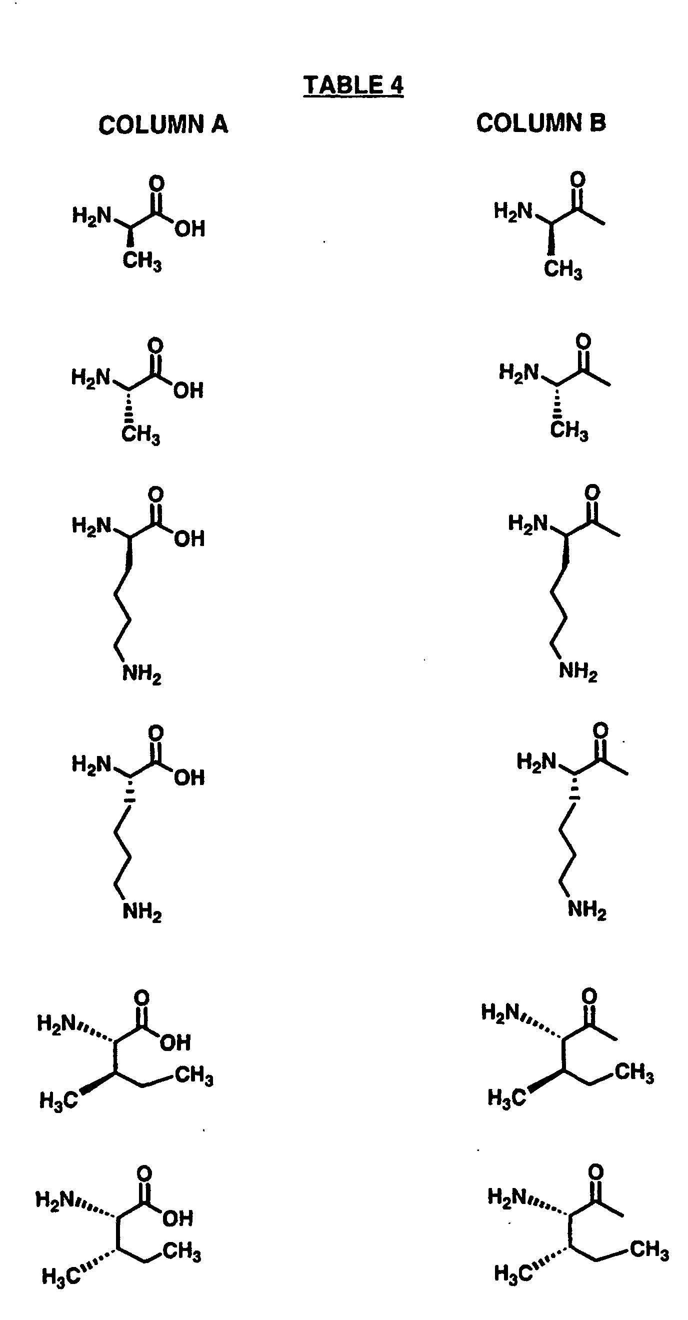

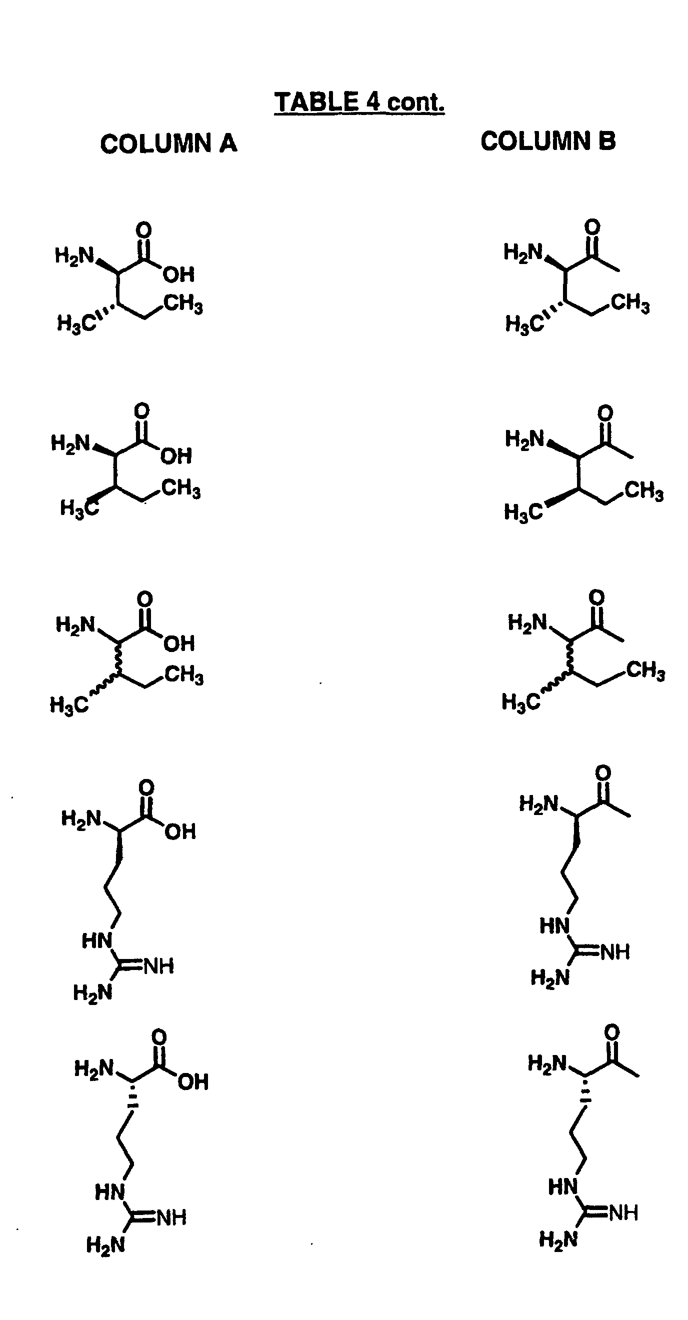

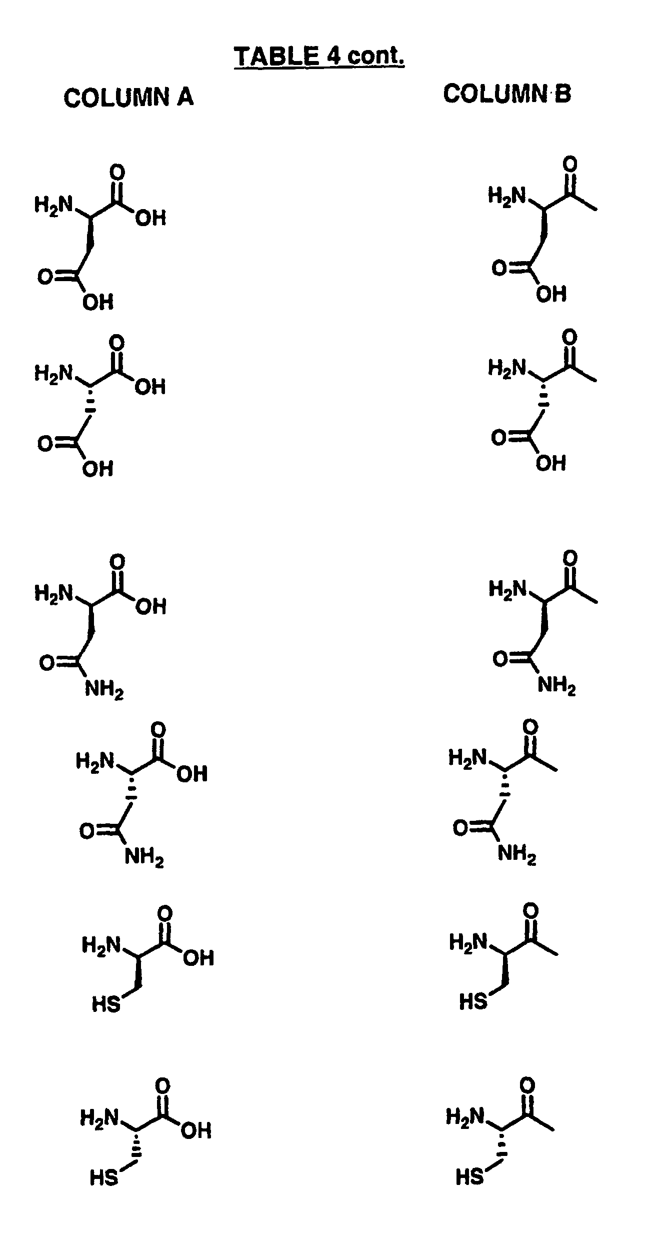

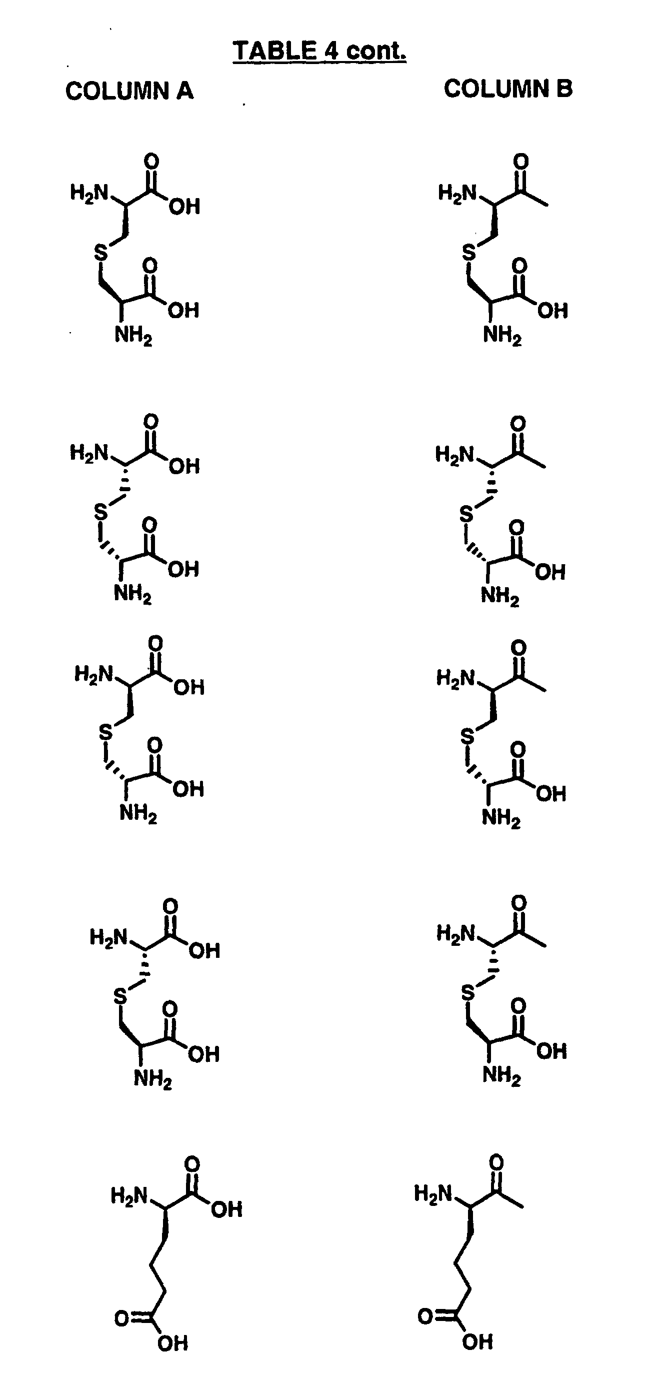

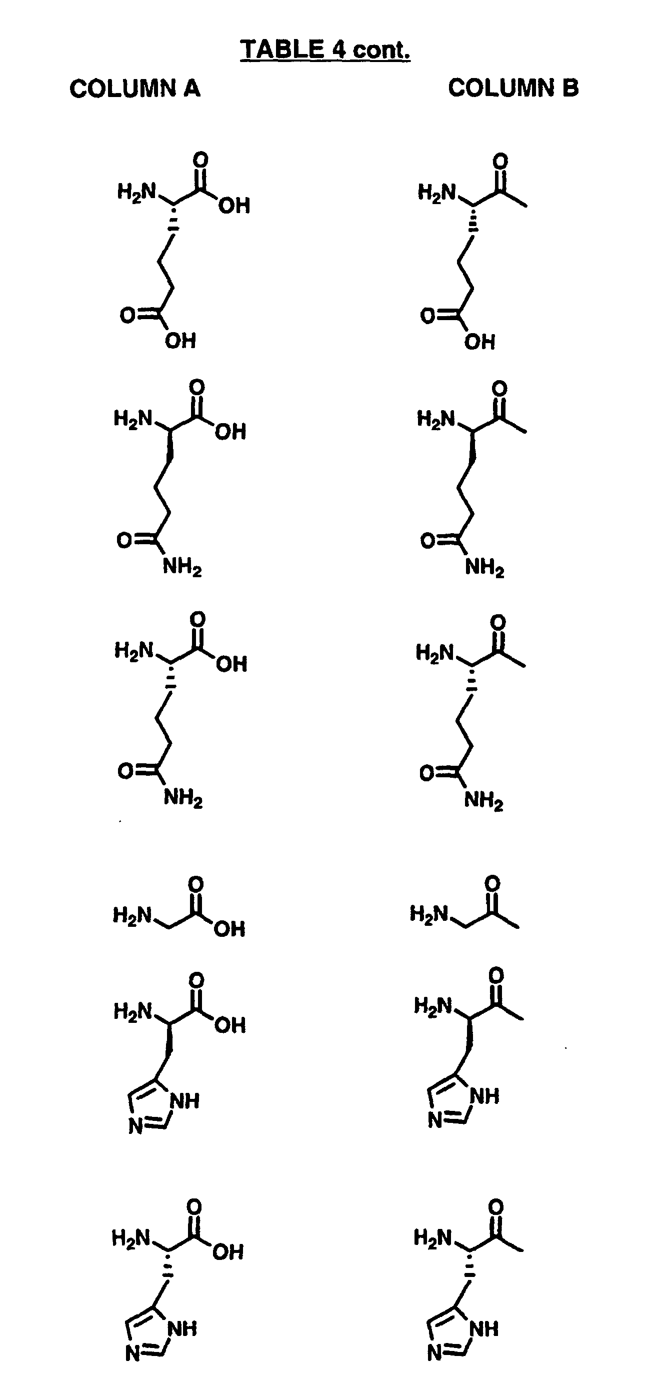

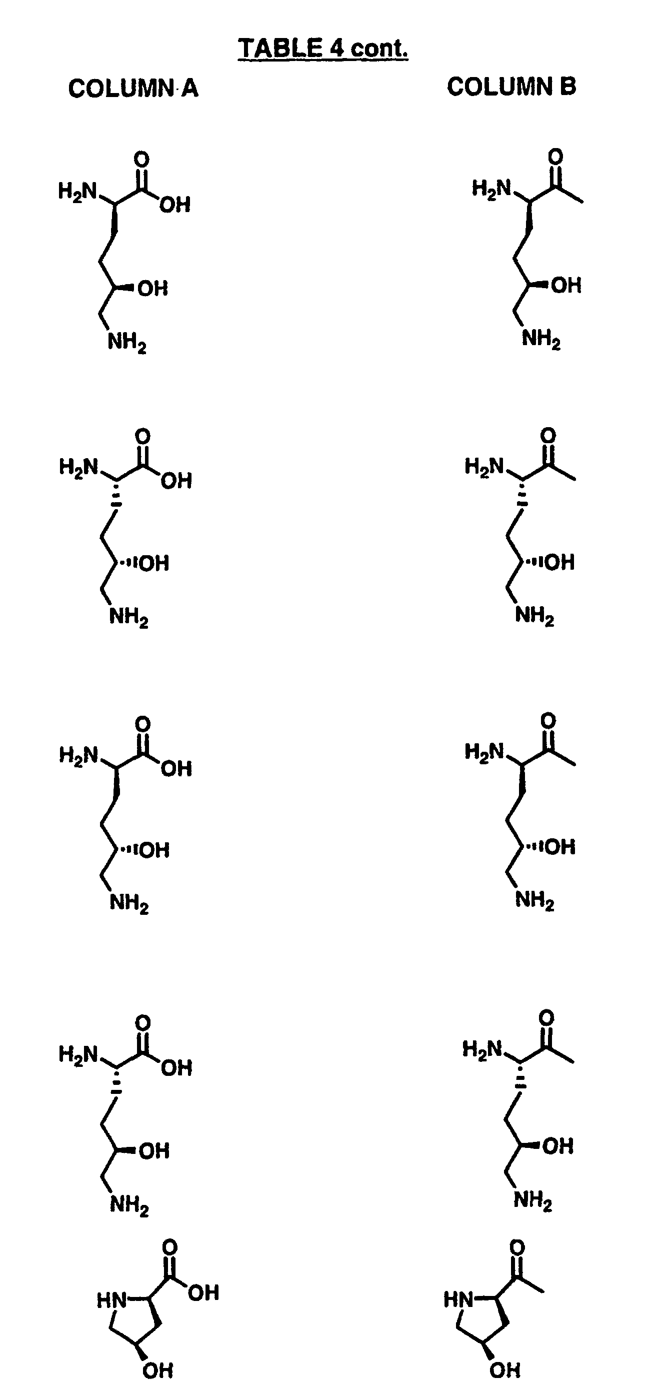

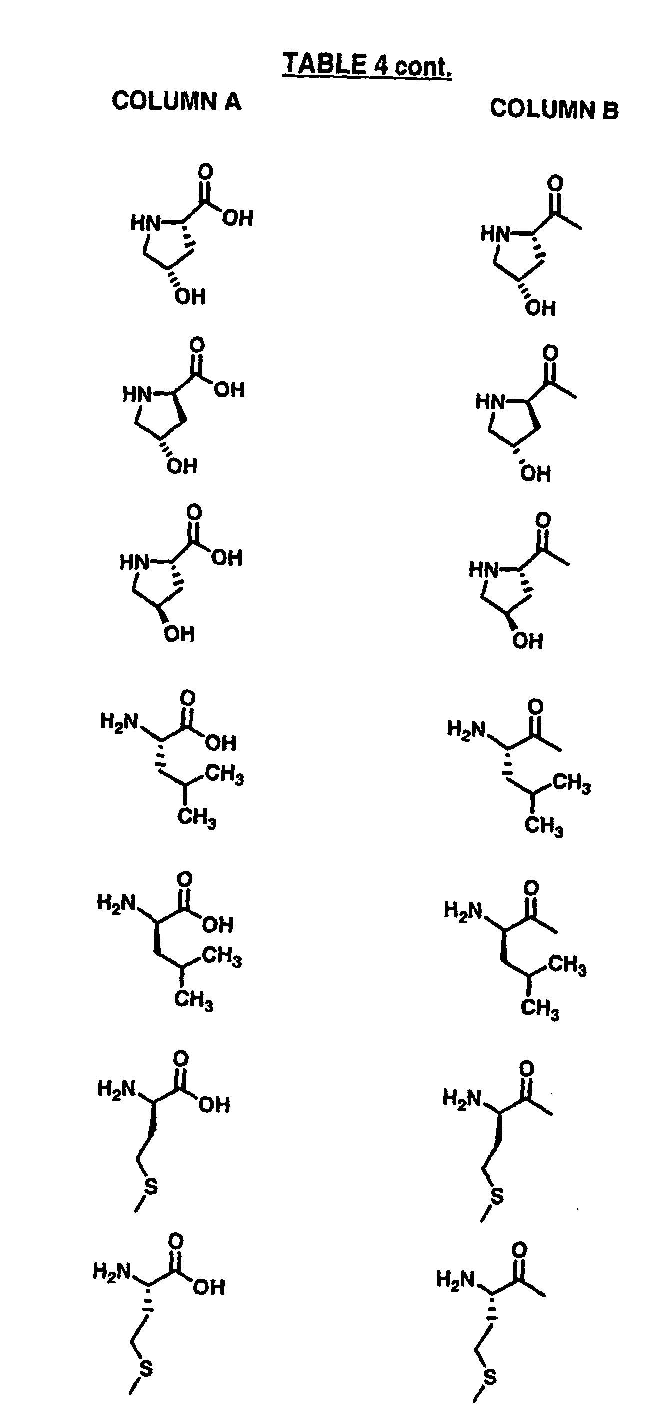

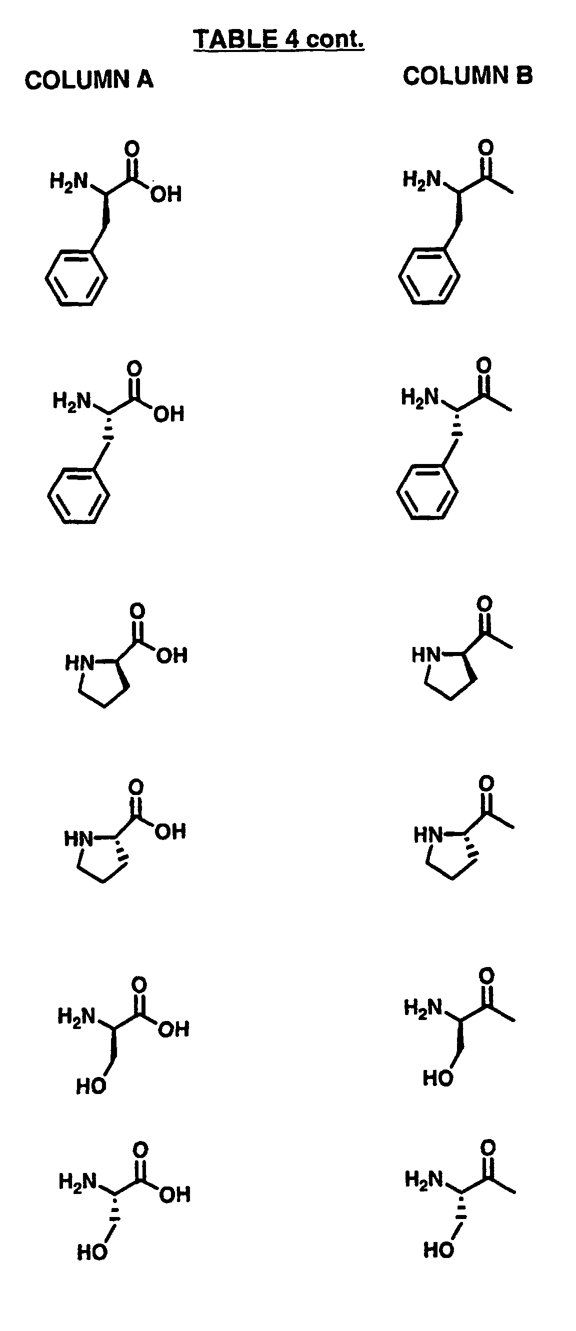

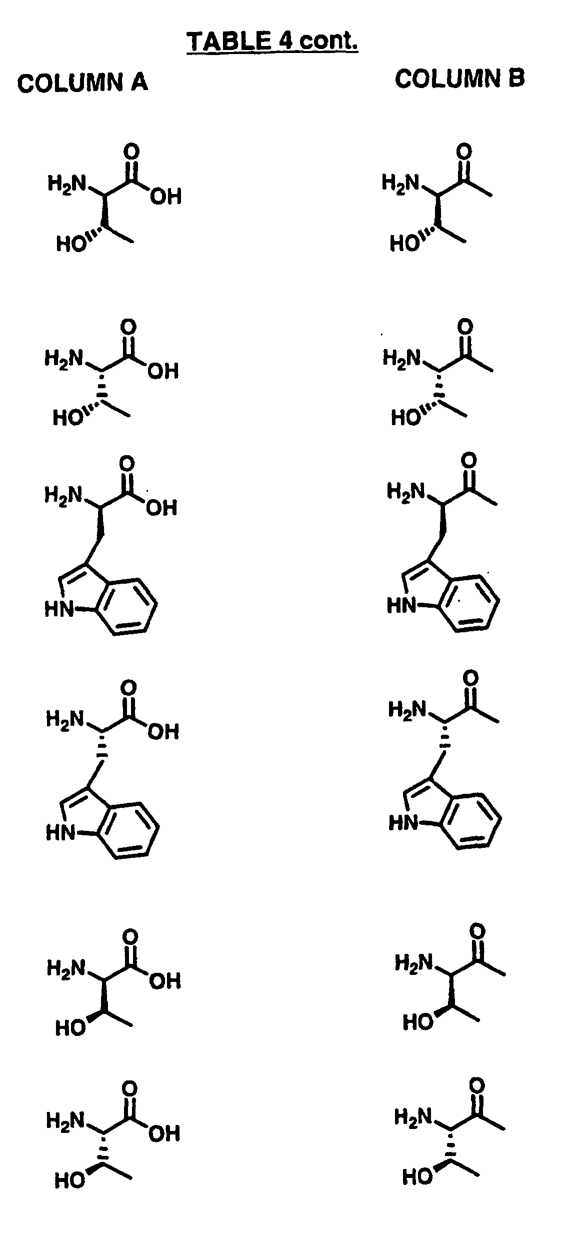

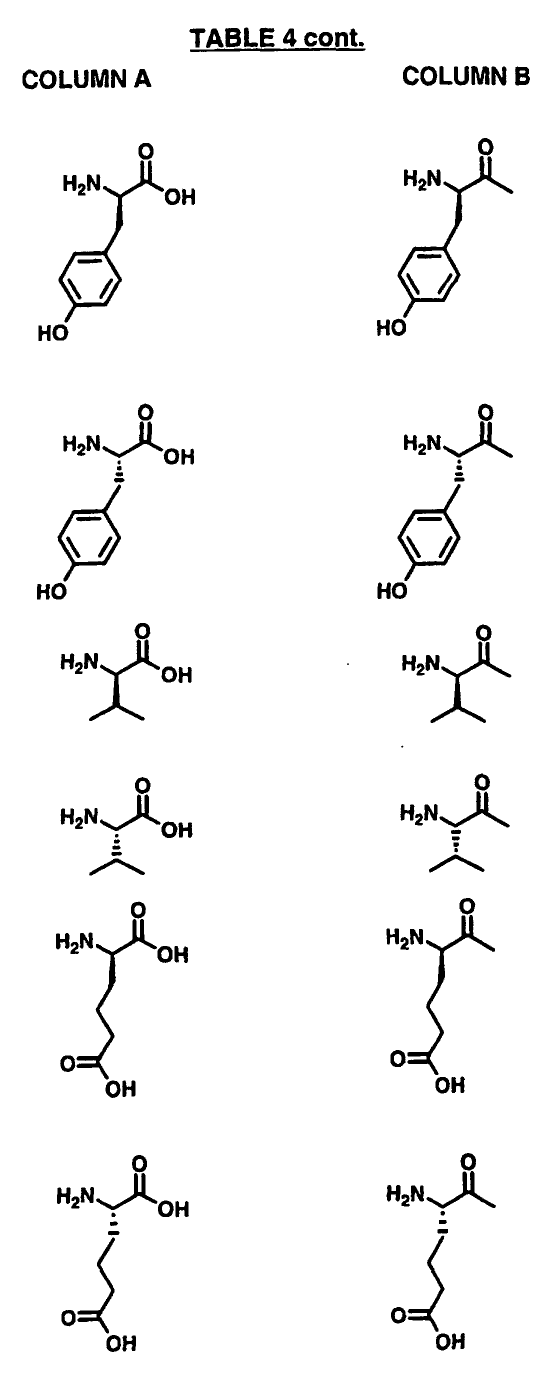

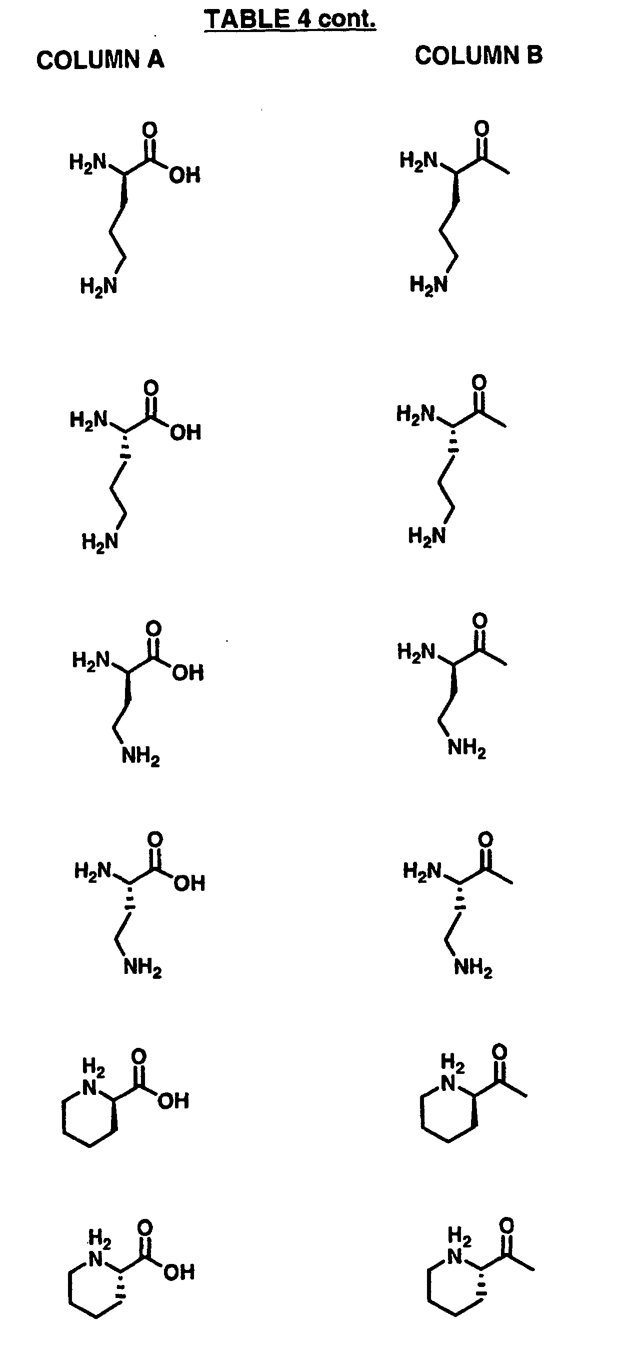

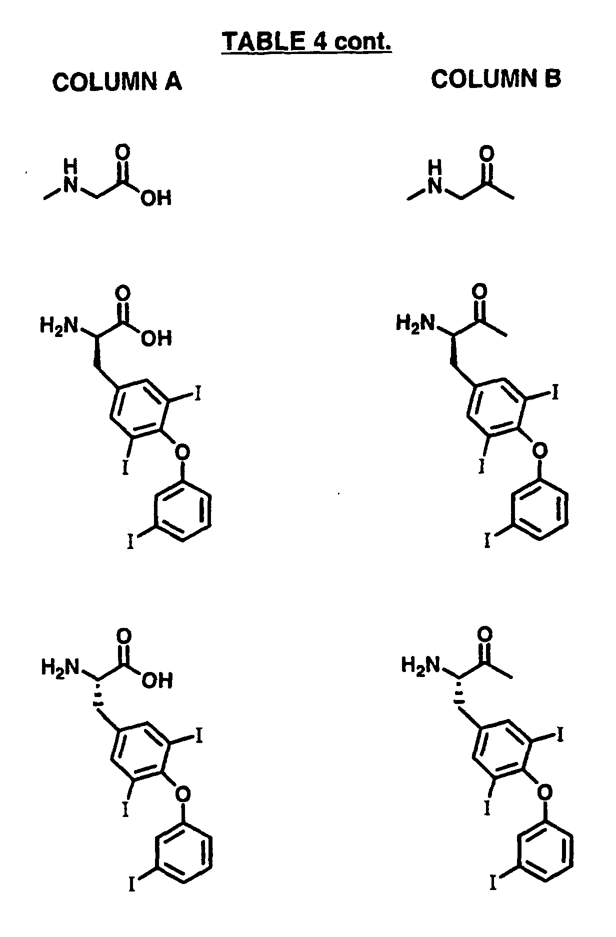

## Claims

1. A compound represented by formula III 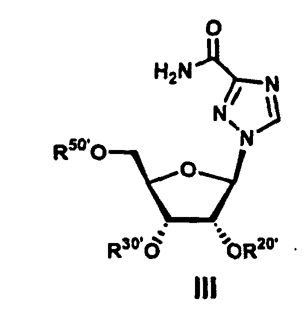 wherein at least one of R^{50'}, R^{30'} or R^{20'} is represented by the formula 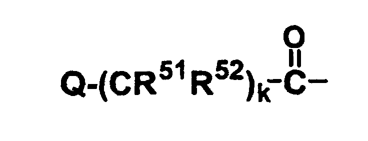 and the other two of R^{50'}, R^{30'} or R^{20'} is H or is represented by the formula 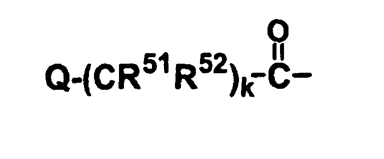 wherein Q is 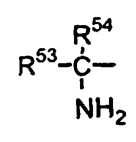 wherein R⁵¹ and R⁵² are independently H, alkyl, alkenyl, alkynyl, (C₃-C₇)cycloalkyl, arylalkyl, or alkyl, alkenyl, alkynyl, (C₃-C₇)cycloalkyl, arylalkyl, substituted by halo, OH, SH , CF₃, SR⁵⁷,OR⁵⁷ or NH₂ or wherein R⁵¹ and R⁵² taken together with the carbon atom in (C R⁵¹ R⁵²) form a cyclopropane , cyclobutane, cyclopentane, or cyclohexane.;
wherein R⁵³ and R⁵⁴ are independently H, alkanoyl,alkyl, aryl, arylalkyl, alkenyl, alkynyl, or alkanoyl, alkyl, aryl, arylalkyl, alkenyl , alkynyl substituted by halo, OH, SH, CF₃, SR⁵⁷, OR⁵⁷; or R⁵³ and R⁵⁴ are independently 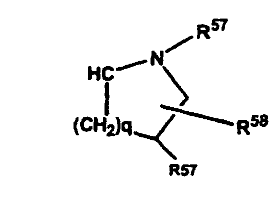 or 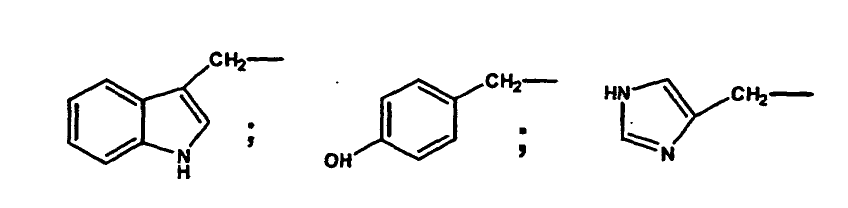 wherein R⁵⁷ is H, alkyl, alkanoyl, alkenoyl, aryl, arylalkyl, alkenyl, alkynyl, or, alkyl, aryl, arylalkyl, alkenyl, alkynyl substituted by halo, OH, SH,CF₃,alkanoylthienyl,or alkanoyloxy;
wherein R⁵⁸ is H, alkyl, aryl, arylalkyl, alkenyl, or alkynyl;
and q is 0,1 or 2; k is 1 or 2;
or a pharmaceutically acceptable salt thereof.

2. A pharmaceutical composition for treating a susceptible viral infection which comprises an effective amount of a compound represented by formula III of claim 1 together with a pharmaceutically acceptable carrier.

3. The use of a compound of formula III for preparation of a medicament for treating a chronic hepatitis C infection which comprises an effective amount of a compound of formula III in association with an effective amount of an interferon alfa sufficient to eradicate detectable HCV-RNA levels, wherein the compound is represented by the formula III of claim 1.

4. A compound represented by the formula IV 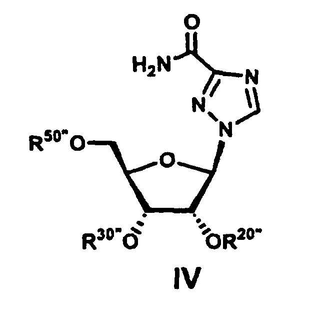 wherein at least one of R^{50"},R^{30"} or R^{20"} is represented by the formula 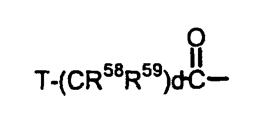 wherein the other two of R^{50"},R^{30"} or R^{20"} are H or represented by the formula 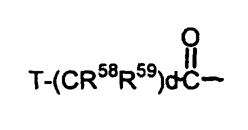 wherein T is a moiety represented by the formulas:
H₂NCH₂-, H₂NCH₂ CH₂- , CH₃CH(H₂N)-, CH₃CH₂CH(H₂N)-,
CH₃(CH₂)₂CH(H₂N)-. (CH₃)₂CHCH(H₂N)-, (CH₃)₂CHCH₂CH(H₂N)-,
CH₃CH₂CH(CH₃ )CH(H₂N)-, PhCH₂CH(H₂N)-, HOOCCH₂CH₂CH(H₂N)-,
HOOCCH₂CH(H₂N)-, HSCH₂CH(H₂N)-, CH₃SCH₂CH₂CH(H₂N)-,
HOCH₂CH(H₂N)-, H₂N(CH2)₄ CH(H₂N)-, CH₃CH(OH)CH(H₂N), 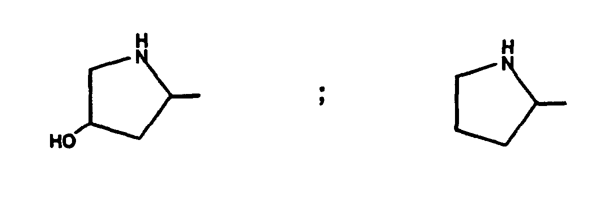 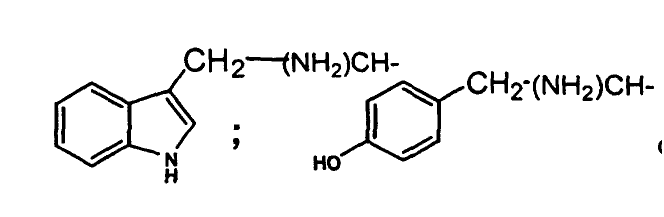 or 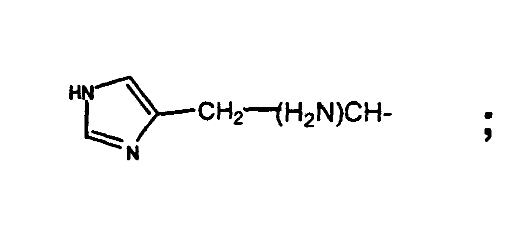 wherein R⁵⁸ and R⁵⁹ are independently H, alkyl, alkenyl, alkynyl,
(C₃-C₇)cycloalkyl, arylalkyl, or alkyl, alkenyl, alkynyl,
(C₃-C₇)cycloalkyl, arylalkyl, substituted by halo, OH, SH, CF₃, SR⁶⁰, OR⁶⁰ or NR⁶⁰R⁶¹ or
wherein R⁵⁸ and R⁵⁹ taken together with the carbon atom in (C R⁵⁸ R⁵⁹) form a cyclopropane, cyclobutane, cyclopentane, or cyclohexane.;
wherein R⁶⁰ is H, alkyl, alkanoyl, alkenoyl, aryl, arylalkyl, alkenyl, alkynyl, or, alkyl, aryl, arylalkyl, alkenyl, alkynyl substituted by halo, OH, SH,CF₃,alkanoylthienyl,or alkanoyloxy;
wherein R⁶¹ is H, alkyl, aryl, arylalkyl, alkenyl,or alkynyl;
and d is 0,1 or 2;
or a pharmaceutically acceptable salt thereof.

5. A pharmaceutical composition for treating a susceptible viral infection which comprises an effective amount of a compound represented by formula IV of claim 4, together with a pharmaceutically acceptable carrier.

6. The use of a compound of formula IV for preparation of a medicament for treating chronic hepatitis C infection which comprises an effective amount of a compound of formula IV in association with an effective amount of an interferon alfa sufficient to eradicate detectable HCV-RNA levels, wherein the compound represented by the formula IV of claim 4.

7. A compound represented by the formula V 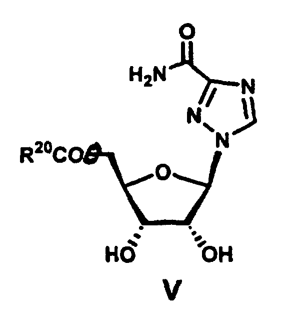 wherein R²⁰ CO- is a natural or unnatural amino acid moiety represented by the formulas 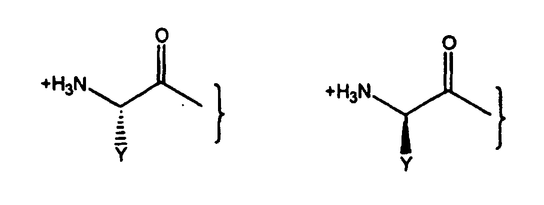 Y = H, CH₃; CH₃CH₂-; CH₃CH₂CH₂-; Me₂CH-; Me₂CH₂CH₂-; CH₃CH₂CH(Me)-PhCH₂-; HOOCCH₂CH₂-; HSCH₂-; HOOCCH₂-; MeSCH₂CH₂-; HOCH₂-; 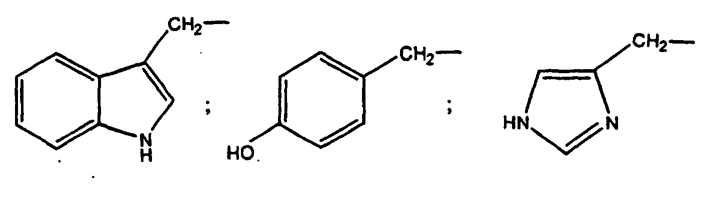 or Y is H₂N(CH₂)₄- or CH₃CH(OH)-
or Y taken together with the α carbon and N form 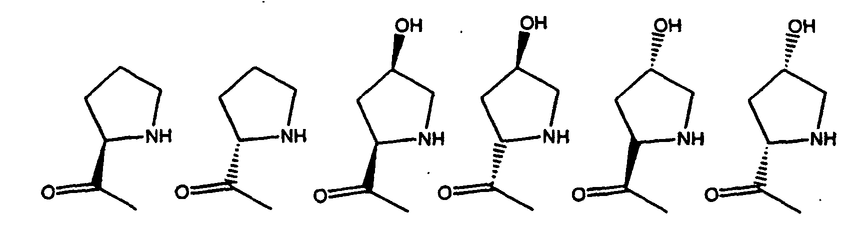 or a pharmaceutically acceptable salt thereof.

8. A pharmaceutical composition for treating susceptible viral infection which comprises an effective amount of a compound represented by formula V of claim 7, together with a pharmaceutically acceptable carrier.

9. The use of a compound of formula V for preparation of a medicament for treating chronic hepatitis C infection which comprises an effective amount of a compound of formula V in association with an effective amount of an interferon alfa sufficient to eradicate detectable HCV-RNA levels, wherein the compound represented by the formula V of claim 7.

10. A compound represented by the formula VI 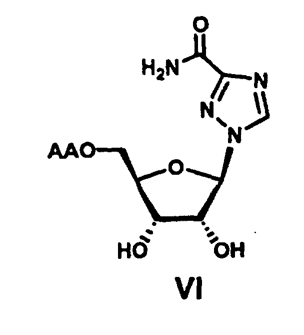 wherein AA is a natural or unnatural amino acid moiety of the formulas in Table AA or a pharmaceutically acceptable salt thereof.

11. A pharmaceutical composition of treating a susceptible viral infection which comprises an effective amount of a compound represented by formula VI of claim 10, together with a pharmaceutically acceptable carrier.

12. The use of a compound of formula VI for preparation of a medicament for treating chronic hepatitis C infection which comprises an effective amount of a compound of formula VI in association with an effective amount of an interferon alfa sufficient to eradicate detectable HCV-RNA levels, wherein the compound represented by the formula VI of claim 10.

13. A compound represented by the formula VII 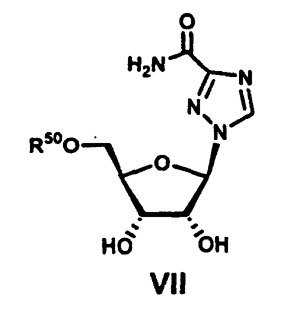 wherein R⁵⁰ is CH₃CH(NH₂)-CO- , CH₃CH₂(CH₃)CHCH(NH₂)-CO- or H₂N(CH₂)₄CH(NH₂)-CO- ;
or a pharmaceutically acceptable salt thereof.

14. The compound of claim 13 wherein pharmaceutically acceptable salt is trifluoroacetate, tosylate, mesylate, or chloride.

15. The compound of claim 13 wherein R⁵⁰ is 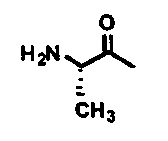 or R⁵⁰ is 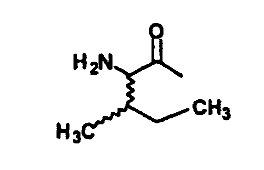 or R⁵⁰ is 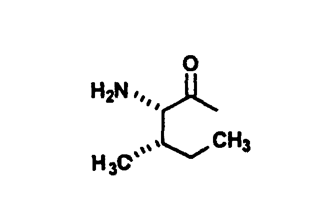 or R⁵⁰ is 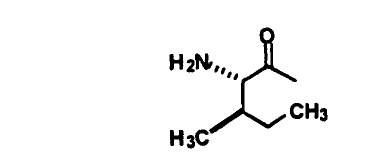 or R⁵⁰ is 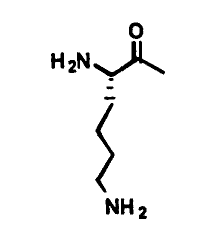 or R⁵⁰ is 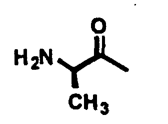 or R⁵⁰ is 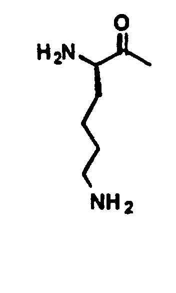 or R⁵⁰ is 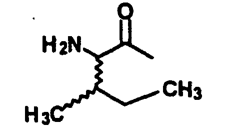 or R⁵⁰ is 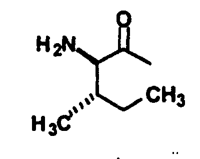 or R⁵⁰ is 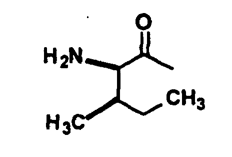 or a pharmaceutically acceptable salt thereof

16. A pharmaceutical composition for treating a susceptible viral infection comprising a compound of claim 13 and at least one pharmaceutically acceptable carrier.

17. The pharmaceutically composition of claim 13 adapted for oral administration.

18. The use of a compound of formula VII for preparation of a medicament for treating a susceptible chronic hepatitis C infection which comprises an effective amount of a compound represented by formula VII of claim 13.

19. The use of a compound of formula VII for preparation of a medicament for treating chronic hepatitis C infection which comprises an effective amount of a compound of formula VII in association with an effective amount of an interferon alfa sufficient to eradicate detectable HCV-RNA levels, wherein the compound represented by the formula VII of claim 13.

20. The use of claim 19 wherein the interferon alfa is interferon alpha-2a, interferon alpha-2b, consensus interferon, pegylated interferon alpha-2a or pegylated interferon alfa -2b.

21. A compound represented by the formula VIII: 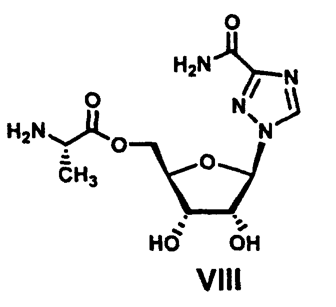 or a pharmaceutically acceptable salt thereof.

22. A pharmaceutical composition comprising an anti-viral effective amount of the compound of claim 21 and a pharmaceutically acceptable carrier.

23. The use of a compound of formula VIII for preparation of a medicament for treating a susceptible chronic hepatitis C infection which comprises an effective amount of a compound represented by formula VIII of claim 21.

24. The use of a compound of formula VIII for preparation of a medicament for treating chronic hepatitis C infection which comprises an effective amount of a compound of formula VIII of claim 21 or a pharmaceutically acceptable salt thereof, in association with an effective amount of an interferon alfa sufficient to eradicate detectable HCV-RNA levels.

25. The use of claim 24 wherein the interferon alfa is interferon alpha-2a, interferon alpha-2b, consensus interferon, pegylated interferon alpha-2a or pegylated interferon alfa -2b.

26. The pharmaceutical composition of claim 22 wherein the compound represented by the formula VIII is adapted for oral administration.

27. The compound of claim 21 wherein the pharmaceutically acceptable salt is trifluoroacetate, tosylate, mesylate, sulfate 2-nappthalenesulfonate or chloride.

## Patentansprüche

1. Verbindung mit der Formel III 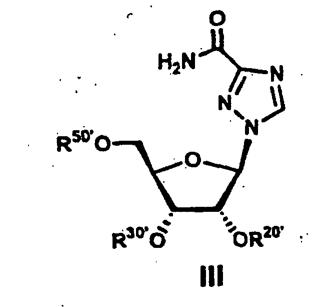 worin mindestens einer von R^{50'}, R^{30'} oder R^{20'} durch die Formel 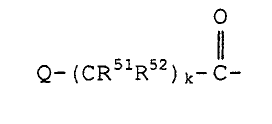 wiedergegeben wird und die beiden anderen von R^{50'}, R^{30'} oder R^{20'} H sind oder durch die Formel 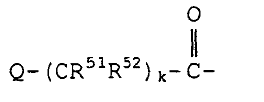 wiedergegeben werden, wobei Q 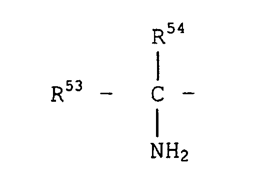 ist, wobei R⁵¹ und R⁵² unabhängig H, Alkyl, Alkenyl, Alkinyl, (C₃- bis C₇)-Cycloalkyl, Arylalkyl oder durch Halogen, OH, SH, CF₃, SR⁵⁷, OR⁵⁷ oder NH₂ substituiertes Alkyl, Alkenyl, Alkinyl, (C₃- bis C₇)-Cycloalkyl, Arylalkyl sind, oder wobei R⁵¹ und R⁵² zusammen mit dem Kohlenstoffatom in (CR⁵¹R⁵²) ein Cyclopropan, Cyclobutan, Cyclopentan oder Cyclohexan bilden;
worin R⁵³ und R⁵⁴ unabhängig H, Alkanoyl, Alkyl; Aryl; Arylalkyl, Alkenyl, Alkinyl oder Alkanoyl; durch Halogen, OH, SH, CF₃, SR⁵⁷, OR⁵⁷ substituiertes Alkyl, Aryl, Arylalkyl, Alkenyl, Alkinyl sind; oder R⁵³ und R⁵⁴ unabhängig 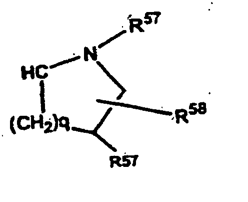 oder 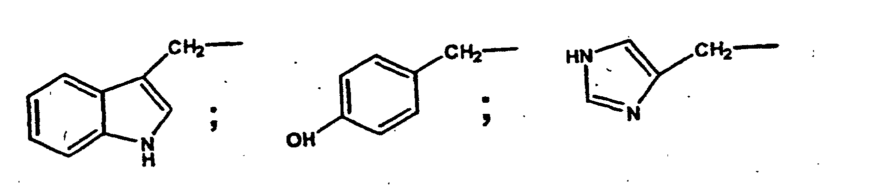 sind, wobei R⁵⁷ H, Alkyl, Alkanoyl, Alkenoyl, Aryl, Arylalkyl, Alkenyl, Alkinyl oder durch Halogen, OH, SH, CF₃, Alkanoylthienyl oder Alkanoyloxy substituiertes Alkyl, Aryl, Arylalkyl, Alkenyl, Alkinyl ist;
worin R⁵⁸ H, Alkyl, Aryl, Arylalkyl, Alkenyl oder Alkinyl ist; und
q 0, 1 oder 2 ist; k 1 oder 2 ist;
oder ein pharmazeutisch annehmbares Salz davon.

2. Pharmazeutische Zusammensetzung zur Behandlung einer beeinflußbaren viralen Infektion, die eine wirksame Menge einer Verbindung, die durch Formel III von Anspruch 1 wiedergegeben wird, zusammen mit einem pharmazeutisch annehmbaren Träger enthält.

3. Verwendung einer Verbindung der Formel III zur Herstellung eines Medikaments zur Behandlung einer chronischen Hepatitis C-Infektion, das eine wirksame Menge einer Verbindung der Formel III zusammen mit einer wirksamen Menge eines Interferon-α enthält, die ausreicht, um nachweisbare HCV-RNA-Gehalte auszulöschen, wobei die Verbindung durch die Formel III von Anspruch 1 wiedergegeben wird.

4. Verbindung mit der Formel IV 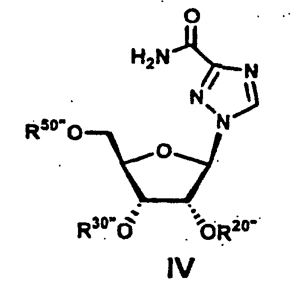 worin mindestens einer von R^{50"}, R^{30"} oder R^{20"} durch die Formel 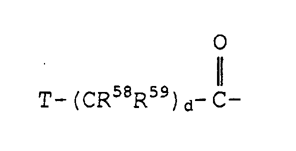 wiedergegeben wird, wobei die beiden anderen von R^{50"}, R^{30"} oder R^{20"} H sind oder durch die Formel 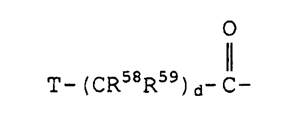 wiedergegeben werden, wobei T eine Einheit ist, die durch die Formeln H₂NCH₂-, H₂NCH₂CH₂-, CH₃CH(H₂N)-, CH₃CH₂CH (H₂N)-, CH₃ (CH₂) ₂CH (H₂N)-, (CH₃)₂CHCH(H₂N)-, (CH₃) ₂CHCH₂CH (H₂N)-, CH₃CH₂CH (CH₃) CH (H₂N) -, PhCH₂CH (H₂N)-, HOOCCH₂CH₂CH (H₂N)-, HOOCCH₂CH (H₂N)-, HSCH₂CH (H₂N)-, CH₃SCH₂CH₂CH (H₂N)-, HOCH₂CH (H₂N) -, H₂N (CH₂) ₄CH (H₂N)-; CH₃CH (OH) CH (H₂N), 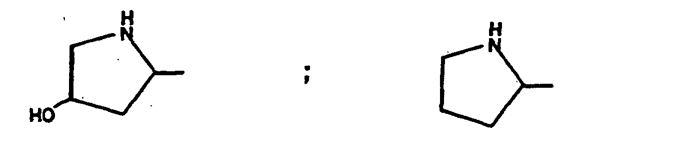 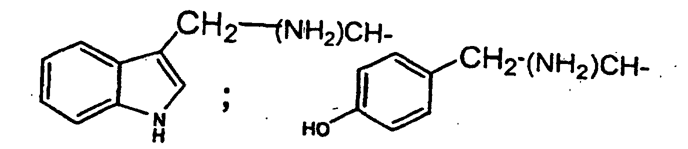 oder 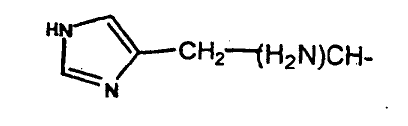 wiedergegeben wird;
worin R⁵⁸ und R⁵⁹ unabhängig H, Alkyl, Alkenyl, Alkinyl, (C₃- bis C₇)-Cycloalkyl, Arylalkyl oder durch Halogen, OH, SH, CF₃, SR⁶⁰, OR⁶⁰ oder NR⁶⁰R⁶¹ substituiertes Alkyl, Alkenyl, Alkinyl, (C₃- bis C₇)-Cycloalkyl, Arylalkyl sind, oder wobei R⁵⁸ und R⁵⁹ zusammen mit dem Kohlenstoffatom in (CR⁵⁸R⁵⁹) ein Cyclopropan, Cyclobutan, Cyclopentan oder Cyclohexan bilden;
worin R⁶⁰ H, Alkyl, Alkanoyl, Alkenoyl, Aryl, Arylalkyl, Alkenyl, Alkinyl, oder durch Halogen, OH, SH, CF₃, Alkanoylthienyl oder Alkanoyloxy substituiertes Alkyl, Aryl, Arylalkyl, Alkenyl, Alkinyl ist;
worin R⁶¹ H, Alkyl, Aryl, Arylalkyl, Alkenyl oder Alkinyl ist; und
d 0, 1 oder 2 ist,
oder ein pharmazeutisch annehmbares Salz davon.

5. Pharmazeutische Zusammensetzung zur Behandlung einer beeinflußbaren viralen Infektion, die eine wirksame Menge einer Verbindung, die durch Formel IV von Anspruch 4 wiedergegeben wird, zusammen mit einem pharmazeutisch annehmbaren Träger enthält.

6. Verwendung einer Verbindung der Formel IV zur Herstellung eines Medikaments zur Behandlung chronischer Hepatitis C-Infektion, das eine wirksame Menge einer Verbindung der Formel IV zusammen mit einer wirksamen Menge eines Interferon-α enthält, die ausreicht, um nachweisbare HCV-RNA-Gehalte auszulöschen, wobei die Verbindung durch die Formel IV von Anspruch 4 wiedergegeben wird.

7. Verbindung mit der Formel V 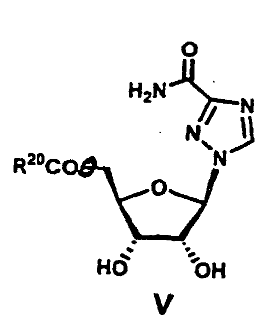 worin R²⁰CO- eine natürliche oder nicht-natürliche Aminosäureneinheit ist, die durch die Formeln 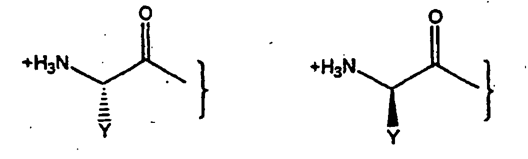 Y = H, CH₃, CH₃CH₂-, CH₃CH₂CH₂-, Me₂CH-, Me₂CH₂CH₂-, CH₃CH₂CH (Me) -, PhCH₂-, HOOCCH₂CH₂-, HSCH₂-, HOOCCH₂-, MeSCH₂CH₂-; HOCH₂-, 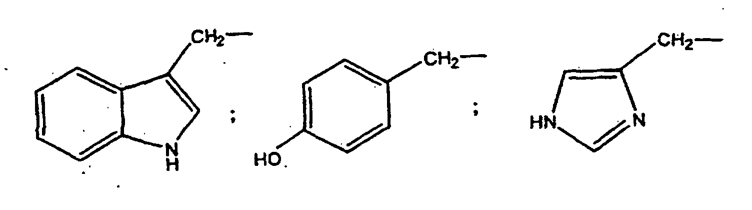 wiedergegeben wird, oder Y H₂N (CH₂) ₄- oder CH₃CH (OH)- ist, oder Y zusammen mit dem α-Kohlenstoff und N 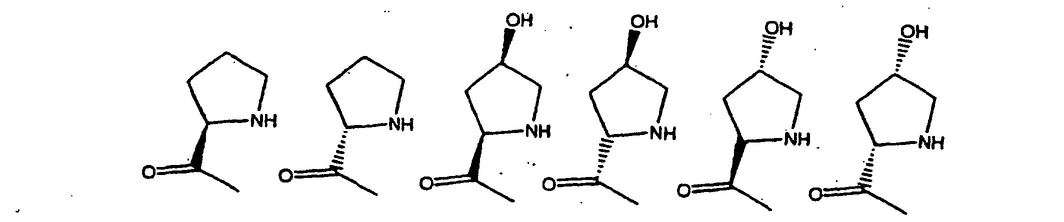 bilden, oder ein pharmazeutisch annehmbares Salz davon.

8. Pharmazeutische Zusammensetzung zur Behandlung einer beeinflußbaren viralen Infektion, die eine wirksame Menge einer Verbindung, die durch Formel V von Anspruch 7 wiedergegeben wird, zusammen mit einem pharmazeutisch annehmbaren Träger enthält.

9. Verwendung einer Verbindung der Formel V zur Herstellung eines Medikaments zur Behandlung chronischer Hepatitis C-Infektion, das eine wirksame Menge einer Verbindung der Formel V zusammen mit einer wirksamen Menge eines Interferon-α enthält, die ausreicht, um nachweisbare HCV-RNA-Gehalte auszulöschen, wobei die Verbindung durch die Formel V von Anspruch 7 wiedergegeben wird.

10. Verbindung mit der Formel VI 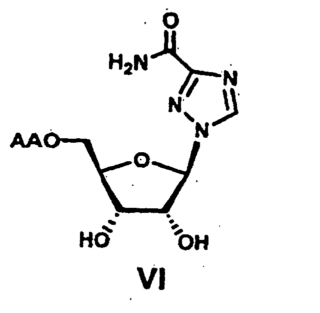 worin AA eine natürliche oder nicht-natürliche Aminosäureeinheit der Formeln in Tabelle AA ist, oder ein pharmazeutisch annehmbares Salz davon.

11. Pharmazeutische Zusammensetzung zur Behandlung einer beeinflußbaren Infektion, die eine wirksame Menge einer Verbindung, die durch Formel VI von Anspruch 10 wiedergegeben wird, zusammen mit einem pharmazeutisch annehmbaren Träger enthält.

12. Verwendung einer Verbindung der Formel VI zur Herstellung eines Medikaments zur Behandlung chronischer Hepatitis C-Infektion, das eine wirksame Menge einer Verbindung der Formel VI zusammen mit einer wirksamen Menge eines Interferon-α enthält, die ausreicht, um nachweisbare HCV-RNA-Gehalte auszulöschen, wobei die Verbindung durch die Formel VI von Anspruch 10 wiedergegeben wird.

13. Verbindung mit der Formel VII 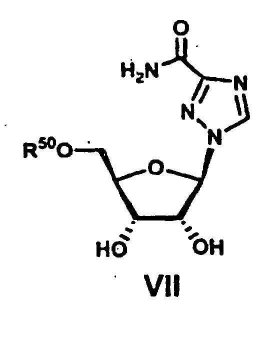 wobei R⁵⁰ CH₃CH (NH₂) -CO-, CH₃CH₂ (CH₃) CHCH (NH₂₎-CO- oder H₂N (CH₂)₄CH (NH₂) -CO- ist, oder ein pharmazeutisch annehmbares Salz davon.

14. Verbindung nach Anspruch 13, bei der das pharmazeutisch annehmbare Salz Trifluoracetat, Tosylat, Mesylat oder Chlorid ist.

15. Verbindung nach Anspruch 13, bei der R⁵⁰ ist, oder R⁵⁰ 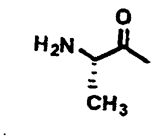 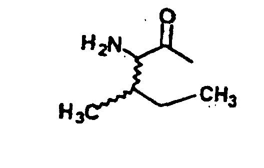 ist, oder R⁵⁰ 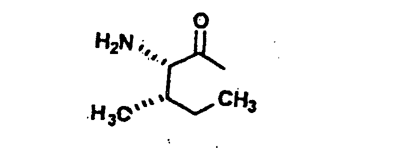 ist, oder R⁵⁰ 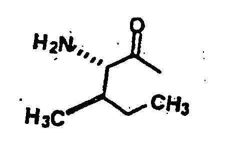 ist, oder R⁵⁰ 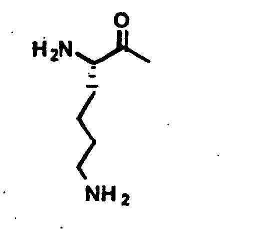 ist, oder R⁵⁰ 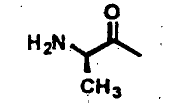 ist, oder R⁵⁰ 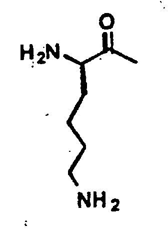 ist, oder R⁵⁰ 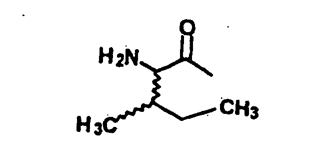 ist, oder R⁵⁰ 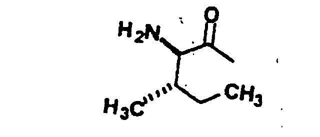 ist, oder R⁵⁰ 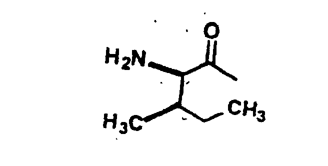 ist, oder ein pharmazeutisch annehmbares Salz davon.

16. Pharmazeutische Zusammensetzung zur Behandlung einer beeinflußbaren viralen Infektion, die eine Verbindung der Formel 13 und mindestens einen pharmazeutisch annehmbaren Träger enthält.

17. Pharmazeutische Zusammensetzung nach Anspruch 13, die für die orale Verabreichung angepasst ist.

18. Verwendung einer Verbindung der Formel VII zur Herstellung eines Medikaments zur Behandlung einer beeinflußbaren chronischen Hepatitis C-Infektion, das eine wirksame Menge einer Verbindung enthält, die durch Formel VII von Anspruch 13 wiedergegeben wird.

19. Verwendung einer Verbindung der Formel VII zur Herstellung eines Medikaments zur Behandlung chronischer Hepatitis C-Infektion, das eine wirksame Menge einer Verbindung der Formel VII zusammen mit einer wirksamen Menge eines Interferon-α enthält, die ausreicht, um nachweisbare HCV-RNA-Gehaltes auszulöschen, wobei die Verbindung durch die Formel VII von Anspruch 13 wiedergegeben wird.

20. Verwendung nach Anspruch 19, bei der das Interferon-α Interferon-α-2a, Interferon-α-2b, Consensus-Interferon, pegyliertes Interferon-α-2a oder pegyliertes Interferon-α-2b ist.

21. Verbindung mit der Formel VIII 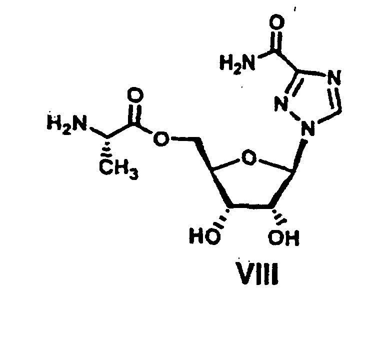 oder ein pharmazeutisch annehmbares Salz davon.

22. Pharmazeutische Zusammensetzung, die eine antiviral wirksame Menge der Verbindung nach Anspruch 21 und einen pharmazeutisch annehmbaren Träger enthält.

23. Verwendung einer Verbindung der Formel VIII zur Herstellung eines Medikaments zur.Behandlung einer beeinflußbaren chronischen Hepatitis C-Infektion, das eine wirksame Menge einer Verbindung enthält, die durch Formel VIII von Anspruch 21 wiedergegeben wird.

24. Verwendung einer Verbindung der Formel VIII zur Herstellung eines Medikaments zur Behandlung chronischer Hepatitis C-Infektion, das eine wirksame Menge einer Verbindung der Formel VIII nach Anspruch 21 oder ein pharmazeutisch annehmbares Salz davon zusammen mit einer wirksamen Menge eines Interferon-α enthält, die ausreicht, um nachweisbare HCV-RNA-Gehalte auszulöschen.

25. Verwendung nach Anspruch 24, bei der das Interferon-α Interferon-α-2a, Interferon-α-2b, Consensus-Interferon, pegyliertes Interferon-α-2a oder pegyliertes Interferon-α-2b ist.

26. Pharmazeutische Zusammensetzung nach Anspruch 22, bei der die durch die Formel VIII wiedergegebene Verbindung für orale Verabreichung angepasst ist.

27. Verbindung nach Anspruch 21, bei der das pharmazeutisch annehmbare Salz Trifluoracetat, Tosylat, Mesylat, Sulfat, 2-Naphthalinsulfonat oder Chlorid ist.

## Revendications

1. Composé de formule III : 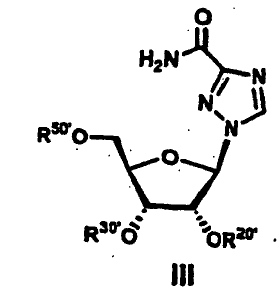 dans laquelle au moins l'un des groupes R^{50'}, R^{30'} et R^{20'} est un groupe de formule 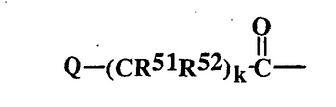 et les deux autres des groupes R^{50'}, R^{30'} et R^{20'} représentent H ou un groupe de formule 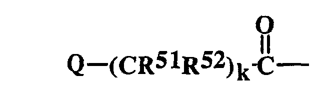 Q représente un groupe 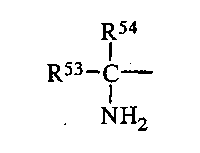 R⁵¹ et R⁵² représentent chacun indépendamment un atome d'hydrogène, un groupe alkyle, alcényle, alcynyle, cycloalkyle en C₃ à C₇ ou arylalkyle, ou un groupe alkyle, alcényle, alcynyle, cycloalkyle en C₃ à C₇ ou arylalkyle, substitué par un ou plusieurs substituants pris parmi les atomes d'halogène, OH, SH, CF₃, SR⁵⁷, OR⁵⁷ et NH₂, ou bien R⁵¹ et R⁵² forment ensemble avec l'atome de carbone de (CR⁵¹R⁵²) un cycle cyclopropane, cyclobutane, cyclopentane ou cyclohexane,
R⁵³ et R⁵⁴ représentent chacun indépendamment un atome d'hydrogène ou un groupe alcanoyle, alkyle, aryle, arylalkyle, alcényle ou alcynyle, éventuellement substitué par un ou plusieurs substituants pris parmi les atomes d'halogène, OH, SH, CF₃, SR⁵⁷ et OR⁵⁷, ou bien R⁵³ et R⁵⁴ représentent chacun indépendamment 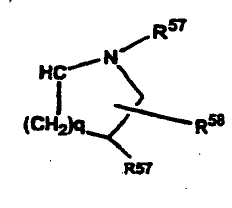 ou, 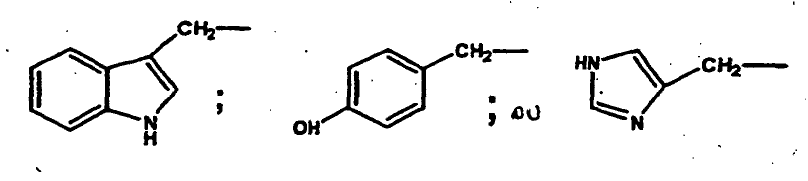 R⁵⁷ représente un atome d'hydrogène, un groupe alkyle, alcanoyle, alcénoyle, aryle, arylalkyle, alcényle ou alcynyle, ou un groupe alkyle, aryle, arylalkyle, alcényle ou alcynyle, substitué par un ou plusieurs substituants pris parmi les atomes d'halogène, OH, SH, CF₃, les groupes alcanoylthiényle et les groupes alcanoyloxy,
R⁵⁸ représente un atome d'hydrogène ou un groupe alkyle, aryle, arylalkyle, alcényle ou alcynyle,
q est égal à 0, 1 ou 2 et k est égal à 1 ou 2,
ou un sel pharmaceutiquement acceptable de ce composé.

2. Composition pharmaceutique pour traiter une infection virale sensible, qui comprend une quantité efficace d'un composé représenté par la formule III de la revendication 1, et un support pharmaceutiquement acceptable.

3. Utilisation d'un composé de formule III pour la préparation d'un médicament pour le traitement d'une infection chronique par l'hépatite C, qui comprend une quantité efficace d'un composé de formule III en association avec une quantité efficace d'un interféron α, ces quantités étant suffisantes pour supprimer des niveaux décelables de ARN-VHC, utilisation dans laquelle le composé est un composé représenté par la formule III de la revendication 1.

4. Composé de formule IV 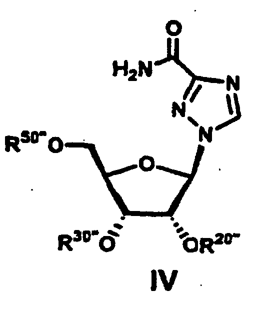 dans laquelle au moins l'un des groupes R^{50"}, R^{30"} et R^{20"} est un groupe de formule 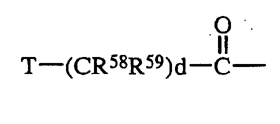 et les deux autres des groupes R^{50"}, R^{30"} et R^{20"} représentent chacun un atome d'hydrogène ou un groupe de formule 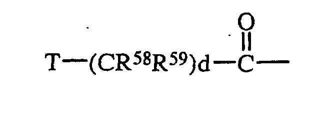 T représente un groupe de formule
H₂NCH₂-, H₂NCH₂ CH₂-, CH₃CH(H₂N)-, CH₃CH₂CH(H₂N)-,
CH₃(CH₂)₂CH(H₂N)-, (CH₃)₂CHCH(H₂N)-, (CH₃)₂CHCH₂CH(H₂N)-,
CH₃CH₂CH(CH₃ )CH(H₂N)-, PhCH₂CH(H₂N)-, HOOCCH₂CH₂CH(H₂N)-,
HOOCCH₂CH(H₂N)-, HSCH₂CH(H₂N)-, CH₃SCH₂CH₂CH(H₂N)-,
HOCH₂CH(H₂N)-, H₂N(CH2)₄ CH(H₂N)-, CH₃CH(OH)CH(H₂N), 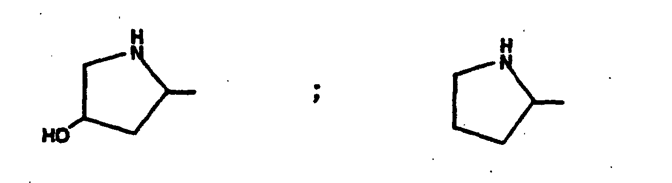 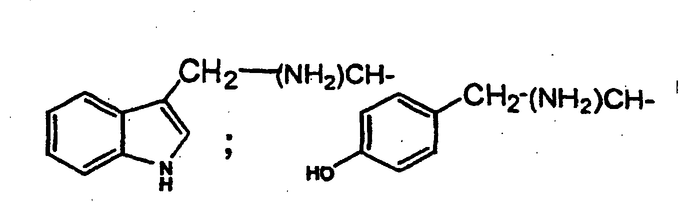 ou 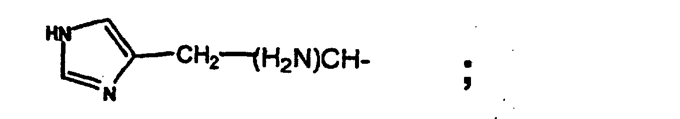 R⁵⁸ et R⁵⁹ représentent chacun indépendamment un atome d'hydrogène, ou un groupe alkyle, alcényle, alcynyle, cycloalkyle en C₃ à C₇ ou arylalkyle, éventuellement substitué par un ou plusieurs substituants choisis parmi les atomes d'halogène, OH, SH, CF₃, SR⁶⁰, OR⁶⁰ et NR⁶⁰R⁶¹, ou bien R⁵⁸ et R⁵⁹ forment ensemble avec l'atome de carbone de (CR⁵⁸R⁵⁹) un cycle cyclopropane, cyclobutane, cyclopentane ou cyclohexane,
R⁶⁰ représente un atome d'hydrogène, un groupe alkyle, alcanoyle, alcénoyle, aryle, arylalkyle, alcényle ou alcynyle, ou un groupe alkyle, aryle, arylalkyle, alcényle ou alcynyle, substitué par un ou plusieurs substituants pris parmi les atomes d'halogène, OH, SH, CF₃, les groupes alcanoylthiényle et les groupes alcanoyloxy,
R⁶¹ représente un atome d'hydrogène ou un groupe alkyle, aryle, arylalkyle, alcényle ou alcynyle,
et d est égal à 0, 1 ou 2,
ou un sel pharmaceutiquement acceptable de ce composé.

5. Composition pharmaceutique pour traiter une infection virale sensible, qui comprend une quantité efficace d'un composé représenté par la formule IV de la revendication 4, et un support pharmaceutiquement acceptable.

6. Utilisation d'un composé de formule IV pour la préparation d'un médicament pour le traitement d'infections chroniques par l'hépatite C, qui comprend une quantité efficace d'un composé de formule IV en association avec une quantité efficace d'un interféron alfa, ces quantités étant suffisantes pour supprimer des niveaux décelables de ARN-VHC, utilisation dans laquelle le composé est un composé représenté par la formule IV de la revendication 4.

7. Composé de formule V 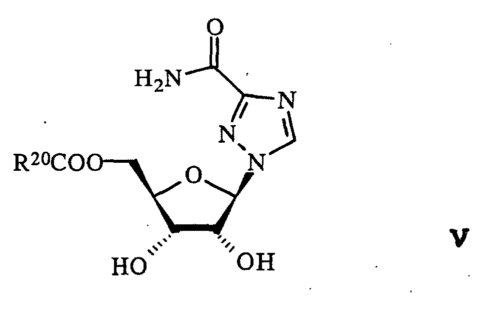 dans laquelle R²⁰CO- représente un fragment d'amino-acide naturel ou synthétique, de formule 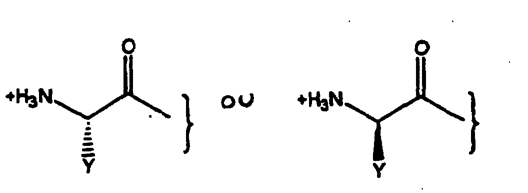 Y représente
H, CH₃; CH₃CH₂-; CH₃CH₂CH₂-; Me₂CH-,Me₂CH₂CH₂-; CH₃CH₂CH(Me)-PhCH₂-; HOOCCH₂CH₂-; HSCH₂-; HOOCCH₂-; MeSCH₂CH₂-; HOCH₂-; 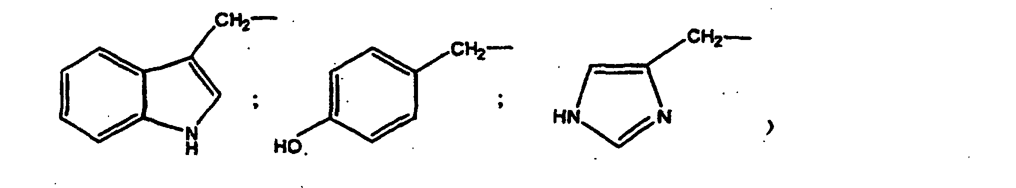 H₂N(CH₂)₄- ou CH₃CH(OH)- ,
ou bien Y forme avec l'atome de carbone α et l'atome d'azote N 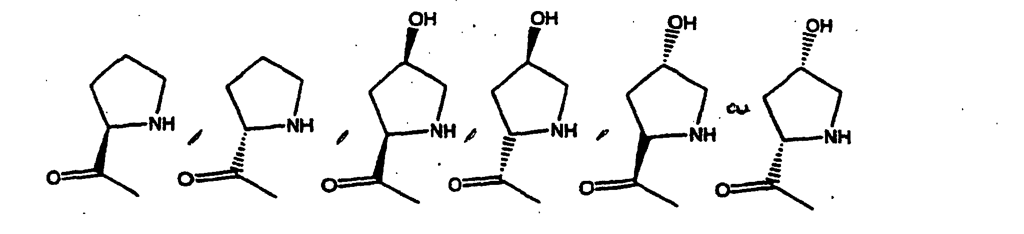 ou un sel pharmaceutiquement acceptable de ce composé.

8. Composition pharmaceutique pour traiter une infection virale sensible, qui comprend une quantité efficace d'un composé représenté par la formule V de la revendication 7, et un support pharmaceutiquement acceptable.

9. Utilisation d'un composé de formule V pour la préparation d'un médicament pour le traitement d'une infection chronique par l'hépatite C, qui comprend une quantité efficace d'un composé de formule V en association avec une quantité efficace d'un interféron α, ces quantités étant suffisantes pour supprimer des niveaux décelables de ARN-VHC, utilisation dans laquelle le composé est un composé représenté par la formule V de la revendication 7.

10. Composé de formule VI 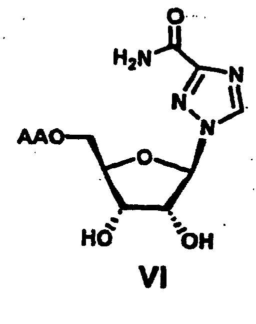 dans laquelle AA représente un fragment d'amino-acide naturel ou synthétique, correspondant aux formules indiquées dans le Tableau AA, ou un sel pharmaceutiquement acceptable de ce composé.

11. Composition pharmaceutique pour traiter une infection virale sensible, qui comprend une quantité efficace d'un composé représenté par la formule VI de la revendication 10, et un support pharmaceutiquement acceptable.

12. Utilisation d'un composé de formule VI pour la préparation d'un médicament pour le traitement d'un infection chronique par l'hépatite C, qui comprend une quantité efficace d'un composé de formule VI en association avec une quantité efficace d'un interféron α, ces quantités étant suffisantes pour supprimer des niveaux décelables de ARN-VHC, utilisation dans laquelle le composé est un composé représenté par la formule VI de la revendication 10.

13. Composé de formule VII 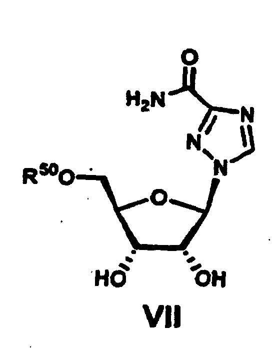 dans laquelle R⁵⁰ représente CH₃CH(NH₂)-CO-, CH₃CH₂(CH₃)CHCH(NH₂)-CO- ou H₂N(CH₂)₄CH(NH₂)-CO-,
ou un sel pharmaceutiquement acceptable de ce composé.

14. Composé selon la revendication 13, pour lequel le sel pharmaceutiquement acceptable est le trifluoroacétate, le tosylate, le mésylate ou le chlorure.

15. Composé selon la revendication 13, pour lequel R⁵⁰ représente 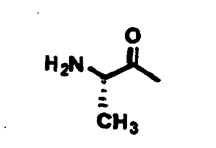 ou 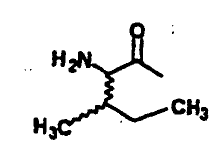 ou 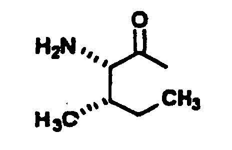 ou 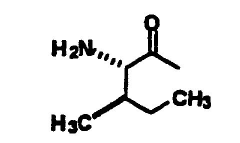 ou 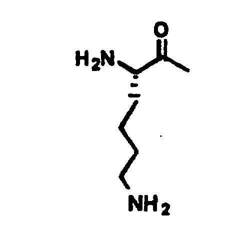 ou 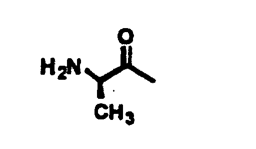 ou 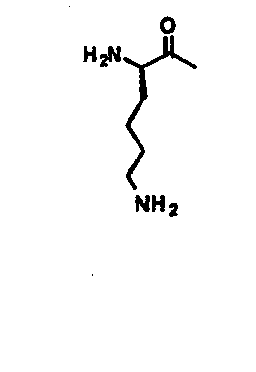 ou 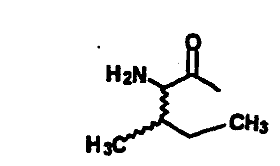 ou 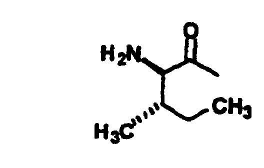 ou 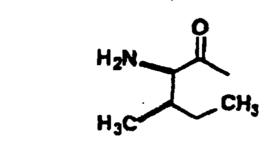 ou un sel pharmaceutiquement acceptable de ce composé.

16. Composition pharmaceutique pour le traitement d'une infection virale sensible, qui comprend un composé selon la revendication 13, et au moins un support pharmaceutiquement acceptable.

17. Composition pharmaceutique selon la revendication 16, qui convient à une administration orale.

18. Utilisation d'un composé de formule VII pour la préparation d'un médicament pour le traitement d'une infection chronique sensible par l'hépatite C, qui comprend une quantité efficace d'un composé représenté par la formule VII de la revendication 13.

19. Utilisation d'un composé de formule VII pour la préparation d'un médicament pour le traitement d'une infection chronique par l'hépatite C, qui comprend une quantité efficace d'un composé de formule VII en association avec une quantité efficace d'un interféron α, ces quantités étant suffisantes pour supprimer des niveaux décelables de ARN-VHC, utilisation dans laquelle le composé est un composé représenté par la formule VII de la revendication 13.

20. Utilisation selon la revendication 19, dans laquelle l'interféron α est de l'interféron α-2a, de l'interféron α-2b, de l'interféron consensus, de l'interféron α-2a pégylé ou de l'interféron α-2b pégylé.

21. Composé de formule VIII : 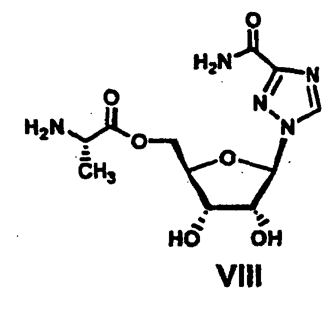 ou un sel pharmaceutiquement acceptable de ce composé.

22. Composition pharmaceutique qui comprend une quantité efficace quant à l'effet antiviral, du composé selon la revendication 21, et un support pharmaceutiquement acceptable.

23. Utilisation d'un composé de formule VIII pour la préparation d'un médicament pour le traitement d'une infection chronique sensible par l'hépatite C, qui comprend une quantité efficace d'un composé représenté par la formule VIII de la revendication 21.

24. Utilisation d'un composé de formule VIII pour la préparation d'un médicament pour le traitement d'une infection chronique par l'hépatite C, qui comprend une quantité efficace d'un composé de formule VIII selon la revendication 21 ou d'un sel pharmaceutiquement acceptable de ce composé, en association avec une quantité efficace d'un interféron α, ces quantités étant suffisantes pour supprimer des niveaux décelables de ARN-VHC.

25. Utilisation selon la revendication 24, dans laquelle l'interféron α est de l'interféron α-2a, de l'interféron α-2b, de l'interféron consensus, de l'interféron α-2a pégylé ou de l'interféron α-2b pégylé.

26. Composition pharmaceutique selon la revendication 22, pour laquelle le composé représenté par la formule VIII est un composé convenant à une administration par voie orale.

27. Composé selon la revendication 21, pour lequel le sel pharmaceutiquement acceptable est le trifluoroacétate, le tosylate, le mésylate, le sulfate, le 2-naphtalènesulfonate ou le chlorure.
